# EUROPEAN PATENT APPLICATION

(11) **EP 2 881 391 A1**
(43) Date of publication of application: **10.06.2015**
(21) Application number: 13195957.9
(22) Date of filing: 05.12.2013
(51) Int. Cl.: C07D 403/12, C07D 413/12, C07D 209/96, A61K 31/404

(54) **Spiroindoline carbocycle derivatives and pharmaceutical compositions thereof**

(71) Applicant: Bayer Pharma Aktiengesellschaft, 13353 Berlin (DE)
(72) Inventor: Panknin, Olaf, 10117 Berlin (DE); Baeurle, Stefan, 10245 Berlin (DE); Schwede, Wolfgang, 16548 Glienicke (DE); Ring, Sven, 7749 Jena (DE); Nowak-Reppel, Katrin, 13187 Berlin (DE); Langer, Gernot, 14612 Falkensee (DE)
(74) Representative: BIP Patents

(57) **Abstract**

Spiroindoline carbocycle derivatives, process for their preparation and pharmaceutical compositions thereof, their use for the treatment and/or prophylaxis of diseases, and their use for the manufacture of medicaments for the treatment and/or prophylaxis of diseases, especially sex-hormone-related diseases in both men and women, in particularly those selcted from the group of endometriosis, uterine fibroids, polycystic ovarian disease, hirsutism, precocious puberty, gonadal steroid-dependent neoplasia such as cancers of the prostate, breast and ovary, gonadotrope pituitary adenomas, sleep apnea, irritable bowel syndrome, premenstrual syndrome, benign prostatic hypertrophy, contraception and infertility (e.g., assisted reproductive therapy such as in vitro fertilization). The present application relates in particular to spiroindoline derivatives as gonadotropin-releasing hormone (GnRH) receptor antagonists.

## Description

### TECHNICAL FIELD

The present invention refers to spiroindoline derivatives as gonadotropin-releasing hormone (GnRH) receptor antagonists, pharmaceutical compositions containing a spiroindoline derivative according to the invention and methods of treating disorders by administration of a spiroindoline derivative according to invention to a mammal, particularly a human, in need thereof.

### BACKGROUND ART

Gonadotropin-releasing hormone (GnRH) is a decapeptide (pGlu-His-Trp-Ser-Tyr-Gly-Leu-Arg-Pro-Gly-NH₂) released from the hypothalamus, also known as luteinizing hormone-releasing hormone (LHRH). GnRH acts on the pituitary gland to stimulate the biosynthesis and release of luteinizing hormone (LH) and follicle-stimulating hormone (FSH). LH released from the pituitary gland is responsible for the regulation of gonadal steroid production in both genders and late ovarian follicle development and ovulation in female mammals, FSH regulates spermatogenesis in males and early follicular development in females. Thus GnRH plays a key role in human reproduction.
As a consequence of its biological significance, synthetic antagonists and agonists to GnRH have been the center of several research activities, particularly in the field of endometriosis, uterine leiomyoma (fibroids), prostate cancer, breast cancer, ovarian cancer, prostatic hyperplasia, assisted reproductive therapy and precocious puberty.

For example, peptidic GnRH agonists, such as leuprorelin (pGlu-His-Trp-Ser-Tyr-d-Leu-Leu-Arg-Pro-NHEt), are described for the use in the treatment of such conditions (The Lancet 2001, 358, 1793 - 1803; Mol. Cell. Endo. 2000, 166, 9 - 14). Said agonists initially induce the synthesis and release of gonadotropins, by binding to the GnRH receptor on the pituitary gonadotrophic cells ('flare-up'). However, chronic administration of GnRH agonists reduces gonadotropin release from the pituitary and results in the down-regulation of the receptor, with the consequence of suppressing sex steroidal hormone production after some period of treatment.

GnRH antagonists, on the contrary, are supposed to suppress gonadotropins from the onset, offering several advantages, in particular a lack of side effects associated with the flare up seen under GnRH superagonist treatment. Several peptidic antagonists with low histamine release potential are known in the art. Said peptidic products show low oral bioavailability which limits their clinical use.

The state of the art involves a number of nonpeptidic compounds for use as GnRH receptor antagonists, for example in WO2011/076687, WO05/007165, WO03/064429 and WO04/067535. Although intensive research has been driven for more than 15 years aiming at non-peptidic GnRH antagonists, none of them succeeded so far to reach the market.

Nevertheless, effective small molecule GnRH receptor ligands, especially compounds which are active as antagonists as well as pharmaceutical compositions containing such GnRH receptor antagonists and methods relating to the use thereof to treat, for example, sex-hormone-related conditions, in particular for the treatment of leiomyoma are still highly required in the pharmaceutical field.

The spiroindoline derivatives according to the present invention aim to fulfill such unmet need, and provide at the same time further advantages over the known art.

Spiroindoline derivatives are known in the art as pharmaceutically active ingredients and as insecticides but their activity as GnRH receptor antagonists has been described in WO2013/107743 for the first time.

The document WO00/66554 describes generic indolines as potential PR antagonists.

The document US2006/63791, page 20, describes the synthesis of a nitroindoline by condensing an aldehyde and a phenylhydrazine under acidic conditions (Fischer indole synthesis) and subsequent reduction of the indolenine intermediate.

*Liu et al.* describes the synthesis of a spirotetrahydropyrane in a similar manner in a one-pot reaction (Tetrahedron 2010, 66, 3, 573-577).

The document WO10/151737, page 224, describes the synthesis of an indolenine mixture in an analogous Fischer indole synthesis by condensing an aldehyde with a phenylhydrazine.

The document WO06/090261, pp. 67-68, describes the synthesis of a spiropiperidine via Fischer indole synthesis and subsequent addition of a Grignard reagent to the indolenine intermediate.

The document WO08/157741, pp. 41-42, describes the synthesis of a spiropiperidine starting from an oxindole precursor via Grignard addition and subsequent deoxygenation.

The document WO93/15051 discloses a generic oxindole as potential vasopressin/oxytocin antagonists.

Further spiroindoline derivatives with pharmaceutical properties were disclosed for example in the documents WO1994/29309 WO1999/6400 and WO2002/47679.

### DISCLOSURE OF THE INVENTION

The aim of the present invention is to provide gonadotropin-releasing hormone (GnRH) receptor antagonists, as well as the methods for their preparation and use, and pharmaceutical compositions containing the same.
In particular, the present invention relates to compounds according to formula **(I)** 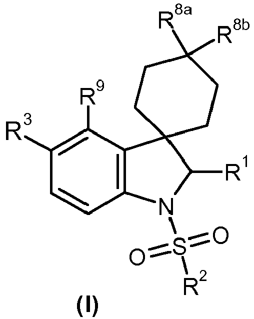 in which
- R¹: is selected from the group consisting of hydrogen, C₁-C₆-alkyl, C₃-C₆-cycloalkyl, C₂-C₆-alkenyl, C₂-C₆-alkynyl, aryl, hydroxy-C₁-C₆-alkyl, C₁-C₆-alkoxy-C₁-C₆-alky;
- R²: is an aryl or heteroaryl group which can be unsubstituted or substituted one to three times with a group R⁴;
- R³: is selected from the group consisting of C(O)N(R^{5a})(R^{5b}), N(H)C(O)R⁶, N(H)C(O)N(R^{5a})(R^{5b}) or N(H)C(O)OR⁷;
- R⁴: is selected from the group consisting of halogen, hydroxy, CN, C₁-C₆-alkyl, halo-C₁-C₆-alkyl, C₁-C₆-alkoxy, halo-C₁-C₆-alkoxy, C(O)OH, C(O)O-C₁-C₆-alkyl, C(O)NH₂, C(O)NH-C₁-C₆-alkyl, C(O)N(C₁-C₆-alkyl)₂ in which the two alkyl groups are independent from each other or together form a 4-, 5-, 6- or 7-membered cyclic amine group;
- R^{5a}, R^{5b} and R⁶: are selected, independently from one another, from the group consisting of hydrogen, C₁-C₆-alkyl, halo-C₁-C₆-alkyl, hydroxy-C₁-C₆-alkyl; C₂-C₆-alkenyl, C₂-C₆-alkynyl, C₁-C₆-alkoxy-C₁-C₆-alkyl, C₃-C₁₀-cycloalkyl, C₃-C₁₀-cycloalkyl-C₁-C₆-alkyl-, aryl, aryl-C₁-C₆-alkyl-, aryl-cyclopropyl, heteroaryl, heteroaryl-C₁-C₆-alkyl-, in which said cycloalkyl, aryl, heteroaryl groups are optionally substituted up to three times with halogen, hydroxy, C₁-C₆-alkyl, C₁-C₆-haloalkyl, C₁-C₆-alkoxy, C₁-C₆-haloalkoxy, C(O)OH, C(O)O-C₁-C₆-alkyl, CN, C(O)NH₂, S(O)₂-C₁-C₆-alkyl, S(O)₂NH₂, S(O)₂N(C₁-C₆-alkyl)₂ in which the two alkyl groups are independent from each other;
- R⁷: is selected from the group consisting of C₁-C₆-alkyl, halo-C₁-C₆-alkyl, hydroxy-C₁-C₆-alkyl, C₂-C₆-alkenyl, C₂-C₆-alkynyl, C₁-C₆-alkoxy-C₁-C₆-alkyl, C₃-C₁₀-cyclalkyl, C₃-C₁₀-cycloalkyl-C₁-C₆-alkyl, aryl, aryl-C₁-C₆-alkyl, heteroaryl, or heteroaryl-C₁-C₆-alkyl in which said cycloalkyl, aryl, heteroaryl group is optionally substituted up to three times with a halogen, hydroxy, C₁-C₆-alkyl, C₁-C₆-haloalkyl, C₁-C₆-alkoxy, C₁-C₆-haloalkoxy, C(O)OH, C(O)O-C₁-C₆-alkyl, CN, C(O)NH₂, S(O)₂-C₁-C₆-alkyl, S(O)₂NH₂, S(O)₂N(C₁-C₆-alkyl)₂ in which the two alkyl groups are independent from each other;
- R^{8a}: is selected from the group consisting of hydrogen, hydroxy, halogen, C₁-C₆-alkyl, halo-C₁-C₆-alkyl;
- R^{8b}: is selected from the group consisting of hydrogen, halogen, C₁-C₆-alkyl, halo-C₁-C₆-alkyl;
or R^{8a} and R^{8b} together form an oxo group or an oxime group
- R⁹: is hydrogen, halogen, hydroxy, CN, C₁-C₆-alkyl, halo-C₁-C₆-alkyl, C₁-C₆-alkoxy, halo-C₁-C₆-alkoxy, C(O)OH, C(O)O-C₁-C₆-alkyl, C(O)NH₂, C(O)NH-C₁-C₆-alkyl, C(O)N(C₁-C₆-alkyl)₂ in which the two alkyl groups are independent from each other.

Compounds according to the invention are the compounds of the formula (I), and the salts, solvates and solvates of the salts thereof, the compounds which are encompassed by formula (I), and are of the formulae mentioned hereinafter, and the salts, solvates and solvates of the salts thereof, and the compounds which are encompassed by formula (I), and are mentioned hereinafter as exemplary embodiments, and the salts, solvates and solvates of the salts thereof, insofar as the compounds encompassed by formula **(I)** and mentioned hereinafter are not already salts, solvates and solvates of the salts.

Hydrates of the compounds of the invention or their salts are stoichiometric compositions of the compounds with water, such as, for example, hemi-, mono- or dihydrates.

Solvates of the compounds of the invention or their salts are stoichiometric compositions of the compounds with solvents.

Solvates which are preferred for the purposes of the present invention are hydrates.

Salts for the purposes of the present invention are preferably pharmaceutically acceptable salts of the compounds according to the invention (for example, see S. M. Berge et al., "Pharmaceutical Salts", J. Pharm. Sci. 1977, 66, 1-19).
Pharmaceutically acceptable salts include acid addition salts of mineral acids, carboxylic acids and sulfonic acids, for example salts of hydrochloric acid, hydrobromic acid, sulfuric acid, phosphoric acid, methanesulfonic acid, ethanesulfonic acid, toluenesulfonic acid, benzenesulfonic acid, naphthalenedisulfonic acid, maleic, fumaric, benzoic, ascorbic, succinic, acetic, trifluoroacetic, oxalic, propionic, tartaric, salicylic, citric, gluconic, lactic, mandelic, cinnamic, aspartic, stearic, palmitic, glycolic, and glutamic acid.

Pharmaceutically acceptable salts also include salts of customary bases, such as for example and preferably alkali metal salts (for example sodium, lithium and potassium salts), alkaline earth metal salts (for example calcium and magnesium salts), and ammonium salts derived from ammonia or organic amines, such as illustratively and preferably ethylamine, diethylamine, triethylamine, ethyldiisopropylamine, monoethanolamine, diethanolamine, triethanolamine, dicyclohexylamine, dimethylaminoethanol, benzylamine, dibenzylamine, N-methylmorpholine, N-methylpiperidine, dihydroabietyl- amine, arginine, lysine, and ethylenediamine.

Also encompassed are salts which are themselves unsuitable for pharmaceutical uses but can be used for example for isolating or purifying the compounds of the invention.

The present invention additionally encompasses prodrugs of the compounds of the invention. The term "prodrugs" encompasses compounds which themselves may be biologically active or inactive, but are converted during their residence time in the body into compounds of the invention (for example by metabolism or hydrolysis).

The present invention includes all possible stereoisomers of the compounds of the present invention as single stereoisomers, or as any mixture of said stereoisomers,
e.g. R- or S- isomers, or E- or Z-isomers, in any ratio.

All isomers, whether separated, pure, partially pure, or in racemic mixture, of the compounds of this invention are encompassed within the scope of this invention. The purification of said isomers and the separation of said isomeric mixtures may be accomplished by standard techniques known in the art. For example, diastereomeric mixtures can be separated into the individual isomers by chromatographic processes or crystallization, and racemates can be separated into the respective enantiomers either by chromatographic processes on chiral phases or by resolution.

If the compounds of the invention may occur in tautomeric forms, the present invention encompasses all tautomeric forms.

Unless otherwise stated, the following definitions apply for the substituents and residues used throughout this specification and claims. The particularly named chemical groups and atoms (for example fluorine, methyl, methyloxy and so on) should be considered as particular forms of embodiment for the respective groups in compounds according to the invention.

The term "halogen", "halogen atom" or "halo" is to be understood as meaning a fluorine, chlorine, bromine or iodine atom.

The term "C₁-C₆-alkyl" is to be understood as preferably meaning a linear or branched, saturated, monovalent hydrocarbon group having 1, 2, 3, 4, 5 or 6 carbon atoms, *e.g.* a methyl, ethyl, propyl, butyl, pentyl, hexyl, isopropyl, isobutyl, sec-butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl group, or an isomer thereof. Particularly, said group has 1, 2 or 3 carbon atoms ("C₁-C₃-alkyl"), methyl, ethyl, n-propyl- or iso-propyl.

The term "halo-C₁-C₆-alkyl" is to be understood as preferably meaning a linear or branched, saturated, monovalent hydrocarbon group in which the term "C₁-C₆-alkyl" is defined *supra,* and in which one or more hydrogen atoms is replaced by a halogen atom, in the same way or differently, *i.e.* one halogen atom being independent from another.
Particularly, said halogen atom is F. Said halo-C₁-C₆-alkyl group is, in particular -CF₃, -CHF₂, -CH₂F, -CF₂CF₃, -CF₂CH₃, or -CH₂CF₃.

The term "C₁-C₆-alkoxy" is to be understood as preferably meaning a linear or branched, saturated, monovalent, hydrocarbon group of formula -O-alkyl, in which the term "alkyl" is defined *supra*, *e.g.* a methoxy, ethoxy, propoxy, isopropoxy, butoxy, isobutoxy, *tert*-butoxy, sec-butoxy, pentyloxy, isopentyloxy, or hexyloxy group, or an isomer thereof.

The term "halo-C₁-C₆-alkoxy" is to be understood as preferably meaning a linear or branched, saturated, monovalent C₁-C₆-alkoxy group, as defined *supra*, in which one or more of the hydrogen atoms is replaced, in the same way or differently, by a halogen atom.
Particularly, said halogen atom is F. Said halo-C₁-C₆-alkoxy group is, for example, -OCF₃, -OCHF₂, -OCH₂F, -OCF₂CF₃, or -OCH₂CF₃.

The term "C₁-C₆-alkoxy-C₁-C₆-alkyl" is to be understood as preferably meaning a linear or branched, saturated, monovalent alkyl group, as defined *supra,* in which one or more of the hydrogen atoms is replaced, in the same way or differently, by a C₁-C₆-alkoxy group, as defined *supra*, *e.g.* methoxyalkyl, ethoxyalkyl, propoxyalkyl, isopropoxyalkyl, butoxyalkyl, isobutoxyalkyl, *tert*-butoxyalkyl, sec-butoxyalkyl, pentyloxyalkyl, isopentyloxyalkyl, hexyloxyalkyl group, in which the term "C₁-C₆-alkyl" is defined *supra,* or an isomer thereof.

The term "halo-C₁-C₆-alkoxy-C₁-C₆-alkyl" is to be understood as preferably meaning a linear or branched, saturated, monovalent C₁-C₆-alkoxy-C₁-C₆-alkyl group, as defined *supra,* in which one or more of the hydrogen atoms is replaced, in the same way or differently, by a halogen atom.
Particularly, said halogen atom is F. Said halo-C₁-C₆-alkoxy-C₁-C₆-alkyl group is, for example, -CH₂CH₂OCF₃, -CH₂CH₂OCHF₂, -CH₂CH₂OCH₂F, -CH₂CH₂OCF₂CF₃, or - CH₂CH₂OCH₂CF₃.

The term "C₂-C₆-alkenyl" is to be understood as preferably meaning a linear or branched, monovalent hydrocarbon group, which contains one or more double bonds, and which has 2, 3, 4, 5, 6 carbon atoms, particularly 2 or 3 carbon atoms ("C₂-C₃-alkenyl"), it being understood that in the case in which said alkenyl group contains more than one double bond, then said double bonds may be isolated from, or conjugated with, each other.
Said alkenyl group is, for example, a vinyl, allyl, (E)-2-methylvinyl, (Z)-2-methylvinyl, homoallyl, (E)-but-2-enyl, (Z)-but-2-enyl, (E)-but-1-enyl, (Z)-but-1-enyl, pent-4-enyl, (E)-pent-3-enyl, (Z)-pent-3-enyl, (E)-pent-2-enyl, (Z)-pent-2-enyl, (E)-pent-1-enyl, (Z)-pent-1-enyl, hex-5-enyl, (E)-hex-4-enyl, (Z)-hex-4-enyl, (E)-hex-3-enyl, (Z)-hex-3-enyl, (E)-hex-2-enyl, (Z)-hex-2-enyl, (E)-hex-1-enyl, (Z)-hex-1-enyl, isopropenyl, 2-methylprop-2-enyl, 1-methylprop-2-enyl, 2-methylprop-1-enyl, (E)-1-methylprop-1-enyl, (Z)-1-methylprop-1-enyl, 3-methylbut-3-enyl, 2-methylbut-3-enyl, 1-methylbut-3-enyl, 3-methylbut-2-enyl, (E)-2-methylbut-2-enyl, (Z)-2-methylbut-2-enyl, (E)-1-methylbut-2-enyl, (Z)-1-methylbut-2-enyl, (E)-3-methylbut-1-enyl, (Z)-3-methylbut-1-enyl, (E)-2-methylbut-1-enyl, (Z)-2-methylbut-1-enyl, (E)-1-methylbut-1-enyl, (Z)-1-methylbut-1-enyl, 1,1-dimethylprop-2-enyl, 1-ethylprop-1-enyl, 1-propylvinyl, 1-isopropylvinyl, 4-methylpent-4-enyl, 3-methylpent-4-enyl, 2-methylpent-4-enyl, 1-methylpent-4-enyl, 4-methylpent-3-enyl, (E)-3-methylpent-3-enyl, (Z)-3-methylpent-3-enyl, (E)-2-methylpent-3-enyl, (Z)-2-methylpent-3-enyl, (E)-1-methylpent-3-enyl, (Z)-1-methylpent-3-enyl, (E)-4-methylpent-2-enyl, (Z)-4-methylpent-2-enyl, (E)-3-methylpent-2-enyl, (Z)-3-methylpent-2-enyl, (E)-2-methylpent-2-enyl, (Z)-2-methylpent-2-enyl, (E)-1-methylpent-2-enyl, (Z)-1-methylpent-2-enyl, (E)-4-methylpent-1-enyl, (Z)-4-methylpent-1-enyl, (E)-3-methylpent-1-enyl, (Z)-3-methylpent-1-enyl, (E)-2-methylpent-1-enyl, (Z)-2-methylpent-1-enyl, (E)-1-methylpent-1-enyl, (Z)-1-methylpent-1-enyl, 3-ethylbut-3-enyl, 2-ethylbut-3-enyl, 1-ethylbut-3-enyl, (E)-3-ethylbut-2-enyl, (Z)-3-ethylbut-2-enyl, (E)-2-ethylbut-2-enyl, (Z)-2-ethylbut-2-enyl, (E)-1-ethylbut-2-enyl, (Z)-1-ethylbut-2-enyl, (E)-3-ethylbut-1-enyl, (Z)-3-ethylbut-1-enyl, 2-ethylbut-1-enyl, (E)-1-ethylbut-1-enyl, (Z)-1-ethylbut-1-enyl, 2-propylprop-2-enyl, 1-propylprop-2-enyl, 2-isopropylprop-2-enyl, 1-isopropylprop-2-enyl, (E)-2-propylprop-1-enyl, (Z)-2-propylprop-1-enyl, (E)-1-propylprop-1-enyl, (Z)-1-propylprop-1-enyl, (E)-2-isopropyl prop- 1 -enyl, (Z)-2-isopropyl prop- 1 -enyl, (E)-1-isopropylprop-1-enyl, (Z)-1-isopropylprop-1-enyl, (E)-3,3-dimethylprop-1-enyl, (Z)-3,3-dimethylprop-1-enyl, 1-(1,1-dimethylethyl)vinyl, buta-1,3-dienyl, penta-1,4-dienyl, hexa-1,5-dienyl, or methylhexadienyl group. Particularly, said group is vinyl or allyl.

The term "C₂-C₆-alkynyl" is to be understood as preferably meaning a linear or branched, monovalent hydrocarbon group which contains one or more triple bonds, and which contains 2, 3, 4, 5, 6 carbon atoms, particularly 2 or 3 carbon atoms ("C₂-C₃-alkynyl").
Said C₂-C₁₀-alkynyl group is, for example, ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-inyl, hex-3-inyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-inyl, 1,1-dimethylbut-3-ynyl, 1,1-dimethylbut-2-ynyl, or 3,3-dimethylbut-1-ynyl group. Particularly, said alkynyl group is ethynyl, prop-1-ynyl, or prop-2-ynyl.

The term "C₃-C₁₀-cycloalkyl" is to be understood as preferably meaning a saturated, monovalent, mono-, or bicyclic hydrocarbon ring which contains 3, 4, 5, 6, 7, 8, 9, or 10 carbon atoms, particularly 3, 4, 5, or 6 carbon atoms ("C₃-C₆-cycloalkyl").
Said C₃-C₁₀-cycloalkyl group is for example, a monocyclic hydrocarbon ring, *e.g.* a cyclopropyl, cyclobutyl, cyclopentyl, cyclohexyl, cycloheptyl, cyclooctyl, cyclononyl or cyclodecyl group, or a bicyclic hydrocarbon ring, *e.g.* a perhydropentalenylene or decalin ring. Said cycloalkyl ring can optionally contain one or more double bonds *e.g.* cycloalkenyl, such as a cyclopropenyl, cyclobutenyl, cyclopentenyl, cyclohexenyl, cycloheptenyl, cyclooctenyl, cyclononenyl, or cyclodecenyl group, wherein the bond between said ring with the rest of the molecule may be to any carbon atom of said ring, be it saturated or unsaturated.

The term "aryl" is to be understood as preferably meaning a monovalent, aromatic or partially aromatic, mono-, bi- or tricyclic hydrocarbon ring having 6, 7, 8, 9, 10, 11, 12, 13 or 14 carbon atoms (a "C₆-C₁₄-aryl" group), particularly a ring having 6 carbon atoms (a "C₆-aryl" group), *e.g.* a phenyl group, or a biphenyl group, or a ring having 9 carbon atoms (a "C₉-aryl" group), *e.g.* an indanyl or indenyl group, or a ring having 10 carbon atoms (a "C₁₀-aryl" group), *e.g.* a tetralinyl, dihydronaphthyl, or naphthyl group, or a ring having 13 carbon atoms, (a "C₁₃-aryl" group), *e.g.* a fluorenyl group, or a ring having 14 carbon atoms, (a "C₁₄-aryl" group), *e.g.* an anthranyl group.

The term "heteroaryl" is understood as preferably meaning a monovalent, aromatic or partially aromatic, mono- or bicyclic ring system having 5, 6, 7, 8, 9, 10, 11, 12, 13 or 14 ring atoms (a "5- to 14-membered heteroaryl" group), particularly 5 or 6 or 9 or 10 atoms, and which can partially be saturated, and which contains at least one heteroatom which may be identical or different, said heteroatom being such as oxygen, nitrogen or sulfur, and can be monocyclic, bicyclic, or tricyclic, and in addition in each case can be benzocondensed. Preference is given to 6-membered heteroaryl radicals having up to 2 nitrogen atoms, and to 5-membered heteroaryl radicals having up to 3 heteroatoms. Particularly, heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, thia-4H-pyrazolyl, tetrazolyl and benzo derivatives thereof, such as, for example, benzofuranyl, benzothienyl, benzoxazolyl, benzisoxazolyl, benzimidazolyl, benzotriazolyl, indazolyl, indolyl, isoindolyl,; or pyridyl, pyridazinyl, pyrimidinyl, pyrazinyl, triazinyl, *etc.*, and benzo derivatives thereof, such as, for example, quinolinyl, quinazolinyl, isoquinolinyl, *etc.*; or azocinyl, indolizinyl, purinyl and benzo derivatives thereof; or cinnolinyl, phthalazinyl, quinazolinyl, quinoxalinyl, naphthpyridinyl, pteridinyl, carbazolyl, acridinyl, phenazinyl, phenothiazinyl, phenoxazinyl, xanthenyl, or oxepinyl. More particularly, heteroaryl is selected from thienyl, oxazolyl, thiazolyl, 1H-tetrazol-5-yl, pyridyl, benzothienyl, or furanyl.

The term "C₁-C₆", as used throughout this text, *e.g.* in the context of the definition of "C₁-C₆-alkyl", "C₁-C₆-haloalkyl", "C₁-C₆-alkoxy", or "C₁-C₆-haloalkoxy" is to be understood as meaning an alkyl group having a finite number of carbon atoms of 1 to 6, *i.e.* 1, 2, 3, 4, 5, or 6 carbon atoms. It is to be understood further that said term "C₁-C₆" is to be interpreted as any sub-range comprised therein, *e.g.* C₁-C₆, C₂-C₅, C₃-C₄, C₁-C₂, C₁-C₃, C₁-C₄, C₁-C₅ , C₁-C_{6;} particularly C₁-C₂, C₁-C₃, C₁-C₄, C₁-C₅ , C₁-C₆; more particularly C₁-C₄; in the case of "C₁-C₆-haloalkyl" or "C₁-C₆-haloalkoxy" even more particularly C₁-C₂.

Similarly, as used herein, the term "C₂-C₆", as used throughout this text, *e.g.* in the context of the definitions of "C₂-C₆-alkenyl" and "C₂-C₆-alkynyl", is to be understood as meaning an alkenyl group or an alkynyl group having a finite number of carbon atoms of 2 to 6, *i.e.* 2, 3, 4, 5, or 6 carbon atoms. It is to be understood further that said term "C₂-C₆" is to be interpreted as any sub-range comprised therein, *e.g.* C₂-C₆, C₃-C₅, C₃-C₄, C₂-C₃, C₂-C₄, C₂-C_{5;} particularly C₂-C₃.

Further, as used herein, the term "C₃-C₁₀", as used throughout this text, *e.g.* in the context of the definition of "C₃-C₁₀-cycloalkyl", is to be understood as meaning a cycloalkyl group having a finite number of carbon atoms of 3 to 10, *i.e.* 3, 4, 5, 6, 7, 8, 9 or 10 carbon atoms, particularly 3, 4, 5 or 6 carbon atoms. It is to be understood further that said term "C₃-C₁₀" is to be interpreted as any sub-range comprised therein, *e.g.* C₃-C₁₀, C₄-C₉, C₅-C₈, C₆-C₇ ; particularly C₃-C₆.

Oxo represents a double-bonded oxygen atom.

Oxim represents an imine with the general formula =N-OH.

As used herein, the term "one or more times", *e.g.* in the definition of the substituents of the compounds of the general formulae of the present invention, is understood as meaning "one, two, three, four or five times, particularly one, two, three or four times, more particularly one, two or three times, even more particularly one or two times".

Throughout this document, for the sake of simplicity, the use of singular language is given preference over plural language, but is generally meant to include the plural language if not otherwise stated. E.g., the expression "A method of treating a disease in a patient, comprising administering to a patient an effective amount of a compound of formula **(I)**" is meant to include the simultaneous treatment of more than one disease as well as the administration of more than one compound of formula **(I)**.

Particular forms of embodiment of compounds of the general formula **(I)** as described above are going to be illustrated in the following.

In conjunction with the above or below definitions and embodiments, compounds according to formula **(I)**, are in particular those of formula **(Ia)** 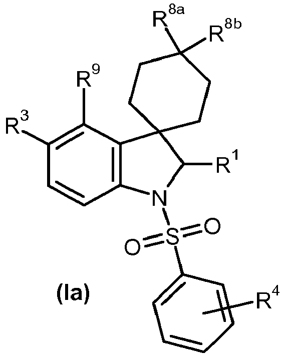 in which
- R¹: is selected from the group consisting of hydrogen, C₁-C₆-alkyl, C₃-C₆-cycloalkyl, C₂-C₆-alkenyl;
- R³: is selected from the group consisting of C(O)N(R^{5a})(R^{5b}), C(O)NH(R^{5a}), and N(H)C(O)R⁶;
- R⁴: is selected from the group consisting of halogen, hydroxy, CN, C₁-C₆-alkyl, halo-C₁-C₆-alkyl, C₁-C₆-alkoxy, halo-C₁-C₆-alkoxy, C(O)OH, C(O)O-C₁-C₆-alkyl, C(O)NH₂, C(O)NH-C₁-C₆-alkyl, C(O)N(C₁-C₆-alkyl)₂ in which the two alkyl groups are independent from each other or together form a 4-, 5-, 6- or 7-membered cyclic amine group;
- R^{5a}, R^{5b} and R⁶: are selected, independently from one another, from the group consisting of hydrogen, C₃-C₁₀-cycloalkyl-C₁-C₆-alkyl-, aryl, aryl-C₁-C₆-alkyl-, aryl-cyclopropyl, heteroaryl, heteroaryl-C₁-C₆-alkyl-, in which said cycloalkyl, aryl, heteroaryl groups are optionally substituted up to three times with halogen, C₁-C₆-alkyl, C₁-C₆-haloalkyl, C₁-C₆-alkoxy, C₁-C₆-haloalkoxy, C(O)OH, C(O)O-C₁-C₆-alkyl, S(O)₂-C₁-C₆-alkyl, S(O)₂NH₂, S(O)₂N(C₁-C₆-alkyl)₂ in which the two alkyl groups are independent from each other;
- R⁷: is selected from the group consisting of C₁-C₆-alkyl, halo-C₁-C₆-alkyl, hydroxy-C₁-C₆-alkyl, C₂-C₆-alkenyl, C₂-C₆-alkynyl, C₁-C₆-alkoxy-C₁-C₆-alkyl, C₃-C₁₀-cyclolkyl, C₃-C₁₀-cycloalkyl-C₁-C₆-alkyl, aryl, aryl-C₁-C₆-alkyl-, heteroaryl, or heteroaryl-C₁-C₆-alkyl- in which said cycloalkyl, aryl, heteroaryl group is optionally substituted up to three times with a halogen, hydroxy, an C₁-C₆-alkyl, C₁-C₆-haloalkyl, C₁-C₆-alkoxy, C₁-C₆-haloalkoxy, C(O)OH, C(O)O-C₁-C₆-alkyl, CN, C(O)NH₂, S(O)₂-C₁-C₆-alkyl, S(O)₂NH₂, S(O)₂N(C₁-C₆-alkyl)₂ in which the two alkyl groups are independent from each other.
- R^{8a}: is selected from the group consisting of hydrogen, hydroxy, halogen, C₁-C₆-alkyl, halo-C₁-C₆-alkyl;
- R^{8b}: is selected from the group consisting of hydrogen, halogen, C₁-C₆-alkyl, halo-C₁-C₆-alkyl; or
- R^{8a} and R^{8b}: together form an oxo group or an oxime group;
- R⁹: is hydrogen, halogen, hydroxy, CN, C₁-C₆-alkyl, halo-C₁-C₆-alkyl, C₁-C₆-alkoxy, halo-C₁-C₆-alkoxy, C(O)OH, C(O)O-C₁-C₆-alkyl, C(O)NH₂, C(O)NH-C₁-C₆-alkyl, C(O)N(C₁-C₆-alkyl)₂ in which the two alkyl groups are independent from each other.

Furthermore, in conjunction with the above or below definitions and embodiments, compounds according to formula **(I)**, are in particular those of formula **(Ib)** 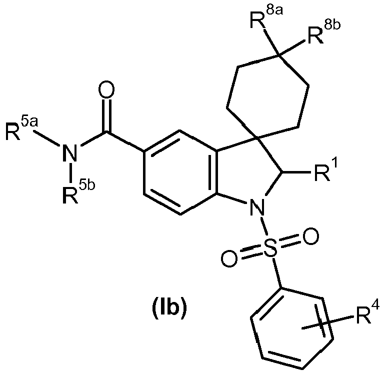 in which
- R¹: is selected from the group consisting of hydrogen, C₁-C₆-alkyl, C₃-C₆-cycloalkyl, C₂-C₆-alkenyl;
- R⁴: is selected from the group consisting of halogen, hydroxy, CN, C₁-C₆-alkyl, halo-C₁-C₆-alkyl, C₁-C₆-alkoxy, halo-C₁-C₆-alkoxy, C(O)OH, C(O)O-C₁-C₆-alkyl, C(O)NH₂, C(O)NH-C₁-C₆-alkyl, C(O)N(C₁-C₆-alkyl)₂ in which the two alkyl groups are independent from each other or together form a 4-, 5-, 6- or 7-membered cyclic amine group;
- R^{5a} and R^{5b}: are selected, independently from one another, from the group consisting of hydrogen, C₃-C₁₀-cycloalkyl-C₁-C₆-alkyl-, aryl, aryl-C₁-C₆-alkyl-, aryl-cyclopropyl, heteroaryl, heteroaryl-C₁-C₆-alkyl-, in which said cycloalkyl, aryl, heteroaryl groups are optionally substituted up to three times with halogen, C₁-C₆-alkyl, C₁-C₆-haloalkyl, C₁-C₆-alkoxy, C₁-C₆-haloalkoxy, C(O)OH, C(O)O-C₁-C₆-alkyl, S(O)₂-C₁-C₆-alkyl, S(O)₂NH₂, S(O)₂N(C₁-C₆-alkyl)₂ in which the two alkyl groups are independent from each other;
- R⁷: is selected from the group consisting of C₁-C₆-alkyl, halo-C₁-C₆-alkyl, hydroxy-C₁-C₆-alkyl, C₂-C₆-alkenyl, C₂-C₆-alkynyl, C₁-C₆-alkoxy-C₁-C₆-alkyl, C₃-C₁₀-cyclalkyl, C₃-C₁₀-cycloalkyl-C₁-C₆-alkyl, aryl, aryl-C₁-C₆-alkyl-, heteroaryl, or heteroaryl-C₁-C₆-alkyl- in which said cycloalkyl, aryl, heteroaryl group is optionally substituted up to three times with a halogen, hydroxy, an C₁-C₆-alkyl, C₁-C₆-haloalkyl, C₁-C₆-alkoxy, C₁-C₆-haloalkoxy, C(O)OH, C(O)O-C₁-C₆-alkyl, CN, C(O)NH₂, S(O)₂-C₁-C₆-alkyl, S(O)₂NH₂, S(O)₂N(C₁-C₆-alkyl)₂ in which the two alkyl groups are independent from each other.
- R^{8a}: is selected from the group consisting of hydrogen, hydroxy, halogen, C₁-C₆-alkyl, halo-C₁-C₆-alkyl;
- R^{8b}: is selected from the group consisting of hydrogen, halogen, C₁-C₆-alkyl, halo-C₁-C₆-alkyl; or
- R^{8a} and R^{8b}: together form an oxo group or an oxime group

According to a particular a form of embodiment, a compound according to the present invention comprises a group R¹ being C₃-C₆-cycloalky or C₂-C₆-alkenyl.

According to a further particular a form of embodiment, a compound according to the present invention comprises a group R³ being a C(O)NH(R^{5a}).

A compound according to the invention specifically coprises a group R⁴ being a single group in para or meta position and being selected from the group consisting of halogen, hydroxy, CN, C₁-C₆-alkyl, halo-C₁-C₆-alkyl, C₁-C₆-alkoxy, halo-C₁-C₆-alkoxy, C(O)OH, C(O)O-C₁-C₆-alkyl; more specifically R⁴ is a single group in para or meta position selected from the group consisting of halogen, in particular F, C₁-C₆-alkoxy, C(O)OH, C(O)O-C₁-C₆-alkyl; even more specifically R⁴ is a single group in para position selected from the group consisting of F, C(O)OH, C(O)OCH₃.

According to a particular form of embodiment, a compound of formula **(I)**, **(Ia)** or **(Ib)** the grounps R^{5a} , R^{5b} or R⁶ are selected, independently from one another, from the group consisting of hydrogen, C₃-C₁₀-cycloalkyl-C₁-C₆-alkyl-, aryl, aryl-C₁-C₆-alkyl-, aryl-cyclopropyl, heteroaryl, heteroaryl-C₁-C₆-alkyl-, in which said cycloalkyl, aryl, heteroaryl groups are optionally substituted up to three times with halogen, C₁-C₆-alkyl, C₁-C₆-haloalkyl, C₁-C₆-alkoxy, C₁-C₆-haloalkoxy, C(O)OH, C(O)O-C₁-C₆-alkyl, S(O)₂-C₁-C₆-alkyl, S(O)₂NH₂, S(O)₂N(C₁-C₆-alkyl)₂ in which the two alkyl groups are independent from each other; Furthermore, R^{8a} in a compound according to the present invention is selected from the group consisting of hydrogen, hydroxy, halogen, and R^{8b} is selected from the group consisting of hydrogen, halogen, C₁-C₆-alkyl, halo-C₁-C₆-alkyl; more particularly R^{8a} and R^{8b} together form an oxo group or an oxime group.

In accordance to a further particular form of embodiment of the present invention R⁹ is hydrogen, halogen;

Furthermore, in conjunction with the above or below definitions and embodiments, compounds according to formula **(I)**, are in particular those of formula **(Ia)** in which
- R¹: is C₃-C₁₀-cycloalkyl;
- R⁴: is a fluorine atom in para position;
- R³: is selected from the group consisting of C(O)N(R^{5a})(R^{5b}), C(O)NH(R^{5a}), and N(H)C(O)R⁶
- R^{5a}, R^{5b} or R⁶: are selected, independently from one another, from the group consisting of hydrogen, C₃-C₁₀-cycloalkyl-C₁-C₆-alkyl-, aryl, aryl-C₁-C₆-alkyl-, aryl-cyclopropyl, heteroaryl, heteroaryl-C₁-C₆-alkyl-, in which said cycloalkyl, aryl, heteroaryl groups are optionally substituted up to three times with halogen, C₁-C₆-alkyl, C₁-C₆-haloalkyl, C₁-C₆-alkoxy, C₁-C₆-haloalkoxy, C(O)OH, C(O)O-C₁-C₆-alkyl, S(O)₂-C₁-C₆-alkyl, S(O)₂NH₂, S(O)₂N(C₁-C₆-alkyl)₂ in which the two alkyl groups are independent from each other;
- R^{8a}: is selected from the group consisting of hydrogen, hydroxy, halogen; and
- R^{8b}: is selected from the group consisting of hydrogen, halogen, C₁-C₆-alkyl, halo-C₁-C₆-alkyl;
- R⁹: is a hydrogen.

Furthermore, in conjunction with the above or below definitions and embodiments, compounds according to formula **(I)**, are in particular those of formula **(IIa)** 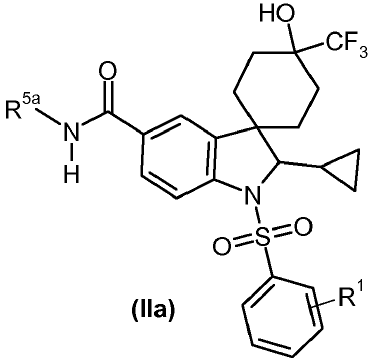 in which
- R¹: single group in para or meta position and is selected from the group consisting of halogen, in particular an F, C₁-C₆-alkoxy, C(O)OH, C(O)O-C₁-C₆-alkyl,
- R^{5a}: is selected from the group consisting of hydrogen, C₃-C₁₀-cycloalkyl-C₁-C₆-alkyl-, aryl, aryl-C₁-C₆-alkyl-, aryl-cyclopropyl, heteroaryl, heteroaryl-C₁-C₆-alkyl-, in which said cycloalkyl, aryl, heteroaryl groups are optionally substituted up to three times with halogen, C₁-C₆-alkyl, C₁-C₆-haloalkyl, C₁-C₆-alkoxy, C₁-C₆-haloalkoxy, C(O)OH, C(O)O-C₁-C₆-alkyl, S(O)₂-C₁-C₆-alkyl, S(O)₂NH₂, S(O)₂N(C₁-C₆-alkyl)₂ in which the two alkyl groups are independent from each other.

Further compounds according to formula **(I)** are as also those of formula **(IIb)** 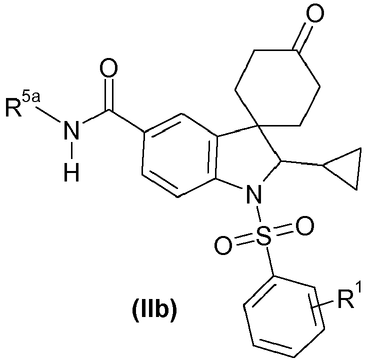 in which
- R¹: single group in para or meta position and is selected from the group consisting of halogen, C₁-C₆-alkoxy, C(O)OH, C(O)O-C₁-C₆-alkyl,
- R^{5a}: is selected from the group consisting of hydrogen, C₃-C₁₀-cycloalkyl-C₁-C₆-alkyl-, aryl, aryl-C₁-C₆-alkyl-, aryl-cyclopropyl, heteroaryl, heteroaryl-C₁-C₆-alkyl-, in which said cycloalkyl, aryl, heteroaryl groups are optionally substituted up to three times with halogen, C₁-C₆-alkyl, C₁-C₆-haloalkyl, C₁-C₆-alkoxy, C₁-C₆-haloalkoxy, C(O)OH, C(O)O-C₁-C₆-alkyl, S(O)₂-C₁-C₆-alkyl, S(O)₂NH₂, S(O)₂N(C₁-C₆-alkyl)₂ in which the two alkyl groups are independent from each other;

A compound of formula **(I)**, **(Ia)**, **(Ib), (IIa)** or **(IIb)** according to the invention specifically coprises a group R^{5a} is selected from the group consisting of C₃-C₁₀-cycloalkyl-C₁-C₆-alkyl-, aryl, aryl-C₁-C₆-alkyl-, aryl-cyclopropyl, heteroaryl, heteroaryl-C₁-C₆-alkyl-, in which said cycloalkyl, aryl, heteroaryl groups are optionally substituted up to three times with a halogen, halo-C₁-C₄-alkyl, or more particularly a group R^{5a} which is selected from the group consisting of C₃-C₁₀-cycloalkyl-C₁-C₆-alkyl-, aryl, aryl-C₁-C₆-alkyl-, aryl-cyclopropyl, heteroaryl, heteroaryl-C₁-C₆-alkyl-, in which said cycloalkyl, aryl, heteroaryl groups are optionally substituted up to three times with a F, Cl, CF₃.

Compounds according to the invention are:
N-[(3-chloropyridin-2-yl)methyl]-2'-cyclopropyl-1'-[(4-fluorophenyl)sulfonyl]-4-hydroxy-4-(trifluoromethyl)-1',2'-dihydrospiro[cyclohexane-1,3'-indole]-5'-carboxamide
N-(2-chlorobenzyl)-2'-cyclopropyl-1'-[(4-fluorophenyl)sulfonyl]-4-hydroxy-1',2'-dihydrospiro[cyclohexane-1,3'-indole]-5'-carboxamide
N-(2-chlorobenzyl)-2'-cyclopropyl-1'-[(4-fluorophenyl)sulfonyl]-4-oxo-1',2'-dihydrospiro[cyclohexane-1,3'-indole]-5'-carboxamide
N-(2-chlorobenzyl)-2'-cyclopropyl-1'-[(4-fluorophenyl)sulfonyl]-4-hydroxy-4-methyl-1',2'-dihydrospiro[cyclohexane-1,3'-indole]-5'-carboxamide
N-(2-chlorobenzyl)-2'-cyclopropyl-1'-[(4-fluorophenyl)sulfonyl]-4-(hydroxyimino)-1',2'-dihydrospiro[cyclohexane-1,3'-indole]-5'-carboxamide
N-(2-chlorobenzyl)-2'-cyclopropyl-4,4-difluoro-1'-[(4-fluorophenyl)sulfonyl]-1',2'-dihydrospiro[cyclohexane-1,3'-indole]-5'-carboxamide
N-{[3-chloro-5-(trifluoromethyl)pyridin-2-yl]methyl}-2'-cyclopropyl-1'-[(4-fluorophenyl)sulfonyl]-4-hydroxy-1',2'-dihydrospiro[cyclohexane-1,3'-indole]-5'-carboxamide
N-{[3-chloro-5-(trifluoromethyl)pyridin-2-yl]methyl}-2'-cyclopropyl-1'-[(4-fluorophenyl)sulfonyl]-4-oxo-1',2'-dihydrospiro[cyclohexane-1,3'-indole]-5'-carboxamide
methyl 3-({5'-[(2-chlorobenzyl)carbamoyl]-2'-cyclopropylspiro[cyclohexane-1,3'-indol]-1'(2'H)-yl}sulfonyl)benzoate
3-({5'-[(2-chlorobenzyl)carbamoyl]-2'-cyclopropylspiro[cyclohexane-1,3'-indol]-1'(2'H)-yl}sulfonyl)benzoic acid
methyl 3-{[5'-{[(3-chloropyridin-2-yl)methyl]carbamoyl}-2'-cyclopropylspiro[cyclohexane-1,3'-indol]-1'(2'H)-yl]sulfonyl}benzoate
1'-[(4-fluorophenyl)sulfonyl]-4-hydroxy-2'-(3-methylbut-2-en-1-yl)-N-(1H-pyrazol-3-yl)-4-(trifluoromethyl)-1',2'-dihydrospiro[cyclohexane-1,3'-indole]-5'-carboxamide
1'-[(4-fluorophenyl)sulfonyl]-4-hydroxy-2'-(3-methylbut-2-en-1-yl)-N-(3-methylphenyl)-4-(trifluoromethyl)-1',2'-dihydrospiro[cyclohexane-1,3'-indole]-5'-carboxamide
3-[({1'-[(4-fluorophenyl)sulfonyl]-4-hydroxy-2'-(3-methylbut-2-en-1-yl)-4-(trifluoromethyl)-1',2'-dihydrospiro[cyclohexane-1,3'-indol]-5'-yl}carbonyl)amino]benzoic acid
1'-[(4-fluorophenyl)sulfonyl]-4-hydroxy-2'-(3-methylbut-2-en-1-yl)-N-[3-(methylsulfonyl)phenyl]-4-(trifluoromethyl)-1',2'-dihydrospiro[cyclohexane-1,3'-indole]-5'-carboxamide
1'-[(4-fluorophenyl)sulfonyl]-4-hydroxy-2'-(3-methylbut-2-en-1-yl)-N-(3-sulfamoylphenyl)-4-(trifluoromethyl)-1',2'-dihydrospiro[cyclohexane-1,3'-indole]-5'-carboxamide
1'-[(4-fluorophenyl)sulfonyl]-4-hydroxy-2'-(3-methylbut-2-en-1-yl)-N-(1,2-oxazol-3-yl)-4-(trifluoromethyl)-1',2'-dihydrospiro[cyclohexane-1,3'-indole]-5'-carboxamide
1'-[(4-fluorophenyl)sulfonyl]-4-hydroxy-2'-(3-methylbut-2-en-1-yl)-N-(5-methylpyridin-3-yl)-4-(trifluoromethyl)-1',2'-dihydrospiro[cyclohexane-1,3'-indole]-5'-carboxamide
1'-[(4-fluorophenyl)sulfonyl]-4-hydroxy-2'-(3-methylbut-2-en-1-yl)-4-(trifluoromethyl)-N-[5-(trifluoromethyl)pyridin-3-yl]-1',2'-dihydrospiro[cyclohexane-1,3'-indole]-5'-carboxamide
1'-[(4-fluorophenyl)sulfonyl]-4-hydroxy-2'-(3-methylbut-2-en-1-yl)-N-(1,3-oxazol-2-yl)-4-(trifluoromethyl)-1',2'-dihydrospiro[cyclohexane-1,3'-indole]-5'-carboxamide
N-(cyclohexylmethyl)-1'-[(4-fluorophenyl)sulfonyl]-4-hydroxy-2'-(3-methylbut-2-en-1-yl)-4-(trifluoromethyl)-1',2'-dihydrospiro[cyclohexane-1,3'-indole]-5'-carboxamide
N-(2-chlorobenzyl)-1'-[(4-fluorophenyl)sulfonyl]-4-hydroxy-2'-(3-methylbut-2-en-1-yl)-4-(trifluoromethyl)-1',2'-dihydrospiro[cyclohexane-1,3'-indole]-5'-carboxamide
1'-[(4-fluorophenyl)sulfonyl]-4-hydroxy-2'-(3-methylbut-2-en-1-yl)-4-(trifluoromethyl)-N-{[3-(trifluoromethyl)pyridin-2-yl]methyl}-1',2'-dihydrospiro[cyclohexane-1,3'-indole]-5'-carboxamide
2'-cyclopropyl-1'-[(4-fluorophenyl)sulfonyl]-4-hydroxy-N-(3-methylphenyl)-4-(trifluoromethyl)-1',2'-dihydrospiro[cyclohexane-1,3'-indole]-5'-carboxamide
2'-cyclopropyl-1'-[(4-fluorophenyl)sulfonyl]-4-hydroxy-N-(5-methylpyridin-3-yl)-4-(trifluoromethyl)-1',2'-dihydrospiro[cyclohexane-1,3'-indole]-5'-carboxamide
N-(2-chlorobenzyl)-1'-[(4-fluorophenyl)sulfonyl]-4-hydroxy-2'-methyl-4-(trifluoromethyl)-1',2'-dihydrospiro[cyclohexane-1,3'-indole]-5'-carboxamide
methyl 3-{[2'-cyclopropyl-4-hydroxy-5'-(1,2-oxazol-3-ylcarbamoyl)-4-(trifluoromethyl)spiro[cyclohexane-1,3'-indol]-1'(2'H)-yl]sulfonyl}benzoate
methyl 3-{[2'-cyclopropyl-5'-({[3-fluoro-5-(trifluoromethyl)pyridin-2-yl]methyl}carbamoyl)-4-hydroxy-4-(trifluoromethyl)spiro[cyclohexane-1,3'-indol]-1'(2'H)-yl]sulfonyl}benzoate
methyl 3-{[2'-cyclopropyl-4-hydroxy-4-(trifluoromethyl)-5'-({[3-(trifluoromethyl)pyridin-2-yl]methyl}carbamoyl)spiro[cyclohexane-1,3'-indol]-1'(2'H)-yl]sulfonyl}benzoate
methyl 3-[({2'-cyclopropyl-1'-[(4-fluorophenyl)sulfonyl]-4-hydroxy-4-(trifluoromethyl)-1',2'-dihydrospiro[cyclohexane-1,3'-indol]-5'-yl}carbonyl)amino]benzoate
methyl 3-[({2'-cyclopropyl-4-hydroxy-1'-[(3-methoxyphenyl)sulfonyl]-4-(trifluoromethyl)-1',2'-dihydrospiro[cyclohexane-1,3'-indol]-5'-yl}carbonyl)amino]benzoate
methyl 3-[({1'-[(4-fluorophenylsulfonyl]-4-hydroxy-2'-(3-methylbut-2-en-1-yl)-4-(trifluoromethyl)-1',2'-dihydrospiro[cyclohexane-1,3'-indol]-5'-yl}carbonyl)amino]benzoate
2'-cyclopropyl-4-hydroxy-1'-[(3-methoxyphenyl)sulfonyl]-N-(1 H-pyrazol-3-yl)-4-(trifluoromethyl)-1',2'-dihydrospiro[cyclohexane-1,3'-indole]-5'-carboxamide
2'-cyclopropyl-4-hydroxy-1'-[(3-methoxyphenyl)sulfonyl]-N-(3-methylphenyl)-4-(trifluoromethyl)-1',2'-dihydrospiro[cyclohexane-1,3'-indole]-5'-carboxamide
3-[({2'-cyclopropyl-4-hydroxy-1'-[(3-methoxyphenyl)sulfonyl]-4-(trifluoromethyl)-1',2'-dihydrospiro[cyclohexane-1,3'-indol]-5'-yl}carbonyl)amino]benzoic acid
2'-cyclopropyl-4-hydroxy-1'-[(3-methoxyphenyl)sulfonyl]-N-[3-(methylsulfonyl)phenyl]-4-(trifluoromethyl)-1',2'-dihydrospiro[cyclohexane-1,3'-indole]-5'-carboxamide
2'-cyclopropyl-4-hydroxy-1'-[(3-methoxyphenyl)sulfonyl]-N-(3-sulfamoylphenyl)-4-(trifluoromethyl)-1',2'-dihydrospiro[cyclohexane-1,3'-indole]-5'-carboxamide
2'-cyclopropyl-4-hydroxy-1'-[(3-methoxyphenyl)sulfonyl]-N-(1,2-oxazol-3-yl)-4-(trifluoromethyl)-1',2'-dihydrospiro[cyclohexane-1,3'-indole]-5'-carboxamide
2'-cyclopropyl-4-hydroxy-1'-[(3-methoxyphenyl)sulfonyl]-N-(5-methylpyridin-3-yl)-4-(trifluoromethyl)-1',2'-dihydrospiro[cyclohexane-1,3'-indole]-5'-carboxamide
2'-cyclopropyl-4-hydroxy-1'-[(3-methoxyphenyl)sulfonyl]-4-(trifluoromethyl)-N-[5-(trifluoromethyl)pyridin-3-yl]-1',2'-dihydrospiro[cyclohexane-1,3'-indole]-5'-carboxamide
2'-cyclopropyl-4-hydroxy-1'-[(3-methoxyphenyl)sulfonyl]-N-(1,3-oxazol-2-yl)-4-(trifluoromethyl)-1',2'-dihydrospiro[cyclohexane-1,3'-indole]-5'-carboxamide
N-(cyclohexylmethyl)-2'-cyclopropyl-4-hydroxy-1'-[(3-methoxyphenyl)sulfonyl]-4-(trifluoromethyl)-1',2'-dihydrospiro[cyclohexane-1,3'-indole]-5'-carboxamide
N-(2-chlorobenzyl)-2'-cyclopropyl-4-hydroxy-1'-[(3-methoxyphenyl)sulfonyl]-4-(trifluoromethyl)-1',2'-dihydrospiro[cyclohexane-1,3'-indole]-5'-carboxamide
2'-cyclopropyl-4-hydroxy-1'-[(3-methoxyphenyl)sulfonyl]-4-(trifluoromethyl)-N-{[3-(trifluoromethyl)pyridin-2-yl]methyl}-1',2'-dihydrospiro[cyclohexane-1,3'-indole]-5'-carboxamide
N-[(3-chloropyridin-2-yl)methyl]-2'-cyclopropyl-4-hydroxy-1'-[(3-methoxyphenyl)sulfonyl]-4-(trifluoromethyl)-1',2'-dihydrospiro[cyclohexane-1,3'-indole]-5'-carboxamide
2'-cyclopropyl-1'-[(4-fluorophenyl)sulfonyl]-4-hydroxy-N-[3-(methylsulfonyl)phenyl]-4-(trifluoromethyl)-1',2'-dihydrospiro[cyclohexane-1,3'-indole]-5'-carboxamide
2'-cyclopropyl-1'-[(4-fluorophenyl)sulfonyl]-4-hydroxy-N-(3-sulfamoylphenyl)-4-(trifluoromethyl)-1',2'-dihydrospiro[cyclohexane-1,3'-indole]-5'-carboxamide
3-{[2'-cyclopropyl-4-hydroxy-5'-(1,2-oxazol-3-ylcarbamoyl)-4-(trifluoromethyl)spiro[cyclohexane-1,3'-indol]-1'(2'H)-yl]sulfonyl}benzoic acid
3-{[2'-cyclopropyl-5'-({[3-fluoro-5-(trifluoromethyl)pyridin-2-yl]methyl}carbamoyl)-4-hydroxy-4-(trifluoromethyl)spiro[cyclohexane-1,3'-indol]-1'(2'H)-yl]sulfonyl}benzoic acid
3-{[2'-cyclopropyl-4-hydroxy-4-(trifluoromethyl)-5'-({[3-(trifluoromethyl)pyridin-2-yl]methyl}carbamoyl)spiro[cyclohexane-1,3'-indol]-1'(2'H)-yl]sulfonyl}benzoic acid
N-[1-(4-chlorophenyl)cyclopropyl]-2'-cyclopropyl-1'-[(4-fluorophenyl)sulfonyl]-4-hydroxy-4-(trifluoromethyl)-1',2'-dihydrospiro[cyclohexane-1,3'-indole]-5'-carboxamide
2'-cyclopropyl-1'-[(4-fluorophenyl)sulfonyl]-4-hydroxy-N-(1H-pyrazol-3-yl)-4-(trifluoromethyl)-1',2'-dihydrospiro[cyclohexane-1,3'-indole]-5'-carboxamide
2'-cyclopropyl-N-(2,3-dihydro-1H-inden-1-yl)-1'-[(4-fluorophenyl)sulfonyl]-4-hydroxy-4-(trifluoromethyl)-1',2'-dihydrospiro[cyclohexane-1,3'-indole]-5'-carboxamide
2'-cyclopropyl-1'-[(4-fluorophenyl)sulfonyl]-4-hydroxy-4-(trifluoromethyl)-N-[3-(trifluoromethyl)phenyl]-1',2'-dihydrospiro[cyclohexane-1,3'-indole]-5'-carboxamide
N-(3-chlorophenyl)-2'-cyclopropyl-1'-[(4-fluorophenyl)sulfonyl]-4-hydroxy-4-(trifluoromethyl)-1',2'-dihydrospiro[cyclohexane-1,3'-indole]-5'-carboxamide
2'-cyclopropyl-1'-[(4-fluorophenyl)sulfonyl]-4-hydroxy-N-(1,2-oxazol-3-yl)-4-(trifluoromethyl)-1',2'-dihydrospiro[cyclohexane-1,3'-indole]-5'-carboxamide
1'-{[3-(azetidin-1-ylcarbonyl)phenyl]sulfonyl}-2'-cyclopropyl-4-hydroxy-N-(1,2-oxazol-3-yl)-4-(trifluoromethyl)-1',2'-dihydrospiro[cyclohexane-1,3'-indole]-5'-carboxamide
2'-cyclopropyl-1'-[(4-fluorophenyl)sulfonyl]-4-hydroxy-4-(trifluoromethyl)-N-[5-(trifluoromethyl)pyridin-3-yl]-1',2'-dihydrospiro[cyclohexane-1,3'-indole]-5'-carboxamide
2'-cyclopropyl-N-(cyclopropylmethyl)-1'-[(4-fluorophenyl)sulfonyl]-4-hydroxy-4-(trifluoromethyl)-1',2'-dihydrospiro[cyclohexane-1,3'-indole]-5'-carboxamide
N-(2-chloro-4-fluorobenzyl)-2'-cyclopropyl-1'-[(4-fluorophenyl)sulfonyl]-4-hydroxy-4-(trifluoromethyl)-1',2'-dihydrospiro[cyclohexane-1,3'-indole]-5'-carboxamide
N-(cyclohexylmethyl)-2'-cyclopropyl-1'-[(4-fluorophenyl)sulfonyl]-4-hydroxy-4-(trifluoromethyl)-1',2'-dihydrospiro[cyclohexane-1,3'-indole]-5'-carboxamide
N-(2-chlorobenzyl)-2'-cyclopropyl-1'-[(4-fluorophenyl)sulfonyl]-4-hydroxy-4-(trifluoromethyl)-1',2'-dihydrospiro[cyclohexane-1,3'-indole]-5'-carboxamide
1'-{[3-(azetidin-1-ylcarbonyl)phenyl]sulfonyl)-2'-cyclopropyl-N-{[3-fluoro-5-(trifluoromethyl)pyridin-2-yl]methyl}-4-hydroxy-4-(trifluoromethyl)-1',2'-dihydrospiro[cyclohexane-1,3'-indole]-5'-carboxamide
N-[(3-chloro-5-fluoropyridin-2-yl)methyl]-2'-cyclopropyl-1'-[(4-fluorophenyl)sulfonyl]-4-hydroxy-4-(trifluoromethyl)-1',2'-dihydrospiro[cyclohexane-1,3'-indole]-5'-carboxamide
2'-cyclopropyl-1'-[(4-fluorophenyl)sulfonyl]-4-hydroxy-4-(trifluoromethyl)-N-{[3-(trifluoromethyl)pyridin-2-yl]methyl}-1',2'-dihydrospiro[cyclohexane-1,3'-indole]-5'-carboxamide
1'-{[3-(azetidin-1-ylcarbonyl)phenyl]sulfonyl)-2'-cyclopropyl-4-hydroxy-4-(trifluoromethyl)-N-{[3-(trifluoromethyl)pyridin-2-yl]methyl}-1',2'-dihydrospiro[cyclohexane-1,3'-indole]-5'-carboxamide

Another embodiment of the present invention provides compounds according to general formula **(I)**, **(Ia)**, **(Ib)**, **(IIa)** or **(IIb)** and related specific embodiments for use as a medicament.

In another embodiment, the present invention provides a method of treating GnRH related disorder in a patient in need of such treatment, comprising administering to the patient an effective amount of a compound according to the invention as defined above.

In still another aspect, the invention provides use of a compound according to the invention as defined above for manufacturing a pharmaceutical composition for the treatment or prevention of GnRH related disorders.

The term "treating" or "treatment" as stated throughout this document is used conventionally, e.g., the management or care of a subject for the purpose of combating, alleviating, reducing, relieving, improving the condition of a disease or disorder, such as for example endometriosis and uterine fibroids.

The term "subject" or "patient" includes organisms which are capable of suffering from a disorder or who could otherwise benefit from the administration of a compound of the invention, such as human and non-human animals. Preferred humans include human patients suffering from or prone to suffering from disorders, such as for example endometriosis and uterine fibroids. The term "non-human animals" includes vertebrates, e.g., mammals, such as non-human primates, sheep, cows, dogs, cats and rodents, e.g., mice, and non-mammals, such as chickens, amphibians, reptiles, etc.

In another aspect, the invention provides a pharmaceutical composition comprising a compound according to the invention, together with a pharmaceutically acceptable carrier.

In still another aspect, the invention provides a process for preparing a pharmaceutical composition. The process includes the step of combining at least one compound according to the invention as defined above with at least one pharmaceutically acceptable carrier, and bringing the resulting combination into a suitable administration form.

The compounds according to general formula **(I)**, **(Ia)**, **(Ib), (IIa)** or **(IIb)** are used as a medicament. In particular, said compounds are used to treat sexual hormone-related conditions in both men and women, as well as a mammal in general (also referred to herein as a "subject"). For example, such conditions include endometriosis, uterine fibroids, polycystic ovarian disease, hirsutism, precocious puberty, gonadal steroid-dependent neoplasia such as cancers of the prostate, breast and ovary, gonadotrope pituitary adenomas, sleep apnea, irritable bowel syndrome, premenstrual syndrome, benign prostatic hypertrophy, and infertility (e.g., assisted reproductive therapy such as in vitro fertilization).

The compounds according to general formula **(I)**, **(Ia)**, **(Ib), (IIa)** or **(IIb)** are further used as contraceptive.

The compounds of this invention are also useful as an adjunct to treatment of growth hormone deficiency and short stature, and for the treatment of systemic lupus erythematosus.

According to a further embodiment of the present invention the compounds according to general formula **(I)**, **(Ia)**, **(Ib), (IIa)** or **(IIb)** are also useful and can be used in combination with androgens, estrogens, progestins, SERMs, antiestrogens and antiprogestins for the treatment of endometriosis, uterine fibroids, and in contraception, as well as in combination with an angiotensin-converting enzyme inhibitor, an angiotensin II-receptor antagonist, or a renin inhibitor for the treatment of uterine fibroids.

A combination of compounds according to general formula **(I)**, **(Ia)**, **(Ib), (IIa)** or **(IIb)** with bisphosphonates and other agents for the treatment and/or prevention of disturbances of calcium, phosphate and bone metabolism, and in combination with estrogens, SERMs, progestins and/or androgens for the prevention or treatment of bone loss or hypogonadal symptoms such as hot flushes during therapy with a GnRH antagonist is also part of the present invention.

The methods of this invention include administering an effective amount of a GnRH receptor antagonist, preferably in the form of a pharmaceutical composition, to a mammal in need thereof. Thus, in still a further embodiment, pharmaceutical compositions are disclosed containing one or more GnRH receptor antagonists of this invention in combination with a pharmaceutically acceptable carrier and/or diluent.

These and other aspects of the invention will be apparent upon reference to the following detailed description. To this end, various references are set forth herein which describe in more detail certain background information, procedures, compounds and/or compositions, and are each hereby incorporated by reference in their entirety.

The compounds of the present invention may generally be utilized as the free acid or free base. Alternatively, the compounds of this invention may be used in the form of acid or base addition salts.

Thus, the term "pharmaceutically acceptable salt" of compounds of general formula **(I)**, **(Ia)**, **(Ib), (IIa)** or **(IIb)** is intended to encompass any and all acceptable salt forms.

In addition, prodrugs are also included within the context of this invention. Prodrugs are any covalently bonded carriers that release a compound of general formula **(I)**, **(Ia)**, **(Ib)**, **(IIa)** or **(IIb)** *in vivo* when such prodrug is administered to a patient. Prodrugs are generally prepared by modifying functional groups in a way such that the modification is cleaved, either by routine manipulation or *in vivo*, yielding the parent compound.
Prodrugs include, for example, compounds of this invention wherein hydroxy, amine or sulfhydryl groups are bonded to any group that, when administered to a patient, cleaves to form the hydroxy, amine or sulfhydryl groups. Thus, representative examples of prodrugs include (but are not limited to) acetate, formate and benzoate derivatives of alcohol and amine functional groups of the compounds of general formula **(I)**, **(Ia)**, **(Ib), (IIa)** or **(IIb)**. Further, in the case of a carboxylic acid (-COOH), esters may be employed, such as methyl esters, ethyl esters, and the like.

With regard to stereoisomers, the compounds of general formula **(I)**, **(Ia)**, **(Ib), (IIa)** or **(IIb)** may have chiral centers and may occur as racemates, racemic mixtures and as individual enantiomers or diastereomers. All such isomeric forms are included within the present invention, including mixtures thereof. Furthermore, some of the crystalline forms of the compounds of general formula **(I)**, **(Ia)**, **(Ib), (IIa)** or **(IIb)** may exist as polymorphs, which are included in the present invention. In addition, some of the compounds of general formula **(I)**, **(Ia)**, **(Ib), (IIa)** or **(IIb)** may also form solvates with water or other organic solvents. Such solvates are similarly included within the scope of this invention.

The effectiveness of a compound as a GnRH receptor antagonist may be determined by various assay techniques. Assay techniques well known in the field include the use of cultured pituitary cells for measuring GnRH activity (Vale et al., Endocrinology 1972, 91, 562 - 572) and the measurement of radioligand binding to rat pituitary membranes (Perrin et al., Mol. Pharmacol. 1983, 23, 44 - 51) or to membranes from cells expressing cloned receptors as described below. Other assay techniques include (but are not limited to) measurement of the effects of GnRH receptor antagonists on the inhibition of GnRH-stimulated calcium flux, modulation of phosphoinositol hydrolysis, and the circulating concentrations of gonadotropins in the castrate animal. Descriptions of these techniques, the synthesis of radiolabeled ligand, the employment of radiolabeled ligand in radioimmunoassay, and the measurement of the effectiveness of a compound as a GnRH receptor antagonist follow.

In another embodiment of the invention, pharmaceutical compositions containing one or more GnRH receptor antagonists are disclosed. For the purposes of administration, the compounds of the present invention may be formulated as pharmaceutical compositions.

Pharmaceutical compositions of the present invention comprise a GnRH receptor antagonist of the present invention and a pharmaceutically acceptable carrier and/or diluent. The GnRH receptor antagonist is present in the composition in an amount which is effective to treat a particular disorder that is, in an amount sufficient to achieve GnRH receptor antagonist activity, and preferably with acceptable toxicity to the patient. Typically, the pharmaceutical compositions of the present invention may include a GnRH receptor antagonist in an amount from 0.1 mg to 500 mg per day dosage depending upon the route of administration, and more typically from 0.5 mg to 150 mg per day. Appropriate concentrations and dosages can be readily determined by one skilled in the art.

Determination of a therapeutically effective amount or a prophylactically effective amount of the compounds of the invention can be readily made by the physician or veterinarian (the "attending clinician"), as one skilled in the art, by the use of known techniques and by observing results obtained under analogous circumstances. The dosages may be varied depending upon the requirements of the patient in the judgment of the attending clinician; the severity of the condition being treated and the particular compound being employed. In determining the therapeutically effective amount or dose, and the prophylactically effective amount or dose, a number of factors are considered by the attending clinician, including, but not limited to: the specific GnRH mediated disorder involved; pharmacodynamic characteristics of the particular agent and its mode and route of administration; the desired time course of treatment; the species of mammal; its size, age, and general health; the specific disease involved; the degree of or involvement or the severity of the disease; the response of the individual patient; the particular compound administered; the mode of administration; the bioavailability characteristics of the preparation administered; the dose regimen selected; the kind of concurrent treatment (i.e., the interaction of the compound of the invention with other coadministered therapeutics); and other relevant circumstances.

Treatment can be initiated with smaller dosages, which are less than the optimum dose of the compound. Thereafter, the dosage may be increased by small increments until the optimum effect under the circumstances is reached. For convenience, the total daily dosage may be divided and administered in portions during the day if desired.

Pharmaceutically acceptable carrier and/or diluents are familiar to those skilled in the art. For compositions formulated as liquid solutions, acceptable carriers and/or diluents include saline and sterile water, and may optionally include antioxidants, buffers, bacteriostats and other common additives. The compositions can also be formulated as pills, capsules, granules, or tablets which contain, in addition to a GnRH receptor antagonist, diluents, dispersing and surface active agents, binders, and lubricants. One skilled in this art may further formulate the GnRH receptor antagonist in an appropriate manner, and in accordance with accepted practices, such as those disclosed in Remington's Pharmaceutical Sciences, Gennaro, Ed., Mack Publishing Co., Easton, PA 1990.

In another embodiment, the present invention provides a method for treating sex-hormone-related conditions as discussed above. Such methods include administering of a compound of the present invention to a warm-blooded animal in an amount sufficient to treat the condition. In this context, "treat" includes prophylactic administration. Such methods include systemic administration of a GnRH receptor antagonist of this invention, preferably in the form of a pharmaceutical composition as discussed above. As used herein, systemic administration includes oral and parenteral methods of administration. For oral administration, suitable pharmaceutical compositions of GnRH receptor antagonists include powders, granules, pills, tablets, and capsules as well as liquids, syrups, suspensions, and emulsions. These compositions may also include flavorants, preservatives, suspending, thickening and emulsifying agents, and other pharmaceutically acceptable additives. For parenteral administration, the compounds of the present invention can be prepared in aqueous injection solutions which may contain, in addition to the GnRH receptor antagonist, buffers, antioxidants, bacteriostats, and other additives commonly employed in such solutions.

### MODE(S) FOR CARRYING OUT THE INVENTION

The following examples are provided for purposes of illustration, not limitation. In summary, the GnRH receptor antagonists of this invention may be assayed by the general methods disclosed above, while the following examples disclose the synthesis of representative compounds of this invention.

### EXPERIMENTAL DETAILS AND GENERAL PROCESSES

The following table lists the abbreviations used in this paragraph and in the examples section as far as they are not explained within the text body.

| **Abbreviation** | **Meaning** |
|---|---|
| Ac | acetyl |
| aq. | aqueous |
| BOC | *tert*-butyloxycarbonyl |
| br. s. | broad singlet |
| d | doublet |
| dbr | broad doublet |
| dd | doublet of doublets |
| dt | doublet of triplets |
| DCM | dichloromethane |
| DIPEA | *N,N*-diisopropylethylamine |
| DMF | *N,N*-dimethylformamide |
| DMSO | dimethyl sulfoxide |
| eq. | equivalent(s) |
| ESI | electrospray ionization |
| HATU | 2-(7-aza-1 H-benzotriazole-1-yl)-1,1,3,3-tetramethyluronium hexafluorophosphate) |
| HOAt | 1-hydroxy-7-azabenzotriazole |
| HPLC | high performance liquid chromatography |
| LCMS | liquid chromatography mass spectrometry |
| LDA | lithium diisopropylamide |
| m | multiplet |
| mc | centred multiplet |
| MS | mass spectrometry |
| NMR | nuclear magnetic resonance spectroscopy: chemical shifts (δ) are given in ppm |
| q | quartet |
| qbr | broad quartet |
| Rt | retention time |
| r.t. or rt or room temp. | room temperature |
| s | singlet |
| sat. | saturated |
| t | triplet |
| TBAF | tetrabutylammonium fluoride |
| TEA | triethylamine |
| TLC | thin layer chromatography |
| TFA | trifluoroacetic acid |
| THF | tetrahydrofuran |
| UPLC | ultra performance liquid chromatography |
| UPLC-MS | ultra performance liquid chromatography - mass spectrometry |

NMR peak forms are stated as they appear in the spectra, possible higher order effects have not been considered. Chemical shifts are given in ppm; all spectra were calibrated to solvent residual peak. Integrals are given as integers.

Ultra performance liquid chromatography / liquid chromatography mass spectrometry - methods:
The terms "UPLC-MS (ESI+)" or "UPLC-MS (ESI-)" refer to the following conditions: Instrument: Waters Acquity UPLC-MS SQD 3001; column: Acquity UPLC BEH C18 1.7 50x2.1 mm; eluent A: water + 0.1 % vol. formic acid (99%), eluent B: acetonitrile; gradient: 0-1.6 min 1-99% B, 1.6-2.0 min 99% B; flow 0.8 mL/min; temperature: 60 °C; injection: 2 µl; DAD scan: 210-400 nm; ELSD; or
Instrument: Waters Acquity UPLC-MS SQD 3001; column: Acquity UPLC BEH C18 1.7 50x2.1 mm; eluent A: water + 0.05% vol. formic acid (98%), eluent B: acetonitrile + 0.05% vol. formic acid (98%); gradient: 0-1.6 min 1-99% B, 1.6-2.0 min 99% B; flow 0.8 mL/min; temperature: 60 °C; injection: 2 µl;DAD scan: 210-400 nm; ELSD; or
Instrument: Waters Acquity UPLC-MS SQD 3001; column: Acquity UPLC BEH C18 1.7 50x2.1 mm; Eluent A: water + 0.2% vol. ammonia (32%), eluent B: acetonitrile; gradient: 0-1.6 min 1-99% B, 1.6-2.0 min 99% B; flow 0.8 mL/min; temperature: 60 °C; injection: 2 µl; DAD scan: 210-400 nm; ELSD

Chemical names were generated according to the IUPAC rules [ACD/Name Batch ver. 12.00] or using AutoNom2000 as implemented in MDL ISIS Draw [MDL Information Systems Inc. (Elsevier MDL)]. In some cases generally accepted names of commercially available reagents were used in place of IUPAC names or AutoNom2000 generated names. Stereodescriptors are used according to Chemical Abstracts.

Reactions employing microwave irradiation may be run with a Biotage Initiator^{®} microwave oven optionally equipped with a robotic unit. The reported reaction times employing microwave heating are intended to be understood as fixed reaction times after reaching the indicated reaction temperature.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be stirred out using a suitable solvent. In some cases, the compounds may be purified by chromatography, particularly flash column chromatography, using for example prepacked silica gel cartridges, e.g. Biotage SNAP cartidges KP-Sil^{®} or KP-NH^{®} in combination with a Biotage autopurifier system (SP4^{®} or Isolera Four^{®}) and eluents such as gradients of hexane/ethyl acetate or DCM/methanol. In some cases, the compounds may be purified by preparative HPLC using for example a Waters autopurifier equipped with a diode array detector and/or on-line electrospray ionization mass spectrometer in combination with a suitable prepacked reverse phase column and eluents such as gradients of water and acetonitrile which may contain additives such as trifluoroacetic acid, formic acid or aqueous ammonia.

In some cases, purification methods as described above can provide those compounds of the present invention which possess a sufficiently basic or acidic functionality in the form of a salt, such as, in the case of a compound of the present invention which is sufficiently basic, a trifluoroacetate or formate salt for example, or, in the case of a compound of the present invention which is sufficiently acidic, an ammonium salt for example. A salt of this type can either be transformed into its free base or free acid form, respectively, by various methods known to the persion skilled in the art, or be used as salts in subsequent biological assays. It is to be understood that the specific form (e.g. salt, free base etc.) of a compound of the present invention as isolated and as described herein is not necessarily the only form in which said compound can be applied to a biological assay in order to quantify the specific biological activity.

The following scheme and general procedures illustrate general synthetic routes to the compounds of general formula (I) of the invention and are not intended to be limiting. It is obvious to the person skilled in the art that the order of transformations as exemplified in Scheme 1 can be modified in various ways. The order of transformations exemplified in Scheme 1 is therefore not intended to be limiting. In addition, interconversion of substituents, for example of residues R¹, R², R³, R⁴, R⁵, R^{5a} and R^{5b} can be achieved before and/or after the exemplified transformations. These modifications can be such as the introduction of protecting groups, cleavage of protecting groups, reduction or oxidation of functional groups, halogenation, metallation, substitution or other reactions known to the person skilled in the art. These transformations include those which introduce a functionality which allows for further interconversion of substituents. Appropriate protecting groups and their introduction and cleavage are well-known to the person skilled in the art (see for example T.W. Greene and P.G.M. Wuts, Protective Groups in Organic Synthesis, 3rd edition, Wiley 1999).

Compounds of general formula **8** can be synthesized according to the procedures depicted in Scheme **1** from suitably functionalized carboxylic acids of formula **7** by reaction with appropriate amines HN(R^{5a})(R^{5b}) **10**. For amide formation, however, all processes that are known from peptide chemistry to the person skilled in the art may be applied. The acids of general formula **7** can be reacted with an appropriate amine in aprotic polar solvents, such as for example DMF, acetonitrile or *N*-methylpyrrolid-2-one via an activated acid derivative, which is obtainable for example with hydroxybenzotriazole and a carbodiimide such as for example diisopropylcarbodiimide, or else with preformed reagents, such as for example O-(7-azabenzotriazol-1-yl)-1,1,3,3-tetramethyluronium hexafluorophosphate (see for example Chem. Comm. 1994, 201 - 203), or else with activating agents such as dicyclohexylcarbodiimide / *N,N*-dimethylaminopyridine or *N*-ethyl-*N*',*N*'-dimethylaminopropylcarbodiimide / *N,N*-dimethylaminopyridine. The addition of a suitable base such as for example N-methylmorpholine, TEA or DIPEA may be necessary. In certain cases, the activated acid derivative might be isolated prior to the reaction with the amine. Amide formation may also be accomplished via the acid halide (which can be formed from a carboxylic acid by reaction with e.g. oxalyl chloride, thionyl chloride or sulfuryl chloride), mixed acid anhydride (which can be formed from a carboxylic acid by reaction with e.g. isobutylchloroformate), imidazolide (which can be formed from a carboxylic acid by reaction with e.g. carbonyldiimidazole) or azide (which can be formed from a carboxylic acid by reaction with e.g. diphenylphosphorylazide).

Carboxylic acids of general formula **7** in turn can be obtained from carboxylic esters of formula **6** by saponification with inorganic bases such as lithium hydroxide, potassium hydroxide or sodium hydroxide in a suitable solvent such as methanol, THF, water or mixtures thereof at temperatures between 0°C and the boiling point of the solvent(mixture), typically at room temperature. Alternatively, carboxylic acids of general formula **7** may be directly formed from aryl bromides of general formula **5** under palladium catalyzed carbonylation conditions. Thus, bromides of formula **5** can be reacted in a suitable solvent such as for example dimethyl sulfoxide in the presence of a carbon monoxide source such as for example molybdenum hexacarbonyl or under a carbon monoxide atmosphere at pressures between 1 and 20 bar and in the presence of a palladium catalyst system such as for example palladium(II) acetate / 1,1'-bis(diphenylphosphino)ferrocene and a base such as potassium acetate at temperatures between room temperature and the boiling point of the solvent, preferably at 100°C.

Carboxylic esters of general formula **6** can be synthesized from aryl bromides of formula **5** by reaction with an appropriate alcohol under palladium catalyzed carbonylation conditions. Bromides of formula **5** might be reacted in a polar aprotic solvent such as for example dimethylsulfoxide with an appropriate alcohol such as methanol in the presence of a carbon monoxide source such as for example molybdenum hexacarbonyl or under a carbon monoxide atmosphere at pressures between 1 and 20 bar and in the presence of a suitable palladium catalyst such as bis(triphenylphosphine) palladium (II) dichloride and a base such as for example triethylamine at temperatures between room temperature and the boiling point of the solvent, preferably at 100°C.

Alternatively, amides of general formula **8** may be directly synthesized from aryl bromides of formula **5** by reaction with appropriate amines HN(R^{5a})(R^{5b}) **10** under palladium catalyzed carbonylation conditions. For this carbonylation all processes that are known to the person skilled in the art may be applied. Bromides of formula **5** can be reacted in a polar aprotic solvent such as for example dioxane with an appropriate amine in the presence of a carbon monoxide source such as for example molybdenum hexacarbonyl or under a carbon monoxide atmosphere at pressures between 1 and 20 bar and in the presence of a palladium catalyst such as for example palladium(II) acetate and a base such as sodium carbonate at temperatures between room temperature and the boiling point of the solvent, preferably at 110°C. It might be necessary to add a ligand such as tri-tert-butylphosphonium tetrafluoroborate to the mixture.

Sulfonamides of general formula **5** in turn can be formed from indolines of general formula **4** by reaction with electrophiles of formula R²-SO₂-Cl in an organic solvent such as dichloromethane, 1,2-dichloroethane or acetonitrile in the presence of a tertiary amine base such as triethylamine or DIPEA and optionally in the presence of 4-dimethylaminopyridine at temperatures between room temperature and the boiling point of the solvent, typically at 80°C. Alternatively, indolines of general formula **4** may be reacted with electrophiles of formula R²-SO₂-Cl without additional solvent in the presence of a tertiary base such as triethylamine or pyridine at room temperature to give sulfonamides of general formula **5.** In the above procedures, electrophiles R²-SO₂-Cl are either commercially available, known compounds or may be formed from known compounds by known methods by a person skilled in the art.

Indolines of general formula **4** can be synthesized from suitably functionalized indolenines of general formula **3** by either reduction or addition of a nucleophile. For reduction, the indolenines **3** can be reacted in a suitable organic solvent such as for example methanol in the presence of a reducing agent such as for example sodium borohydride, sodium (triacetoxy)borohydride or sodium cyanoborohydride at temperatures between 0°C and the boiling point of the solvent, typically at room temperature. In case of a nucleophilic addition, the indolenines **3** can be reacted in a suitable organic solvent such as for example THF with a nucleophile R¹-M (where M is a metallic species; R¹-M is for example a Grignard reagent) at temperatures between 0°C and the boiling point of the solvent, typically at room temperature (see WO06/090261, pp. 67-68 for a similar procedure). It might be necessary to add a Lewis acid such as boron trifluoride diethyl etherate to the mixture.

Alternatively, **3** can be reacted in a suitable organic solvent such as for example toluene with a Grignard reagent R¹-M in the presence of copper(I) chloride at temperatures between room temperature and the boiling point of the solvent, typically at 120°C to give indolines of general formula **4** (see J. Chem. Soc. Perkin Trans. 1, 1988, 3243-3247).

Indolenines of general formula **3** can be obtained from suitably functionalized enol ethers of general formula **2** and a phenylhydrazine of formula **9** by condensation to give a hydrazone intermediate and a subsequent cyclization reaction (Fischer indole synthesis) in an organic solvent such as for example chloroform or acetic acid and in the presence of a suitable acid such as for example trifluoroacetic acid or hydrochloric acid at temperatures between 0°C and the boiling point of the solvent (see for example Liu et al., Tetrahedron 2010, 66, 3, 573-577 or WO10/151737, p. 224 for similar procedures).

Enol ethers of general formula **2**, which can also be obtained from ketones of general formula **1** and phenylhydrazines of general formula **9**, are either commercially available, known compounds or may be formed from known compounds by known methods by a person skilled in the art.

The synthesis of similar compounds is also described in WO 2013/107743.

The obtained indolines of general formula **8** may be chiral and their diastereomers and/or enantiomers can be separated by chiral HPLC.

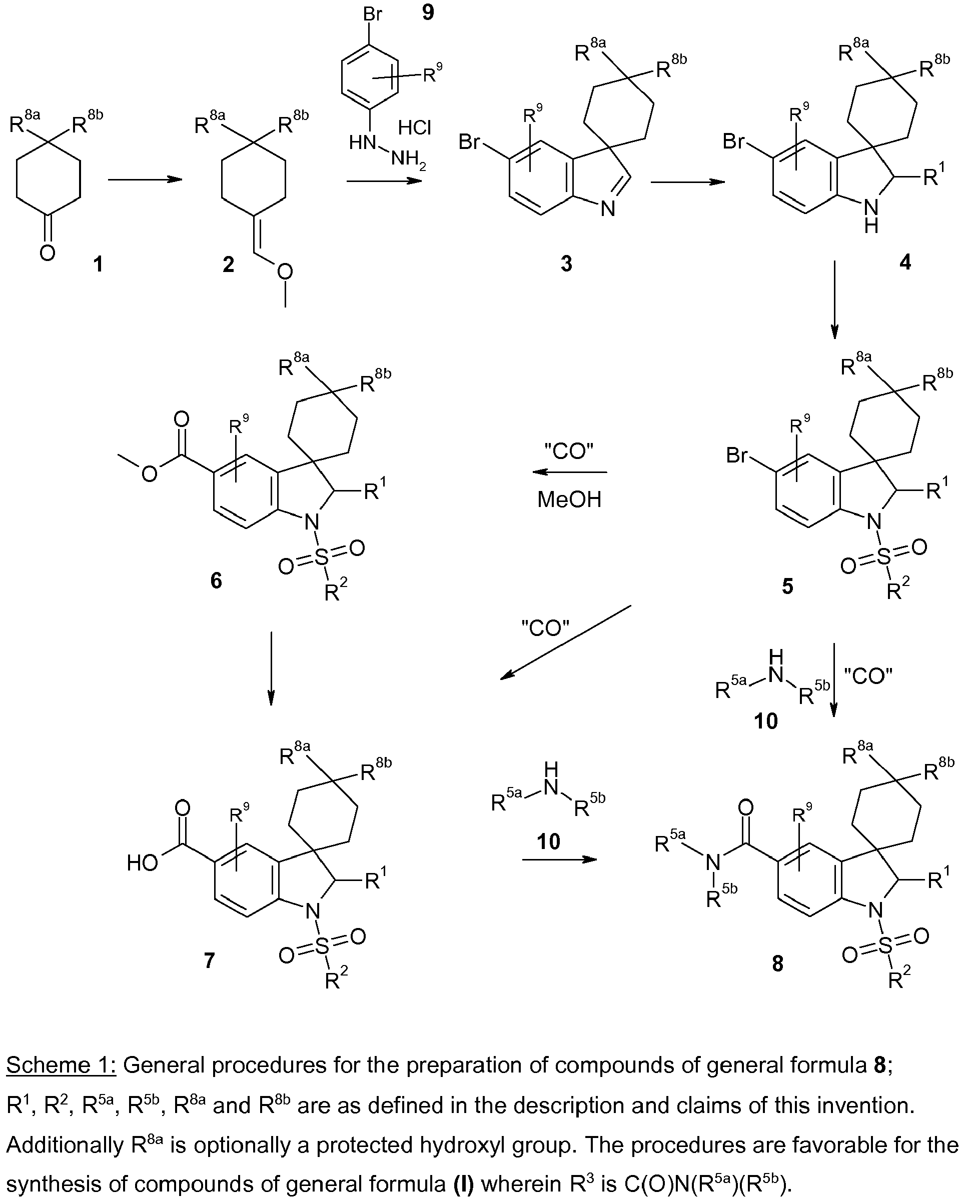

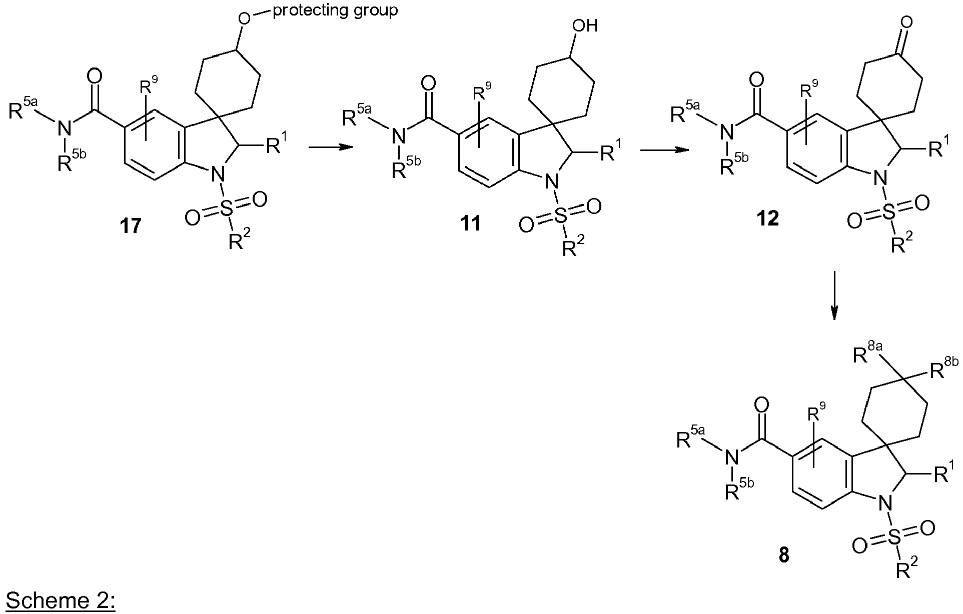

If R^{8a} is a protected hydroxyl group, the compounds of general formula **17** can be used to prepare hydroxy compounds of general formula **11** by cleavage of the protecting group. The compounds of general formula **11** can be oxidized to carbonyl compounds of general formula **12**. The carbonyl group in the compounds of general formula **12** can be modified e.g. by halogenation, alkylation, oxime formation etc.

The above transformations can be done by known methods by a person skilled in the art (see examples 2 - 6 and 8).

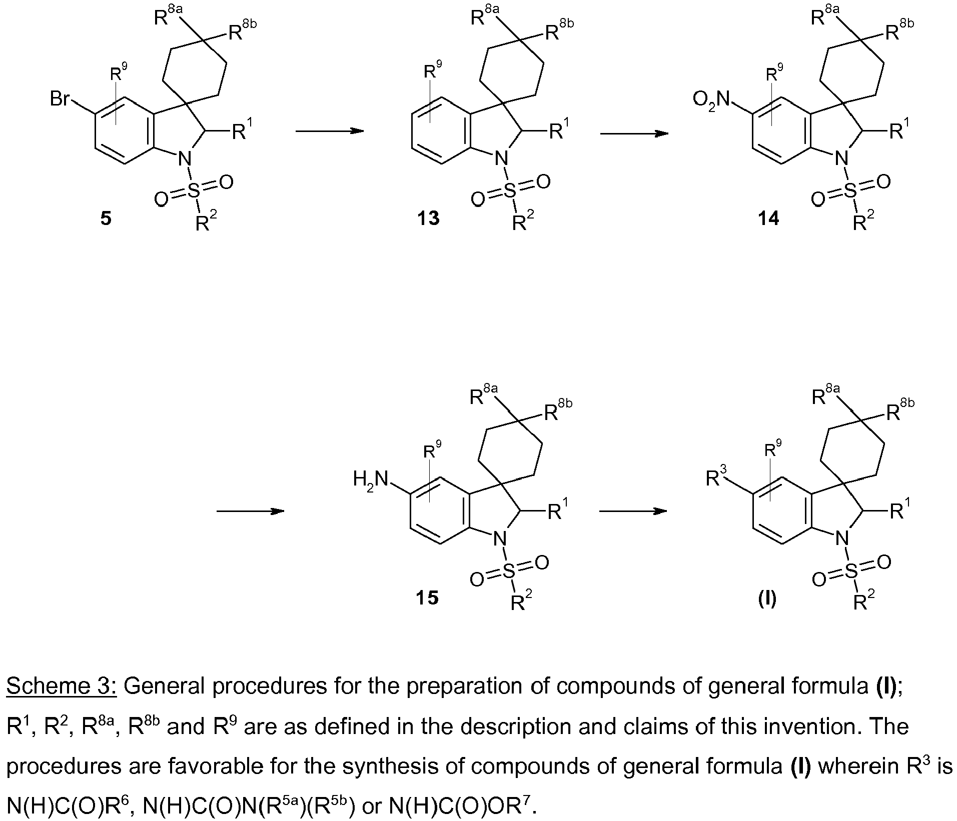

Compounds of general formula **(I)** wherein R³ is N(H)C(O)R⁶, N(H)C(O)N(R⁵a)(R^{5b}) or N(H)C(O)OR⁷ can be synthesized according to the procedures depicted in Scheme 3 from suitably functionalized anilines of general formula **15** by reaction with electrophiles. Thus, anilines of formula **15** can be reacted with appropriate carboxylic acids to form amides of general formula **(I)** wherein R³ is N(H)C(O)R⁶. For amide formation, however, all processes that are known from peptide chemistry to the person skilled in the art may be applied (see the synthesis of compounds of formula **8** in Scheme 1).

Furthermore, anilines of general formula **15** can be reacted with appropriate isocyanates in a suitable organic solvent such as for example DMF and optionally in the presence of a tertiary amine base such as triethylamine or DIPEA at temperatures between 0°C and the boiling point of the solvent to form ureas of general formula **(I)** wherein R³ is N(H)C(O)N(R⁵a)(R^{5b}). Additionally, anilines of general formula **15** can be reacted with appropriate chloroformates or 4-nitrophenylcarbonates in a suitable organic solvent such as for example THF and in the presence of a tertiary amine base such as triethylamine or DIPEA at temperatures between 0°C and the boiling point of the solvent to form carbamates of general formula **(I)** wherein R³ is N(H)C(O)OR⁷.

Anilines of general formula **15** can be obtained from nitroarenes of general formula **14** by reduction. For reduction, all processes that are known to the person skilled in the art can be applied. Nitroarenes **14** can be hydrogenated under an atmosphere of hydrogen at pressures between 1 bar and 100 bar in a suitable solvent such as for example ethyl acetate, methanol or ethanol or by passing hydrogen into the solution in the presence of a metal catalyst such as for example palladium on charcoal at temperatures between 0°C and the boiling point of the solvent, typically at room temperature. The addition of a suitable acid such as for example hydrochloric acid or acetic acid may be necessary.

Nitroarenes of general formula **14** can be synthesized from compounds of general formula **13** by regioselective nitration. For nitration, all processes that are known to the person skilled in the art may be applied. Compounds of formula **13** can be reacted with a mixture of concentrated nitric acid and sulfuric acid or with a mixture of concentrated nitric acid and acetic acid at temperatures between 0°C and the boiling point of the solvent, typically at room temperature.

Compounds of general formula **13** can be obtained from aryl bromides of general formula **5** by dehalogenation methods which are known by a person skilled in the art. For example, the bromides of formula **5** can be hydrogenated under an atmosphere of hydrogen at pressures between 1 bar and 100 bar in a suitable solvent such as for example ethyl acetate, tetrahydrofurane, methanol, ethanol or mixtures thereof or by passing hydrogen into the reaction mixture in the presence of a metal catalyst such as for example palladium on charcoal at temperatures between 0°C and the boiling point of the solvent, typically at room temperature.

Aryl bromides of general formula **5** are obtainable according to the procedures described in Scheme 1.

The synthesis of similar compounds is also described in WO 2013/107743.

Alternatively, anilines of general formula **15** can be obtained from carboxylic acids of general formula **7** by a two step protocol involving *Curtius* rearrangement followed by deprotection as shown in Scheme 4. For deprotection of *tert*-butyloxycarbonyl (Boc) groups, all processes that are known to the person skilled in the art may be applied. The protected aniline of general formula **16** can be reacted in an organic solvent such as for example dichloromethane, diethyl ether or 1,4-dioxane with an acid such as for example trifluoroacetic acid or hydrochloric acid at temperatures between 0°C and the boiling point of the solvent, preferably at room temperature to give **15.**

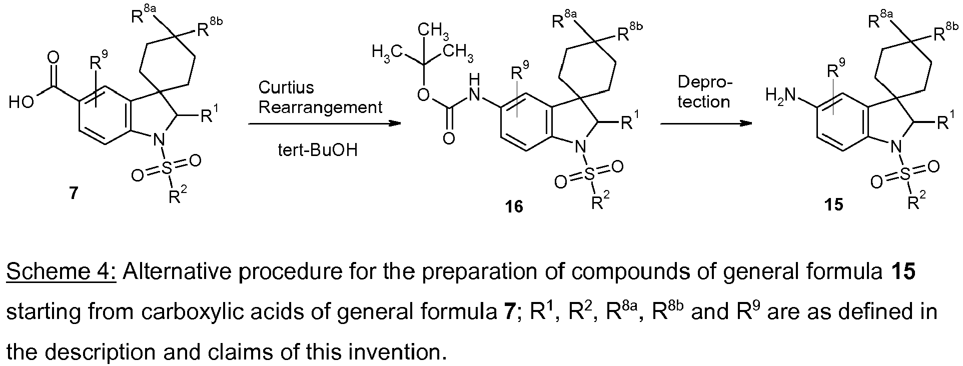

The protected aniline of general formula **16** can be obtained from carboxylic acids of general formula **7** by reaction in an organic solvent such as *tert*-butanol with an azide source such as for example diphenylphosphoryl azide in the presence of an organic base such as for example triethylamine at temperatures between 40°C and 150°C, preferably at 85°C. It might be necessary to add molecular sieves to the mixture.

The synthesis of similar compounds is also described in WO 2013/107743.

### SYNTHESIS OF KEY INTERMEDIATES

### Intermediate A.1 1

### 5'-bromo-4-(trifluoromethyl)spiro[cyclohexane-1,3'-indol]-4-ol

### Step 1:4-(methoxymethylene)-1-(trifluoromethyl)cyclohexanol

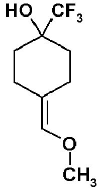

42.0 g (122 mmol) (methoxymethyl)triphenylphosphonium chloride in THF (330 ml) were cooled to 0°C and 22.9 g (204 mmol) KOtBu were added portionwise keeping the temperature below 4°C. After 30 min at 0°C 14.8 g (81.6 mmol) 4-hydroxy-4-(trifluoromethyl)cyclohexanone (CAS [120929-90-0]) dissolved in THF were added dropwise to the orange-red reaction mixture, keeping the temperature below 10°C. After further 30 min, the mixture was allowed to reach rt. Then it was poured into water and extracted three times with ethyl acetate. The combined organic phases were washed with brine, dried with sodium sulfate and concentrated in vacuo. The residue was purified by flash chromatography (SiO₂, hexane/ethyl acetate 0-20%) to obtain 17.6 g (92%) of the desired product as a colorless oil.
¹H-NMR (400MHz, DMSO-d₆): Shift [ppm] = 1.30 - 1.40 (m, 2H), 1.69 - 1.79 (m, 2H), 1.83 - 1.95 (m, 2H), 2.09 - 2.19 (m, 1 H), 2.49 - 2.55 (m, 1 H), 3.47 (s, 3H), 5.74 (s, 1 H), 5.88 (s, 1 H)

### Step 2 Fischer indole synthesis

### Similar to Liu et al., Tetrahedron 2010, 66, 3, 573-577 or WO10/151737, p. 224 5'-bromo-4-(trifluoromethyl)spiro[cyclohexane-1,3'-indol]-4-ol

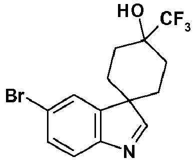

20.4 g (91.3 mmol) 4-bromo-phenylhydrazine hydrochloride and 4-(methoxymethylene)-1-(trifluoromethyl)cyclohexanol (19.2 g, 91.3 mmol) were dissolved in 1 l chloroform. The solution was cooled to 0°C and 23.2 ml (300 mmol) trifluoroacetic acid were added dropwise. The reaction was heated to 50°C for 6 h, then cooled to room temperature and stirred overnight. An aqueous solution of ammonia (25%) was carefully added to reach a pH of about 8. The phases were separated and the organic layer extracted with methylene chloride. The combined organic layers were washed with water, dried with sodium sulfate and the solvents removed. The crude product (34.5 g) was put to the next step without further purification.
UPLC-MS (ESI+): [M + H]⁺ = 348/350 (Br isotope pattern)

### Intermediate A.2

### 5'-bromo-4-{[tert-butyl(dimethyl)silyl]oxy}spiro[cyclohexane-1,3'-indole]

### Step 1: tert-butyl{[4-(methoxymethylidene)cyclohexyl]oxy}dimethylsilane

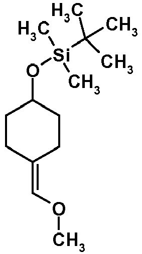

Prepared in analogy to intermediate A.1 (step 1): (Methoxymethyl)triphenylphosphonium chloride (22.5 g, 65.7 mmol), LDA (32.8 ml of a 2 M solution in THF, 65.7 mmol) and 4-{[tert-butyl(dimethyl)silyl]oxy}cyclohexanone (CAS-No. [55145-45-4];10.0 g, 43.8 mmol) were reacted in THF (150 ml) at 0°C for 35 min and at rt for 2 h. The crude product was purified by flash chromatography (SiO₂[KP-NH]-hexane/ethyl acetate) to give the desired enol ether (8.89 g, 79%) as a colourless oil.
¹H-NMR (300MHz, DMSO-d6): Shift [ppm]= 0.02 - 0.04 (m, 6H), 0.85 - 0.86 (m, 9H), 1.17 - 1.34 (m, 2H), 1.60 - 1.71 (m, 2H), 1.77 - 1.89 (m, 2H), 2.04 - 2.12 (m, 1 H), 2.33 - 2.42 (m, 1 H), 3.45 (s, 3H), 3.74 - 3.83 (m, 1 H), 5.82 (s, 1 H)
UPLC-MS (ESI+): [M + H]⁺ = 257; Rt in min 1.75

### Step 2 Fischer indole synthesis

### 5'-bromo-4-{[tert-butyl(dimethyl)silyl]oxy}spiro[cyclohexane-1,3'-indole]

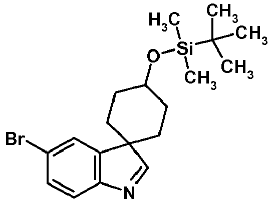

Prepared in analogy to intermediate A.1 (step 2): *Tert*-butyl{[4-(methoxymethylidene)cyclohexyl]oxy}dimethylsilane (8.80 g, 34.3 mmol), 4-bromophenylhydrazine hydrochloride (7.67 g, 34.3 mmol) and trifluoroacetic acid (8.72 ml, 113 mmol) were reacted in chloroform (480 ml) at 60°C for 2.5 h. The crude product obtained (14.3 g) was used without further purification.
UPLC-MS (ESI+): [M + H]+ = 394/396 (Br isotope pattern); Rt in min 1.81

### Intermediate A.3

### 5'-bromospiro[cyclohexane-1,3'-indole]

### Step 1: (methoxymethylidene)cyclohexane

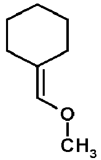

Prepared in analogy to intermediate A.1 (step 1): (Methoxymethyl)triphenylphosphonium chloride (26.2 g, 76.4 mmol), LDA (38.2 ml of a 2 M solution in THF, 76.4 mmol) and cyclohexanone (CAS-No. [108-94-1]; 5.0 g, 51 mmol) were reacted in THF (130 ml) at 0°C for 35 min and at rt for 1.5 h. The crude product (30 g) was used without further purification.

### Step 2 Fischer indole synthesis

### 5'-bromospiro[cyclohexane-1,3'-indole]

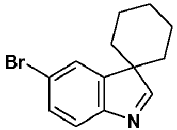

Prepared in analogy to intermediate A.1 (step 2): (Methoxymethylidene)cyclohexane (6.4 g, 51 mmol), 4-bromophenylhydrazine hydrochloride (11.4 g, 50.9 mmol) and trifluoroacetic acid (13 ml, 168 mmol) were reacted in chloroform (300 ml) at 50°C for 2.25 h. The crude product obtained was used without further purification. UPLC-MS (ESI+): [M + H]⁺ = 264/266 (Br isotope pattern); Rt in min 1.35 .

### Intermediate B.1

### Similar to WO06/090261, pp. 67-68

### 5'-bromo-2'-cyclopropyl-4-(trifluoromethyl)-1',2'-dihydrospiro[cyclohexane-1,3'-indol]-4-ol

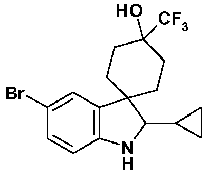

To a stirred solution of intermediate A.1 (14.9 g, 42.8 mmol) in 350 ml THF, 12.2 g (86 mmol) boron trifluoride diethylether complex was added dropwise at 0°C. After 5 min of stirring, 300 ml (150 mmol) cyclopropylmagnesium bromide (0.5 M in THF) were added dropwise, keeping the temperature of the mixture at 5 - 10°C. The mixture was allowed to warm to room temperature and stirred 3 h. Then saturated aqueous ammonium chloride solution was added and the mixture partitioned between ethyl acetate and water. The aqueous phase was extracted with ethyl acetate, the combined organic phases were washed with brine, dried with sodium sulfate, concentrated and purified by flash chromatography (SiO₂-hexane/ethylacetate) to yield 8.85 g (45%) of intermediate B.1.
¹H-NMR (400MHz, DMSO-d6): Shift [ppm] = 0.13 - 0.19 (m, 1H), 0.21 - 0.26 (m, 1H), 0.35 - 0.41 (m, 1 H), 0.45 - 0.52 (m, 1 H), 0.81 - 0.89 (m, 1 H), 1.51 - 2.01 (m, 8H), 2.85 (d, 1 H), 5.74 (s, 1 H), 5.79 (s, 1 H), 6.38 (d, 1 H), 6.94 (d, 1 H), 6.99 (dd, 1 H)

### Intermediate B.2

### 5'-bromo-4-{[tert-butyl(dimethyl)silyl]oxy}-2'-cyclopropyl-1',2'-dihydrospiro[cyclohexane-1,3'-indole]

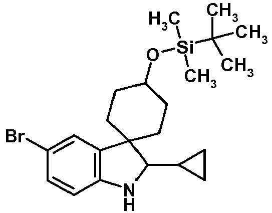

Prepared in analogy to intermediate B.1: Intermediate A.2 (14.3 g, 36.3 mmol), boron trifluoride diethylether complex (13.8 ml, 109 mmol) and 290 ml (145 mmol) cyclopropylmagnesium bromide (0.5 M in THF) were reacted in THF (400 ml) at 0°C for 2 h. After aqueous work up and purification by flash chromatography (SiO₂[KP-NH]-hexane/ethyl acetate) the desired indoline (5.72 g, 32%) was obtained as a mixture of diastereomers.
¹H-NMR (300MHz, DMSO-d6, major isomer): Shift [ppm]= 0.01 - 0.10 (m, 6H), 0.12 - 0.20 (m, 1 H), 0.30 - 0.53 (m, 3H), 0.85 - 0.90 (m, 10H), 1.47 - 1.91 (m, 8H), 2.75 (d, 1 H) [minor isomer: 2.85 (d, 1 H)], 3.82 - 3.89 (m, 1 H) [minor isomer: 3.69 - 3.82 (m, 1 H)], 5.76 (s, 1 H), 6.43 (d, 1 H), 6.98 - 7.12 (m, 2H)
UPLC-MS (ESI+): [M + H]⁺ = 436/438 (Br isotope pattern); Rt in min 1.91

### Intermediate B.3

### 5'-bromo-2'-cyclopropyl-1',2'-dihydrospiro[cyclohexane-1,3'-indole]

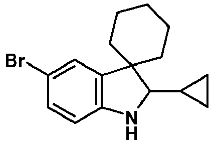

Prepared in analogy to intermediate B.1: Intermediate A.3 (13.5 g, 50.9 mmol), boron trifluoride diethylether complex (24.5 ml, 194 mmol) and cyclopropylmagnesium bromide (509 ml, 0.5 M in THF, 255 mmol) were reacted in THF (413 ml) at 0°C for 4 h. After aqueous work up and purification by flash chromatography (SiO₂-hexane/ethyl acetate) the desired indoline (6.1 g, 39%) was obtained.
¹H-NMR (400MHz, CDCl₃): Shift [ppm]= 0.13 - 0.19 (m, 1 H), 0.36 - 0.42 (m, 1 H), 0.44 - 0.51 (m, 1 H), 0.62 - 0.69 (m, 1 H), 0.82 - 0.91 (m, 2H), 0.94 - 1.03 (m, 1 H), 1.41 - 1.60 (m, 4H), 1.67 - 1.81 (m, 3H), 1.97 - 2.03 (m, 1 H), 2.74 (d, 1 H), 3.89 (br. s., 1 H), 6.50 (d, 1 H), 7.11 (dd, 1H), 7.25 (d, 1H)

### Intermediate C.1

### 5'-bromo-2'-cyclopropyl-1'-[(4-fluorophenyl)sulfonyl]-4-(trifluoromethyl)-1',2'-dihydrospiro[cyclohexane-1,3'-indol]-4-ol

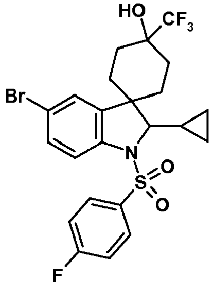

To a solution of indoline B.1 (610 mg, 1.56 mmol) in 15 ml 1,2-dichloroethane 912 mg 4-fluorobenzenesulfonyl chloride (CAS No. [349-88-2], 4.7 mmol), 1.3 ml triethylamine (9.4 mmol) and 9.5 mg (0.08 mmol) 4-dimethylaminopyridine were added at rt and the mixture was stirred at 70°C for 4 h. The reaction mixture was partitioned between water and dichloromethane and extracted with dichloromethane. The combined organic layers were washed with water, dried with sodium sulfate, concentrated and purified by flash chromatography (SiO₂-hexane/ethylacetate) to obtain 690 mg (72%) of the desired product.
¹H-NMR (400MHz, DMSO-d6): Shift [ppm] = 0.28 (d, 1 H), 0.34 - 0.41 (m, 1 H), 0.42 - 0.48 (m, 1 H), 0.52 - 0.63 (m, 2H), 0.91 - 0.99 (m, 1 H), 1.04 - 1.12 (m, 1 H), 1.15 - 1.24 (m, 1 H), 1.60 - 1.77 (m, 3H), 1.88 - 1.96 (m, 1 H), 2.04 - 2.12 (m, 1 H), 3. 86 (d, 1 H), 7.22 (d, 1 H), 7.34 - 7.45 (m, 4H), 7.82 - 7.88 (m, 2H)
HPLC-MS (Cl+): [M + H]⁺ = 548 / 550 (Br isotope pattern)

### Intermediate C.2

### 5'-bromo-4-{[tert-butyl(dimethyl)silyl]oxy}-2'-cyclopropyl-1'-[(4-fluorophenyl)sulfonyl]-1',2'-dihydrospiro[cyclohexane-1,3'-indole]

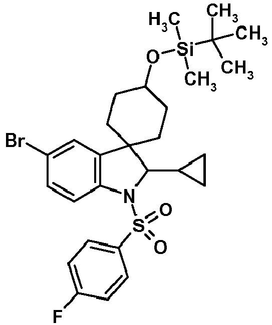

A mixture of indoline B.2 (750 mg, 1.72 mmol), 4-fluorobenzenesulfonyl chloride (CAS No. [349-88-2]; 502 mg, 2.58 mmol) and pyridine (10 eq., 1.4 ml, 17 mmol) was stirred at rt for 4.5 h. The reaction mixture was partitioned between aqueous saturated sodium hydrogencarbonate solution and dichloromethane and extracted with dichloromethane. The combined organic layers were washed with brine, dried with sodium sulfate and concentrated in vacuo. The obtained crude material was purified by flash chromatography (SiO₂-hexane/ethyl acetate) to give the title compound (780 mg, 62%) as a mixture of diastereomers.
¹H-NMR (400MHz, CDCl₃, major isomer): Shift [ppm]= 0.02 - 0.11 (m, 7H), 0.39 - 0.46 (m, 1H), 0.51 - 0.58 (m, 1 H), 0.62 - 0.68 (m, 2H), 0.88 - 0.98 (m, 10H), 1.21 - 1.28 (m, 2H), 1.33 - 1.40 (m, 1H), 1.59 - 1.68 (m, 1H), 1.73 - 1.78 (m, 1H), 1.84 - 1.88 (m, 1H), 2.18 (dt, 1H), 3.81 (d, 1 H), 3.94 (mc, 1H), 7.06 - 7.12 (m, 2H), 7.16 (d, 1H), 7.33 (dd, 1 H), 7.47 (d, 1H), 7.73 - 7.78 (m, 2H)
UPLC-MS (ESI-): [M + HCO2]⁻ = 638/640 (Br isotope pattern); Rt in min 1.95

### Intermediate C.3

### methyl 3-[(5'-bromo-2'-cyclopropylspiro[cyclohexane-1,3'-indol]-1'(2'H)-yl)sulfonyl]benzoate

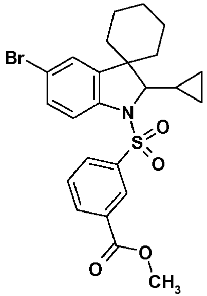

A mixture of indoline B.3 (2.80 g, 9.14 mmol), methyl 3-(chlorosulfonyl)benzoate (CAS No. [63555-50-0]; 3.22 g, 13.7 mmol) and pyridine (7.4 ml, 91 mmol) was stirred at rt for 17 h. The reaction mixture was partitioned between aqueous saturated sodium hydrogencarbonate solution and dichloromethane and extracted with dichloromethane. The combined organic layers were washed with brine, dried with sodium sulfate and concentrated in vacuo. The obtained crude material was purified by flash chromatography (SiO₂[KP-NH]-hexane/ethyl acetate) to give the title compound (3.1 g, 67%).
¹H-NMR (400MHz, CDCl₃): Shift [ppm]= 0.14 - 0.17 (m, 1H), 0.40 - 0.47 (m, 1 H), 0.51 - 0.57 (m, 1 H), 0.63 - 0.76 (m, 3H), 0.95 - 1.03 (m, 1 H), 1.14 - 1.29 (m, 3H), 1.41 - 1.52 (m, 1 H), 1.64 - 1.71 (m, 2H), 1.76 - 1.81 (m, 1 H), 2.11 - 2.14 (m, 1 H), 3.94 - 3.96 (m, 4H), 7.13 (d, 1 H), 7.32 (dd, 1 H), 7.47 - 7.52 (m, 2H), 7.90 (dd, 1 H), 8.19 (dd, 1 H), 8.46 - 8.47 (m, 1 H)

### Intermediate D.1

### methyl 2'-cyclopropyl-1'-[(4-fluorophenyl)sulfonyl]-4-hydroxy-4-(trifluoromethyl)-1',2'-dihydrospiro[cyclohexane-1,3'-indole]-5'-carboxylate

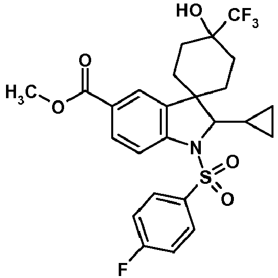

3.0 g (5.47 mmol) intermediate C.1 were placed into a steel autoclave under argon atmosphere and dissolved in 115 ml methanol, 12 ml DMSO and 1.7 ml triethylamine (12 mmol). 784 mg (1.09 mmol) trans-bis(triphenylphosphine) palladium(II) dichloride were added. The mixture was purged three times with carbon monoxide and then stirred for 30 min at 20°C under a carbon monoxide pressure of 9.5 bar. The autoclave was set under vacuo again, then a carbon monoxide pressure of 8.6 bar was applied and the mixture heated to 100°C for 22 h, yielding a maximum pressure of 10.6 bar. The reaction was cooled to rt, the pressure released and the reaction mixture concentrated in vacuo and redissolved in ethyl acetate / water. The layers were separated, the aqueous phase extracted with ethyl acetate, the combined organic layers washed with water and brine, dried with sodium sulfate and the solvents removed in vacuo. The crude product was purified by flash chromatography (SiO₂-hexane / ethyl acetate) to yield the desired methyl ester (2.02 g, 67%).
¹H-NMR (300MHz, DMSO-d6): Shift [ppm] = -0.23 (d, 1H), 0.33 - 0.43 (m, 1H), 0.44 - 0.51 (m, 1H), 0.52 - 0.65 (m, 2H), 0.90 - 1.23 (m, 3H), 1.61 - 1.79 (m, 3H), 1.92 - 2.00 (m, 1 H), 2.07 - 2.17 (m, 1 H), 3.79 (s, 3H), 3. 94 (d, 1 H), 5.76 (s, 1H), 7.34 - 7.40 (m, 2H), 7.57 - 7.62 (m, 2H), 7.85 - 7.92 (m, 3H)

### Intermediate D.2

### methyl 4-{[tert-butyl(dimethyl)silyl]oxy}-2'-cyclopropyl-1'-[(4-fluorophenyl)sulfonyl]-1',2'-dihydrospiro[cyclohexane-1,3'-indole]-5'-carboxylate

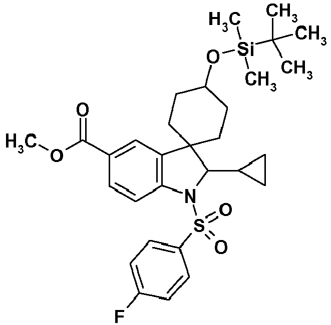

Prepared in analogy to intermediate D.1: The aryl bromide C.2 (506 mg, 0.851 mmol) was carbonylated in a mixture of 20 ml methanol, 2 ml DMSO and triethylamine (0.260 ml, 1.87 mmol) in the presence of trans-bis(triphenylphosphine) palladium(II) dichloride (120 mg, 0.17 mmol). A carbon monoxide pressure of 12 bar was applied at 20°C, after evacuation a carbon monoxide pressure of 13 bar was applied and the autoclave was heated to 100°C internal temperature for 23 h to reach a pressure of 17.6 bar. The reaction was cooled to rt, the pressure released and the reaction mixture partitioned between aqueous saturated sodium hydrogencarbonate solution and ethyl acetate. The phases were separated and the aqueous phase extracted with ethyl acetate. The combined organic layers were washed with brine, dried with sodium sulfate and concentrated in vacuo. The obtained crude material was purified by flash chromatography (SiO₂-hexane/ethyl acetate) to yield the desired methyl ester D.2 (232 mg, 39%) as a mixture of diastereomers.
¹H-NMR (300MHz, CDCl3, major isomer): Shift [ppm]= 0.02 - 0.13 (m, 7H), 0.38 - 0.47 (m, 1 H), 0.52 - 0.61 (m, 1 H), 0.62 - 0.70 (m, 2H), 0.91 - 0.99 (m, 10H), 1.24 - 1.46 (m, 3H), 1.61 - 1.70 (m, 1 H), 1.77 - 1.93 (m, 2H), 2.32 (dt, 1H), 3.87 (d, 1H), 3.88 - 3.90 (m, 3H), 3.93 - 3.99 (m, 1 H), 7.06 - 7.12 (m, 2H), 7.62 (d, 1 H), 7.76 - 7.81 (m, 3H), 7.94 (dd, 1 H).
UPLC-MS (ESI+): [M + H]⁺ = 574; Rt in min 1.82

### Intermediate E.1

### 2'-cyclopropyl-1'-[(4-fluorophenyl)sulfonyl]-4-hydroxy-4-(trifluoromethyl)-1',2'-dihydrospiro[cyclohexane-1,3'-indole]-5'-carboxylic acid

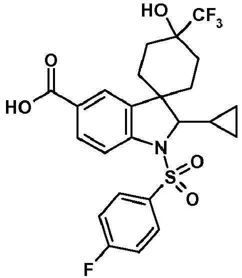

7.46 g (14.1 mmol) methyl ester intermediate D.1 were dissolved in a mixture of 140 ml THF and 30 ml water and 508 mg (21.2 mmol) lithium hydroxide were added. The mixture was stirred at rt until TLC and/or LCMS indicated complete consumption of the starting material (18 h). The mixture was set to pH 4 by addition of 2M aqueous hydrochloric acid and extracted with ethyl acetate. The combined organic layers were washed with brine, dried with sodium sulfate and concentrated in vacuo. The product (7.26 g, 100 %) was used without further purification.
¹H-NMR (300MHz, DMSO-d6): Shift [ppm] = -0.23 (d, 1 H), 0.33 - 0.44 (m, 1 H), 0.44 - 0.64 (m, 3H), 0.90 - 1.24 (m, 3H), 1.61 - 1.79 (m, 3H), 1.93 - 2.00 (m, 1 H), 2.07 - 2.19 (m, 1 H), 3. 93 (d, 1 H), 5.77 (s, 1 H), 7.34 - 7.40 (m, 2H), 7.56 - 7.59 (m, 2H), 7.84 - 7.91 (m, 3H)

### Intermediate E.2

### 4-{[tert-butyl(dimethyl)silyl]oxy}-2'-cyclopropyl-1'-[(4-fluorophenyl)sulfonyl]-1',2'-dihydrospiro[cyclohexane-1,3'-indole]-5'-carboxylic acid

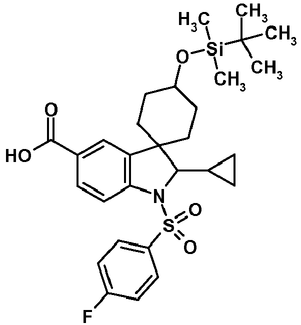

A solution of methyl ester D.2 (667 mg, 1.16 mmol) in 27 ml THF was treated with an aqueous lithium hydroxide solution (11.6 mmol, 278 mg in 5 ml water) and stirred at 60°C for 18 h. The mixture was set to pH 5 by addition of 2M aqueous hydrochloric acid and extracted with ethyl acetate. The combined organic layers were washed with brine, dried with sodium sulfate and concentrated in vacuo. The obtained diastereomeric mixture of E.2 (651 mg, 100%) was used without further purification.
¹H-NMR (300MHz, CDCl3, major isomer): Shift [ppm]= 0.03 - 0.17 (m, 7H), 0.38 - 0.50 (m, 1 H), 0.52 - 0.70 (m, 3H), 0.91 - 1.01 (m, 10H), 1.23 - 1.48 (m, 3H), 1.62 - 1.71 (m, 1 H), 1.78 - 1.94 (m, 2H), 2.34 (dt, 1 H), 3.89 (d, 1 H), 3.97 - 4.01 (m, 1 H), 7.08 - 7.14 (m, 2H), 7.64 - 7.69 (m, 1H), 7.75 - 7.82 (m, 3H), 8.02 (dd, 1 H)

### Intermediate E.3

### 2'-cyclopropyl-1'-{[3-(methoxycarbonyl)phenyl]sulfonyl}-1',2'-dihydrospiro[cyclohexane-1,3'-indole]-5'-carboxylic acid

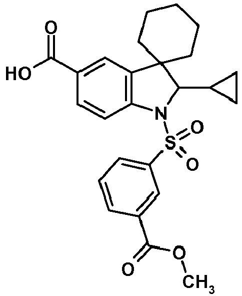

The aryl bromide C.3 (100 mg, 0.198 mmol) was placed into a steel autoclave under argon atmosphere and dissolved in 10 ml DMSO. Palladium(II) acetate (2.2 mg, 0.010 mmol), 1,1'-bis(di-phenylphosphino)ferrocene (23 mg, 0.040 mmol) and potassium acetate (78 mg, 0.79 mmol) were added. The mixture was purged three times with carbon monoxide and stirred for 30 min at 20°C under a carbon monoxide pressure of 10.5 bar. The autoclave was set under vacuo again, a carbon monoxide pressure of 12 bar was applied and the mixture heated to 100°C (internal temperature) for 24 h to reach a pressure of 14.3 bar. The reaction was cooled to rt, the pressure released and the reaction mixture partitioned between aqueous saturated sodium hydrogencarbonate solution and ethyl acetate. The phases were separated and the aqueous phase extracted with ethyl acetate. The combined organic layers were washed with brine, dried with sodium sulfate and concentrated in vacuo. The obtained product (50 mg, 54%) was used without further purification.
HPLC-MS (ES+): [M + H]⁺ = 470

### Intermediate F.1

### 4-{[tert-butyl(dimethyl)silyl]oxy}-N-(2-chlorobenzyl)-2'-cyclopropyl-1'-[(4-fluorophenyl)sulfonyl]-1',2'-dihydrospiro[cyclohexane-1,3'-indole]-5'-carboxamide

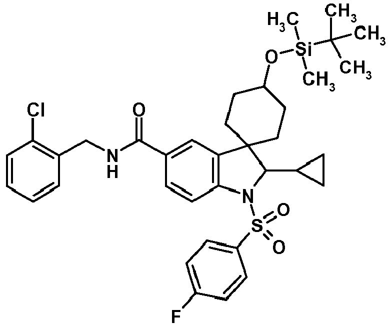

To a solution of intermediate C.2 (1.17 g, 1.97 mmol) in 42 ml 1,4-dioxane (containing ca. 1% water) 1-(2-chlorophenyl)methanamine (CAS No. [89-97-4]; 836 mg, 5.91 mmol), molybdenum hexacarbonyl (519 mg, 1.97 mmol), sodium carbonate (626 mg, 5.91 mmol), tri-tert-butylphosphonium tetrafluoroborate (57 mg, 0.20 mmol) and palladium(II) acetate (44 mg, 0.20 mmol) were added. The reaction mixture was vigorously stirred at 140°C for 8 h and at rt overnight. The reaction mixture was filtrated over Celite^{®} and the filter cake rinsed with ethyl acetate. The filtrate was concentrated in vacuo and subjected to flash chromatography (SiO₂-hexane/ethyl acetate) to give the title compound F.1 (504 mg, 37%) as a mixture of diastereomers.
¹H-NMR (300MHz, DMSO-d6, major isomer): Shift [ppm]= -0.08 - 0.04 (m, 7H), 0.36 - 0.48 (m, 2H), 0.57 - 0.72 (m, 2H), 0.85 - 0.86 (m, 9H), 0.91 - 1.10 (m, 3H), 1.32 - 1.47 (m, 1 H), 1.56 - 1.62 (m, 1 H), 1.70 - 1.76 (m, 1 H), 1.83 - 1.86 (m, 1 H), 2.21 (dt, 1 H), 3.92 - 3.97 (m, 2H) [minor isomer: 3.57 - 3.64 (m, 1 H)], 4.44 - 4.58 (m, 2H), 7.27 - 7.46 (m, 6H), 7.55 (d,
1 H), 7.61 (d, 1 H) [minor isomer: 7.73 (d, 1 H)], 7.83 - 7.91 (m, 3H), 9.01 (td, 1 H) [minor isomer: 8.90 (t, 1 H)]
UPLC-MS (ESI+): [M + H]⁺ = 683/685 (Cl isotope pattern); Rt in min 1.82

### COMPOUNDS ACCORDING TO THE INVENTION:

### Example 1

### N-[(3-chloropyridin-2-yl)methyl]-1'-[(4-fluorophenyl)sulfonyl]-4-hydroxy-2'-methyl-4-(trifluoromethyl)-1',2'-dihydrospiro[cyclohexane-1,3'-indole]-5'-carboxamide

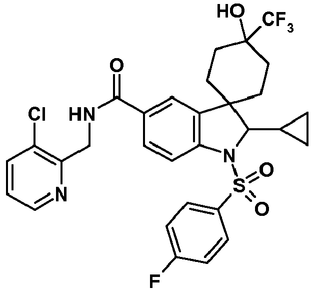

300 mg (0.58 mmol) carboxylic acid intermediate E.1 were dissolved in 4.5 ml DMF. 100 mg (0.70 mmol) 1-(3-chlorpyridin-2-yl)methylamine (CAS No. [500305-98-6]), 267 mg (0.70 mmol) HATU and 0.30 ml (2.16 mmol) triethylamine were added. The reaction mixture was stirred at rt until TLC and/or LCMS indicated complete consumption of the starting material (2 h), then water was added. The formed precipitate was filtered off, washed with water and dried in vacuo at 40°C to yield 371 mg (87%) of the desired amide.
¹H-NMR (400MHz, DMSO-d6): Shift [ppm]= -0.22 (d, 1 H), 0.37 - 0.44 (m, 1 H), 0.45 - 0.52 (m, 1 H), 0.56 - 0.67 (m, 2H), 0.94 - 1.02 (m, 1 H), 1.10 - 1.28 (m, 2H), 1.64 - 1.80 (m, 3H), 1.94 - 2.02 (m, 1 H), 2.17 - 2.26 (m, 1 H), 3. 95 (d, 1 H), 4.65 (d, 2H), 5.75 (s, 1 H), 7.34 - 7.42 (m, 3H), 7.58 (d, 1 H), 7.70 (d, 1 H), 7.85 - 7.93 (m, 4H), 8.47 (dd, 1 H), 8.96 (t, 1 H)

The enantiomers of the racemic material of example 1 were separated by chiral preparative HPLC (System: Dionex: Pump P 580, Gilson: Liquid Handler 215, Knauer: UV-Detektor K-2501; Column: Chiralpak AD-H 5µm 250x30 mm; Solvent hexane / ethanol 70:30 (v/v); Flow: 40 ml/min; Temperature: rt; Injection: 10x0.5 ml, 72 mg/ml EtOH/MeOH; Detection: UV 280 nm) and analytically characterized by HPLC System: Waters: Alliance 2695, DAD 996, ESA: Corona ; Column:Chiralpak AD-H 5µm 150x4.6 mm ; Solvent:Hexan / Ethanol 70:30 (v/v) ; Flow:1.0 ml/min ; Temperature: 25°C ; Solution: 1.0 mg/ml EtOH/MeOH 1:1 ; Injection:5.0 µl ; Detection: DAD 280 nm

### Example 1.1: Rt in min 4.04

### Example 1.2: Rt in min 6.83

### Example 2

### N-(2-chlorobenzyl)-2'-cyclopropyl-1'-[(4-fluorophenyl)sulfonyl]-4-hydroxy-1',2'-dihydrospiro[cyclohexane-1,3'-indole]-5'-carboxamide

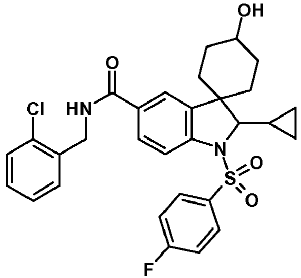

A solution of intermediate F.1 (500 mg, 0.659 mmol) in 3 ml THF was treated with tetra-n-butylammonium fluoride (0.99 ml of a 1M solution in THF, 0.99 mmol) and stirred at rt overnight. The reaction mixture was partitioned between ethyl acetate and water, the phases separated and the aqueous phase extracted with ethyl acetate. The combined organic layers were washed with brine, dried with sodium sulfate and concentrated in vacuo to give the title compound (480 mg, 100%) as a mixture of diastereomers.
¹H-NMR (300MHz, DMSO-d6, major isomer): Shift [ppm]= -0.17 - -0.12 (m, 1 H), 0.32 - 0.46 (m, 2H), 0.54 - 0.72 (m, 2H), 0.83 - 1.17 (m, 4H), 1.48 - 1.57 (m, 1 H), 1.70 - 1.82 (m, 2H), 2.19 - 2.29 (m, 1 H), 3.76 (br. s., 1 H), 3.91 (d, 1 H), 4.42 (d, 1 H) [minor isomer: 4.57 (d, 1 H)], 4.52 (d, 2H), 7.26 - 7.46 (m, 6H), 7.55 (d, 1 H), 7.77 (s, 1 H) [minor isomer: 7.70 (d, 1 H)], 7.83 - 7.88 (m, 3H), 9.04 (t, 1 H) [minor isomer: 8.93 (t, 1 H)]
UPLC-MS (ESI+): [M + H]⁺ = 569/571 (Cl isotope pattern); Rt in min 1.30 and 1.33 min

### Example 3

### N-(2-chlorobenzyl)-2'-cyclopropyl-1'-[(4-fluorophenyl)sulfonyl]-4-oxo-1',2'-dihydrospiro[cyclohexane-1,3'-indole]-5'-carboxamide

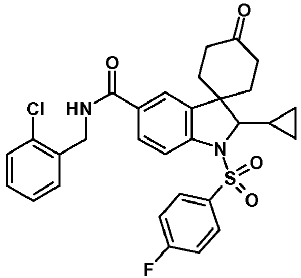

To a freshly prepared solution of Dess-Martin periodinane (350 mg, 0.825 mmol) in 5 ml dichloromethane was added dropwise a solution of example 2 (430 mg, 0.688 mmol) in 5 ml dichloromethane at rt and stirring at rt continued for 1.5 h. The reaction mixture was concentrated in vacuo and subjected to flash chromatography (SiO₂-hexane/ethyl acetate) to give the title compound (266 mg, 65%).
¹H-NMR (300MHz, DMSO-d6): Shift [ppm]= 0.30 - 0.35 (m, 1H), 0.39 - 0.67 (m, 3H), 0.80 - 0.88 (m, 1 H), 1.03 - 1.15 (m, 1 H), 1.22 (dt, 1 H), 1.74 - 1.79 (m, 1 H), 2.32 - 2.36 (m, 3H), 2.54 - 2.73 (m, 2H), 4.27 (d, 1 H), 4.51 (d, 2H), 7.28 - 7.46 (m, 6H), 7.59 (d, 1 H), 7.80 (d, 1 H), 7.86 - 7.92 (m, 3H), 8.92 (t, 1 H)
UPLC-MS (ESI+): [M + H]⁺ = 567/569 (Cl isotope pattern); Rt in min 1.36

The enantiomers of the racemic material of example 3 were separated by chiral preparative HPLC (System: Dionex: Pump P 580, Gilson: Liquid Handler 215, Knauer: UV-Detector K-2501; Column: Chiralcel OZ-H 5µm 250x30 mm; Solvent: ethanol / methanol 50:50 (v/v); Flow: 30 ml/min; Temperature: RT; Injection: 0.5 ml/run, 50 mg/ml EtOH / MeOH 1:1; Detection: UV 280 nm) and analytically characterized by HPLC System: Waters: Alliance 2695, DAD 996, ESA: Corona; Column: Chiralcel OZ-H 5µm 150x4.6 mm; Solvent: ethanol / methanol 50:50 (v/v); Flow: 1.0 ml/min; Temperature: 25°C; Solution: 1.0 mg/ml EtOH / MeOH 1:1; Injection: 5.0 µl; Detection: DAD 280 nm:

### Example 3.1: Rt in min 2.33/3.78 (rotamers)

### Example 3.2: Rt in min 2.81/4.54 (rotamers)

### Example 4

### N-(2-chlorobenzyl)-2'-cyclopropyl-1'-[(4-fluorophenyl)sulfonyl]-4-hydroxy-4-methyl-1',2'-dihydrospiro[cyclohexane-1,3'-indole]-5'-carboxamide

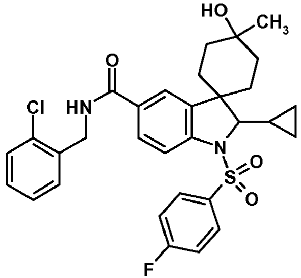

A solution of example 3 (50 mg, 0.084 mmol) in 1.5 ml THF was cooled to -78°C and treated with methyllithium (120 µl of a 1.6M solution in diethyl ether, 0.193 mmol). The mixture was stirred at 0°C for 3 h and at rt overnight. The reaction mixture was partitioned between ethyl acetate and saturated aqueous ammonium chloride solution. The phases were separated and the aqueous phase extracted with ethyl acetate. The combined organic layers were washed with brine, dried with magnesium sulfate and concentrated in vacuo. The product was purified by flash chromatography (SiO₂-hexane/ethyl acetate) to give the title compound (9 mg, 17%) as a mixture of diastereomers.
¹H-NMR (400MHz, DMSO-d6, major isomer): Shift [ppm]= -0.30 (d, 1H), 0.34 - 0.42 (m, 2H), 0.54 - 0.61 (m, 1 H), 0.66 - 0.73 (m, 1 H), 0.91 - 0.97 (m, 1H), 1.01 - 1.04 (m, 1 H), 1.12 - 1.18 (m, 4H), 1.41 -1.58 (m, 3H), 1.81 - 1.84 (m, 1 H), 2.24 (dt, 1 H), 3.98 (d, 1 H), 4.05 (s, 1 H), 4.52 (d, 2H), 7.26 - 7.46 (m, 6H), 7.55 (d, 1 H), 7.72 (d, 1 H), 7.82 - 7.86 (m, 3H), 9.02 (t, 1 H) [minor isomer: 8.94 (t, 1 H)]
UPLC-MS (ESI+): [M + H]⁺ = 583/585 (Cl isotope pattern); Rt in min 1.38

### Example 5

### N-(2-chlorobenzyl)-2'-cyclopropyl-1'-[(4-fluorophenyl)sulfonyl]-4-(hydroxyimino)-1',2'-dihydrospiro[cyclohexane-1,3'-indole]-5'-carboxamide

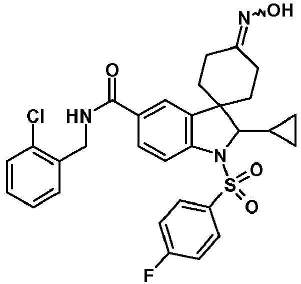

A solution of example 3 (50 mg, 0.084 mmol) in a mixture of toluene and tert-butanol (2 ml, 1:1) was treated with hydroxylamine hydrochloride (28 mg, 0.40 mmol) and the mixture heated to 100°C for 4 h. The reaction mixture was cooled, partitioned between ethyl acetate and water, the phases separated and the aqueous phase extracted with ethyl acetate. The combined organic layers were washed with brine, dried with sodium sulfate and concentrated in vacuo to give the product (48 mg, 89%) as a mixture of E/Z isomers.
¹H-NMR (300MHz, DMSO-d6): Shift [ppm]= 0.09 - 0.24 (m, 1H), 0.37 - 0.55 (m, 2H), 0.58 - 0.67 (m, 1 H), 0.74 - 0.95 (m, 2H), 0.98 - 1.07 (m, 1 H), 1.77 - 2.07 (m, 3H), 2.19 - 2.36 (m, 2H), 2.50 - 2.61 (m, 1H), 4.09 - 4.14 (m, 1 H), 4.51 (d, 2H), 7.25 - 7.46 (m, 6H), 7.58 (d, 1 H), 7.78 (dd, 1 H), 7.84 - 7.92 (m, 3H), 8.91 (t, 1 H), 10.27 - 10.32 (m, 1 H)
UPLC-MS (ESI+): [M + H]⁺ = 582/584 (Cl isotope pattern); Rt in min 1.31

### Example 6

### N-(2-chlorobenzyl)-2'-cyclopropyl-4,4-difluoro-1'-[(4-fluorophenyl)sulfonyl]-1',2'-dihydrospiro[cyclohexane-1,3'-indole]-5'-carboxamide

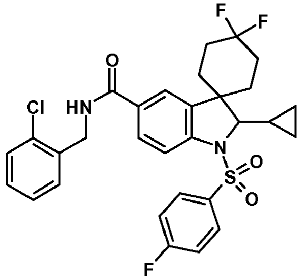

To (diethylamino)sulfur trifluoride (34 µL, 0.26 mmol) at rt was added dropwise a solution of example 3 (50 mg, 0.084 mmol) in 1 ml dichloromethane and the mixture stirred at rt overnight. The reaction mixture was poured onto ice-water, stirred for 20 min and neutralized with aqueous sodium hydrogencarbonate solution (4%). The phases were separated and the aqueous phase extracted with ethyl acetate. The combined organic layers were washed with brine, dried with magnesium sulfate and concentrated in vacuo. The obtained product was purified by flash chromatography (SiO₂-hexane/ethyl acetate) to give the title compound (34 mg, 62%).
¹H-NMR (400MHz, DMSO-d6): Shift [ppm]= 0.09 - 0.12 (m, 1 H), 0.37 - 0.64 (m, 3H), 0.74 - 0.80 (m, 1 H), 0.97 - 1.04 (m, 2H), 1.47 - 1.59 (m, 1 H), 1.97 - 2.21 (m, 4H), 2.50 - 2.58 (m, 1H), 4.13 (d, 1H), 4.52 (d, 2H), 7.27 - 7.46 (m, 6H), 7.55 - 7.61 (m, 1 H), 7.73 - 7.77 (m, 1 H), 7.84 - 7.94 (m, 3H), 8.91 - 8.98 (m, 1 H)
UPLC-MS (ESI+): [M + H]⁺ = 589/591 (Cl isotope pattern); Rt in min 1.49

### Example 7

### N-{[3-chloro-5-(trifluoromethyl)pyridin-2-yl]methyl}-2'-cyclopropyl-1'-[(4-fluorophenyl)sulfonyl]-4-hydroxy-1',2'-dihydrospiro[cyclohexane-1,3'-indole]-5'-carboxamide

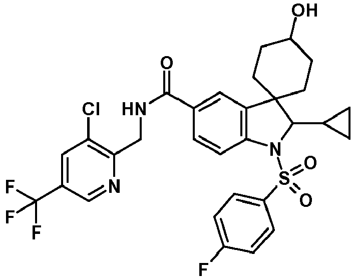

A solution of the carboxylic acid intermediate E.2 (250 mg, 0.447 mmol) in 5 ml DMF was treated with HATU (255 mg, 0.670 mmol), triethylamine (93 µl, 0.67 mmol) and 1-[3-chloro-5-(trifluoromethyl)pyridin-2-yl]methanamine hydrochloride (CAS No. [175277-74-4], 470 mg, 2.23 mmol) and the mixture stirred at rt for 1 h. The reaction mixture was partitioned between dichloromethane and water, the phases separated and the aqueous phase extracted with dichloromethane. The combined organic layers were washed with brine, dried with sodium sulfate and concentrated in vacuo. The obtained product was purified by flash chromatography (SiO₂-hexane/ethyl acetate) to give the title compound (194 mg, 68%) as a mixture of diastereomers.
¹H-NMR (300MHz, DMSO-d6, major isomer): Shift [ppm]= -0.20 - -0.13 (m, 1 H), 0.35 - 0.44 (m, 2H), 0.56 - 0.70 (m, 2H), 0.83 - 1.17 (m, 4H), 1.47 - 1.56 (m, 1 H), 1.70 - 1.81 (m, 2H), 2.19 - 2.27 (m, 1 H), 3.76 (br. s., 1 H), 3.92 (d, 1 H), 4.43 (d, 1 H) [minor isomer: 4.45 (d, 1 H)], 4.72 (d, 2H), 7.37 - 7.43 (m, 2H), 7.55 (d, 1 H) [minor isomer: 7.60 (d, 1 H)], 7.76 (d, 1 H) [minor isomer: 7.70 (s, 1 H)], 7.81 - 7.91 (m, 3H), 8.47 (s, 1 H), 8.90 (s, 1 H), 9.10 (t, 1 H) UPLC-MS (ESI+): [M + H]⁺ = 638/640 (Cl isotope pattern); Rt in min 1.36 and 1.39

### Example 8

### N-{[3-chloro-5-(trifluoromethyl)pyridin-2-yl]methyl}-2'-cyclopropyl-1'-[(4-fluorophenyl)sulfonyl]-4-oxo-1',2'-dihydrospiro[cyclohexane-1,3'-indole]-5'-carboxamide

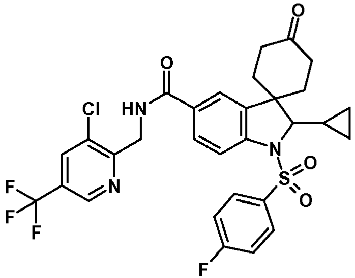

To a freshly prepared solution of Dess-Martin periodinane (140 mg, 0.331 mmol) in 3 ml dichloromethane was added dropwise a solution of example 7 (176 mg, 0.276 mmol) in 3 ml dichloromethane at rt. Stirring was continued at rt for 24 h. The reaction mixture was concentrated in vacuo and subjected to flash chromatography (SiO₂-hexane/ethyl acetate) to give the title compound (90 mg, 48%).
¹H-NMR (300MHz, DMSO-d6): Shift [ppm]= 0.33 - 0.38 (m, 1H), 0.41 - 0.67 (m, 3H), 0.80 - 0.88 (m, 1H), 1.05 - 1.26 (m, 2H), 1.75 - 1.80 (m, 1 H), 2.32 - 2.37 (m, 3H), 2.57 - 2.69 (m, 2H), 4.26 (d, 1 H), 4.72 (d, 2H), 7.38 - 7.44 (m, 2H), 7.59 (d, 1 H), 7.79 (s, 1 H), 7.84 - 7.92 (m, 3H), 8.45 (d, 1 H), 8.89 (d, 1 H), 8.95 (t, 1 H)
UPLC-MS (ESI+): [M + H]⁺ = 636/638 (Cl isotope pattern); Rt in min 1.39

### Example 9

### methyl 3-({5'-[(2-chlorobenzyl)carbamoyl]-2'-cyclopropylspiro[cyclohexane-1,3'-indol]-1'(2'H)-yl}sulfonyl)benzoate

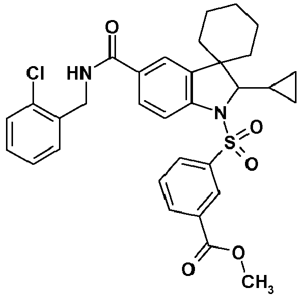

Prepared in analogy to intermediate F.1: Intermediate C.3 (300 mg, 0.595 mmol), 1-(2-chlorophenyl)methanamine (CAS No. [89-97-4]; 253 mg, 1.78 mmol), molybdenum hexacarbonyl (157 mg, 0.595 mmol), sodium carbonate (189 mg, 1.78 mmol), tri-*tert-*butylphosphonium tetrafluoroborate (17 mg, 0.059 mmol) and palladium(II) acetate (13 mg, 0.059 mmol) were reacted in 18 ml 1,4-dioxane (containing ca. 1 % water) at 120°C for 4 h. The reaction mixture was concentrated in vacuo and subjected to flash chromatography (SiO₂-hexane/ethyl acetate) to give the title compound (208 mg, 59%).
¹H-NMR (400MHz, CDCl₃): Shift [ppm]= 0.18 (d, 1 H), 0.38 - 0.45 (m, 1H), 0.51 - 0.57 (m, 1 H), 0.64 - 0.77 (m, 3H), 0.95 - 1.02 (m, 1 H), 1.18 - 1.30 (m, 3H), 1.41 - 1.52 (m, 1 H), 1.63 - 1.66 (m, 1H), 1.77 - 1.84 (m, 2H), 2.12 - 2.16 (m, 1 H), 3.92 (s, 3H), 4.00 (d, 1H), 4.72 (d, 2H), 6.51 (t, 1 H), 7.21 - 7.26 (m, 2H), 7.39 - 7.42 (m, 1 H), 7.46 - 7.50 (m, 2H), 7.54 - 7.63 (m, 3H), 7.89 - 7.92 (m, 1 H), 8.16 - 8.18 (m, 1 H), 8.47 - 8.48 (m, 1 H)
UPLC-MS (ESI+): [M + H]⁺ = 593/595 (Cl isotope pattern); Rt in min 1.54

### Example 10

### 3-({5'-[(2-chlorobenzyl)carbamoyl]-2'-cyclopropylspiro[cyclohexane-1,3'-indol]-1'(2'H)-yl}sulfonyl)benzoic acid

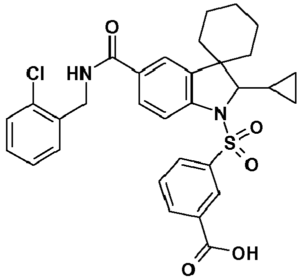

A solution of example 9 (170 mg, 0.286 mmol) in 7.4 ml THF was treated with an aqueous lithium hydroxide solution (2.86 mmol, 68.4 mg in 1 ml water) and stirred at rt for 21.5 h. The mixture was set to pH 2 by addition of 2M aqueous hydrochloric acid and extracted with ethyl acetate. The combined organic layers were washed with brine, dried with sodium sulfate and concentrated in vacuo. The product was stirred in a mixture of dichloromethane and diisopropylether, the solid filtered off, washed with diisopropylether and dried in vacuo to yield the title compound (117 mg, 61 %).
¹H-NMR (300MHz, DMSO-d6): Shift [ppm]= -0.03 (d, 1 H), 0.34 - 0.47 (m, 2H), 0.57 - 0.78 (m, 3H), 0.92 - 1.01 (m, 1 H), 1.06 - 1.19 (m, 2H), 1.25 - 1.35 (m, 1 H), 1.42 - 1.59 (m, 2H), 1.70 - 1.74 (m, 1 H), 1.83 (dt, 1 H), 2.04 - 2.08 (m, 1 H), 3.97 (d, 1 H), 4.51 (d, 2H), 7.25 - 7.37 (m, 3H), 7.43 - 7.46 (m, 1 H), 7.55 (d, 1 H), 7.67 (t, 1 H), 7.72 (d, 1 H), 7.85 (dd, 1 H), 7.99 - 8.01 (m, 1 H), 8.15 - 8.18 (m, 1 H), 8.26 - 8.27 (m, 1H), 8.95 (t, 1 H)
UPLC-MS (ESI+): [M + H]⁺ = 579/581 (Cl isotope pattern); Rt in min 1.40

### Example 11

### methyl 3-{[5'-{[(3-chloropyridin-2-yl)methyl]carbamoyl}-2'-cyclopropylspiro[cyclohexane-1,3'-indol]-1'(2'H)-yl]sulfonyl}benzoate

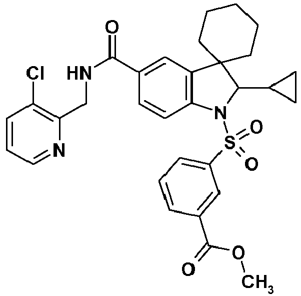

A solution of the carboxylic acid intermediate E.3 (50 mg, 0.11 mmol) in 3 ml DMF was treated with HATU (61 mg, 0.16 mmol), triethylamine (74 µl, 0.53 mmol) and 1-(3-chloropyridin-2-yl)methanamine dihydrochloride (CAS No. [342816-31-3]; 34 mg, 0.16 mmol) and the mixture stirred at rt for 20 h. The reaction mixture was partitioned between ethyl acetate and saturated aqueous sodium hydrogencarbonate solution, the phases separated and the aqueous phase extracted with ethyl acetate. The combined organic layers were washed with brine, dried with sodium sulfate and concentrated in vacuo. The obtained product was purified by preparative TLC to give the title compound (14 mg, 23%).
¹H-NMR (300MHz, CDCl₃): Shift [ppm]= 0.21 (d, 1H), 0.38 - 0.48 (m, 1H), 0.52 - 0.61 (m, 1H), 0.64 - 0.83 (m, 3H), 0.95 - 1.06 (m, 1 H), 1.19 - 1.27 (m, 3H), 1.42 - 1.52 (m, 1 H), 1.65 - 1.67 (m, 1 H), 1.81 - 1.91 (m, 2H), 2.15 - 2.19 (m, 1 H), 3.93 (s, 3H), 4.01 (d, 1H), 4.83 (d, 2H), 7.23 - 7.27 (m, 1 H), 7.49 (t, 1 H), 7.65 - 7.67 (m, 2H), 7.72 - 7.76 (m, 2H), 7.82 (t, 1 H), 7.92 - 7.95 (m, 1 H), 8.16 - 8.20 (m, 1 H), 8.49 - 8.53 (m, 2H)
UPLC-MS (ESI+): [M + H]⁺ = 594/596 (Cl isotope pattern); Rt in min 1.46

**Table 1:The following examples were prepared in analogy to example 1 starting from the appropriate carboxylic acid intermediate and commercially available amines.**

| **Example No** | **Structure** | **Name** | **Analytical data** |
|---|---|---|---|
| 1 | 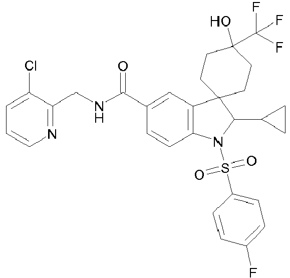 | N-[(3-chloropyridin-2-yl)methyl]-2'-cyclopropyl-1'-[(4-fluorophenyl)sulfo nyl]-4-hydroxy-4-(trifluoromethyl)-1',2'-dihydrospiro[cyclo hexane-1,3'-indole]-5'-carboxamide | ¹H-NMR (400MHz, DMSO-d6): Shift [ppm]= -0.22 (d, 1H), 0.37 - 0.44 (m, 1H), 0.45 - 0.52 (m, 1H), 0.56 - 0.67 (m, 2H), 0.94 - 1.02 (m, 1H), 1.10 - 1.28 (m, 2H), 1.64 - 1.80 (m, 3H), 1.94 - 2.02 (m, 1H), 2.17 - 2.26 (m, 1H), 3.95 (d, 1H), 4.65 (d, 2H), 5.75 (s, 1H), 7.34 - 7.42 (m, 3H), 7.58 (d, 1H), 7.70 (d, 1H), 7.85 - 7.93 (m, 4H), 8.47 (dd, 1H), 8.96 (t, 1H) |
| 1-1 | Enantiomer 1 of Ex. 1 | | System: Waters: Alliance 2695, DAD 996, ESA: Corona ; Column:Chiralpak AD-H 5µm 150x4.6 mm ; Solvent:Hexan / Ethanol 70:30 (v/v) ;; Flow:1.0 ml/min ; Temperature: 25°C ; Solution: 1.0 mg/ml EtOH/MeOH 1:1 ; Injection:5.0 µl ; Detection:; DAD 280 nm; Rt in min 4.04 |
| 1-2 | Enantiomer 2 of Ex. 1 | | Rt in min 6.83 |
| 2 | 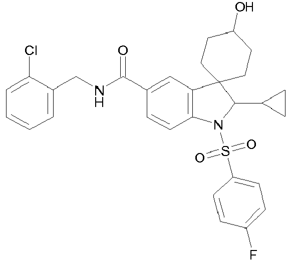 | N-(2-chlorobenzyl)-2'-cyclopropyl-1'-[(4-fluorophenyl)sulfo nyl]-4-hydroxy-1',2'-dihydrospiro[cyclo hexane-1,3'-indole]-5'-carboxamide | [ca. 85:15 Mixture of Diastereomers]; ¹H-NMR (300MHz, DMSO-d6, major isomer): Shift [ppm]= -0.17 - -0.12 (m, 1H), 0.32 - 0.46 (m, 2H), 0.54 - 0.72 (m, 2H), 0.83 - 1.17 (m, 4H), 1.48 - 1.57 (m, 1H), 1.70 - 1.82 (m, 2H), 2.19 - 2.29 (m, 1H), 3.76 (br. s., 1H), 3.91 (d, 1H), 4.42 (d, 1H) [minor isomer: 4.57 (d, 1H)], 4.52 (d, 2H), 7.26 - 7.46 (m, 6H), 7.55 (d, 1H), 7.77 (s, 1H) [minor isomer: 7.70 (d, 1H)], 7.83 - 7.88 (m, 3H), 9.04 (t, 1H) [minor isomer: 8.93 (t, 1H)] |
| | | | UPLC-MS (ESI+): [M + H]+ = 569/571 (Cl isotope pattern).; Rt in min 1.30; 1.33 |
| 3 | 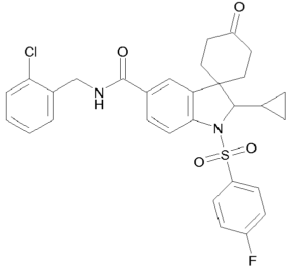 | N-(2-chlorobenzyl)-2'-cyclopropyl-1'-[(4-fluorophenyl)sulfo nyl]-4-oxo-1',2'-dihydrospiro[cyclo hexane-1,3'-indole]-5'-carboxamide | ¹H-NMR (300MHz, DMSO-d6): Shift [ppm]= 0.30 - 0.35 (m, 1H), 0.39 - 0.67 (m, 3H), 0.80 - 0.88 (m, 1H), 1.03 - 1.15 (m, 1H), 1.22 (dt, 1H), 1.74 - 1.79 (m, 1H), 2.32 - 2.36 (m, 3H), 2.54 - 2.73 (m, 2H), 4.27 (d, 1H), 4.51 (d, 2H), 7.28 - 7.46 (m, 6H), 7.59 (d, 1H), 7.80 (d, 1H), 7.86 - 7.92 (m, 3H), 8.92 (t, 1H) |
| | | | UPLC-MS (ESI+): [M + H]+ = 567/569 (Cl isotope pattern).; Rt in min 1.36 |
| 3-1 | Enantiomer 1 of Ex. 3 | | System: Waters: Alliance 2695, DAD 996, ESA: Corona; Column: Chiralcel OZ-H 5µm 150x4.6 mm; Solvent: ethanol / methanol 50:50 (v/v); Flow: 1.0 ml/min; Temperature: 25°C; Solution: 1.0 mg/ml EtOH / MeOH 1:1; Injection: 5.0 µl; Detection: DAD 280 nm; #1: Rt in min 2.33/3.78 (rotamers) |
| 3-2 | Enantiomer 2 of Ex. 3 | | Rt in min 2.81/4.54 (rotamers) |
| 4 | 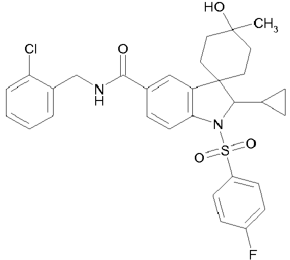 | N-(2-chlorobenzyl)-2'-cyclopropyl-1'-[(4-fluorophenyl)sulfo nyl]-4-hydroxy-4-methyl-1',2'-dihydrospiro[cyclo hexane-1,3'-indole]-5'-carboxamide | [ca. 90:10 Mixture of Diastereomers]; ¹H-NMR (400MHz, DMSO-d6, major isomer): Shift [ppm]= -0.30 (d, 1H), 0.34 - 0.42 (m, 2H), 0.54 - 0.61 (m, 1H), 0.66 - 0.73 (m, 1H), 0.91 - 0.97 (m, 1H), 1.01 - 1.04 (m, 1H), 1.12 - 1.18 (m, 4H), 1.41 - 1.58 (m, 3H), 1.81 - 1.84 (m, 1H), 2.24 (dt, 1H), 3.98 (d, 1H), 4.05 (s, 1H), 4.52 (d, 2H), 7.26 - 7.46 (m, 6H), 7.55 (d, 1 H), 7.72 (d, 1H), 7.82 - 7.86 (m, 3H), 9.02 (t, 1H) [minor isomer: 8.94 (t, 1H)] |
| | | | UPLC-MS (ESI+): [M + H]+ = 583/585 (Cl isotope pattern).; Rt in min 1.38 |
| 5 | 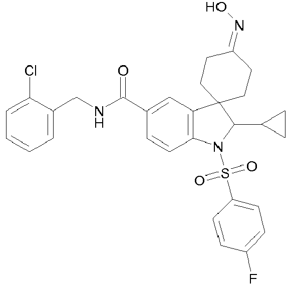 | N-(2-chlorobenzyl)-2'-cyclopropyl-1'-[(4-fluorophenyl)sulfo nyl]-4-(hydroxyimino)-1',2'-dihydrospiro[cyclo hexane-1,3'-indole]-5'-carboxamide | [ca. 1:1 Mixture of E/Z-Isomers]; ¹H-NMR (300MHz, DMSO-d6): Shift [ppm]= 0.09 - 0.24 (m, 1H), 0.37 - 0.55 (m, 2H), 0.58 - 0.67 (m, 1H), 0.74 - 0.95 (m, 2H), 0.98 - 1.07 (m, 1H), 1.77 - 2.07 (m, 3H), 2.19 - 2.36 (m, 2H), 2.50 - 2.61 (m, 1H), 4.09 - 4.14 (m, 1H), 4.51 (d, 2H), 7.25 - 7.46 (m, 6H), 7.58 (d, 1H), 7.78 (dd, 1H), 7.84 - 7.92 (m, 3H), 8.91 (t, 1H), 10.27 - 10.32 (m, 1H) |
| | | | UPLC-MS (ESI+): [M + H]+ = 582/584 (Cl isotope pattern).; Rt in min 1.31 |
| 6 | 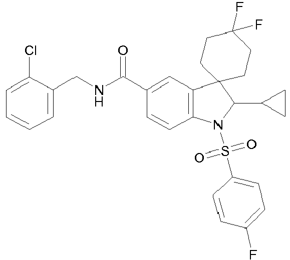 | N-(2-chlorobenzyl)-2'-cyclopropyl-4,4-difluoro-1'-[(4-fluorophenyl)sulfo nyl]-1',2'-dihydrospiro[cyclo hexane-1,3'-indole]-5'-carboxamide | ¹H-NMR (400MHz, DMSO-d6): Shift [ppm]= 0.09 - 0.12 (m, 1H), 0.37 - 0.64 (m, 3H), 0.74 - 0.80 (m, 1H), 0.97 - 1.04 (m, 2H), 1.47 - 1.59 (m, 1H), 1.97 - 2.21 (m, 4H), 2.50 - 2.58 (m, 1H), 4.13 (d, 1H), 4.52 (d, 2H), 7.27 - 7.46 (m, 6H), 7.55 - 7.61 (m, 1H), 7.73 - 7.77 (m, 1H), 7.84 - 7.94 (m, 3H), 8.91 - 8.98 (m, 1H) |
| | | | UPLC-MS (ESI+): [M + H]+ = 589/591 (Cl isotope pattern).; Rt in min 1.49 |
| 7 | 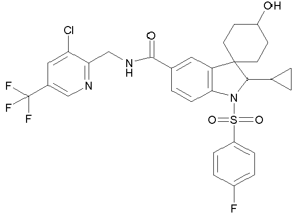 | N-{[3-chloro-5-(trifluoromethyl)py ridin-2-yl]methyl}-2'-cyclopropyl-1'-[(4-fluorophenyl)sulfo nyl]-4-hydroxy-1',2'-dihydrospiro[cyclo hexane-1,3'-indole]-5'-carboxamide | [ca. 75:25 Mixture of Diastereomers]; ¹H-NMR (300MHz, DMSO-d6, major isomer): Shift [ppm]= -0.20 - -0.13 (m, 1H), 0.35 - 0.44 (m, 2H), 0.56 - 0.70 (m, 2H), 0.83 - 1.17 (m, 4H), 1.47 - 1.56 (m, 1H), 1.70 - 1.81 (m, 2H), 2.19 - 2.27 (m, 1H), 3.76 (br. s., 1H), 3.92 (d, 1H), 4.43 (d, 1H) [minor isomer: 4.45 (d, 1H)], 4.72 (d, 2H), 7.37 - 7.43 (m, 2H), 7.55 (d, 1H) [minor isomer: 7.60 (d, 1H)], 7.76 (d, 1H) [minor isomer: 7.70 (s, 1H)], 7.81 - 7.91 (m, 3H), 8.47 (s, 1H), 8.90 (s, 1H), 9.10 (t, 1H) |
| | | | UPLC-MS (ESI+): [M + H]+ = 638/640 (Cl isotope pattern).; Rt in min 1.36; 1.39 |
| 8 | 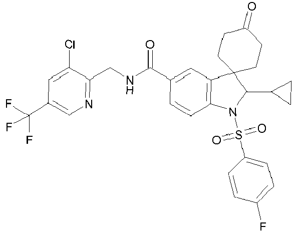 | N-{[3-chloro-5-(trifluoromethyl)py ridin-2-yl]methyl}-2'-cyclopropyl-1'-[(4-fluorophenyl)sulfo nyl]-4-oxo-1',2'-dihydrospiro[cyclo hexane-1,3'-indole]-5'-carboxamide | ¹H-NMR (300MHz, DMSO-d6): Shift [ppm]= 0.33 - 0.38 (m, 1H), 0.41 - 0.67 (m, 3H), 0.80 - 0.88 (m, 1H), 1.05 - 1.26 (m, 2H), 1.75 - 1.80 (m, 1H), 2.32 - 2.37 (m, 3H), 2.57 - 2.69 (m, 2H), 4.26 (d, 1H), 4.72 (d, 2H), 7.38 - 7.44 (m, 2H), 7.59 (d, 1H), 7.79 (s, 1H), 7.84 - 7.92 (m, 3H), 8.45 (d, 1H), 8.89 (d, 1H), 8.95 (t, 1H) |
| | | | UPLC-MS (ESI+): [M + H]+ = 636/638 (Cl isotope pattern).; Rt in min 1.39 |
| 9 | 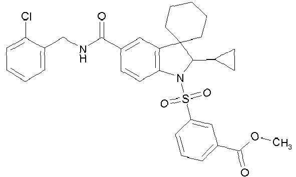 | methyl 3-({5'-[(2-chlorobenzyl)carb amoyl]-2'-cyclopropylspiro[c yclohexane-1,3'-indol]-1'(2'H)-yl}sulfonyl)benzoa te | ¹H-NMR (400MHz, CDCl3): Shift [ppm]= 0.18 (d, 1H), 0.38 - 0.45 (m, 1H), 0.51 - 0.57 (m, 1H), 0.64 - 0.77 (m, 3H), 0.95 - 1.02 (m, 1H), 1.18 - 1.30 (m, 3H), 1.41 - 1.52 (m, 1H), 1.63 - 1.66 (m, 1H), 1.77 - 1.84 (m, 2H), 2.12 - 2.16 (m, 1H), 3.92 (s, 3H), 4.00 (d, 1H), 4.72 (d, 2H), 6.51 (t, 1H), 7.21 - 7.26 (m, 2H), 7.39 - 7.42 (m, 1H), 7.46 - 7.50 (m, 2H), 7.54 - 7.63 (m, 3H), 7.89 - 7.92 (m, 1H), 8.16 - 8.18 (m, 1H), 8.47 - 8.48 (m, 1H) |
| | | | UPLC-MS (ESI+): [M + H]+ = 593/595 (Cl isotope pattern).; Rt in min 1.54 |
| 10 | 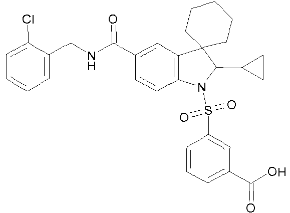 | 3-({5'-[(2-chlorobenzyl)carb amoyl]-2'-cyclopropylspiro[c yclohexane-1,3'-indol]-1'(2'H)-yl}sulfonyl)benzoic acid | ¹H-NMR (300MHz, DMSO-d6): Shift [ppm]= -0.03 (d, 1H), 0.34 - 0.47 (m, 2H), 0.57 - 0.78 (m, 3H), 0.92 - 1.01 (m, 1H), 1.06 - 1.19 (m, 2H), 1.25 - 1.35 (m, 1H), 1.42 - 1.59 (m, 2H), 1.70 - 1.74 (m, 1H), 1.83 (dt, 1H), 2.04 - 2.08 (m, 1 H), 3.97 (d, 1H), 4.51 (d, 2H), 7.25 - 7.37 (m, 3H), 7.43 - 7.46 (m, 1H), 7.55 (d, 1H), 7.67 (t, 1H), 7.72 (d, 1H), 7.85 (dd, 1H), 7.99 - 8.01 (m, 1H), 8.15 - 8.18 (m, 1H), 8.26 - 8.27 (m, 1H), 8.95 (t, 1H) |
| | | | UPLC-MS (ESI+): [M + H]+ = 579/581 (Cl isotope pattern).; Rt in min 1.40 |
| 11 | 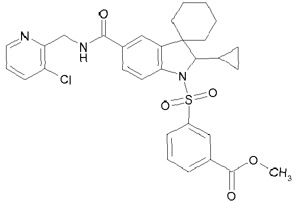 | methyl 3-{[5'-{[(3-chloropyridin-2-yl)methyl]carbamo yl}-2'-cyclopropylspiro[c yclohexane-1,3'-indol]-1'(2'H)-yl]sulfonyl}benzoa te | ¹H-NMR (300MHz, CDCl3): Shift [ppm]= 0.21 (d, 1H), 0.38 - 0.48 (m, 1H), 0.52 - 0.61 (m, 1H), 0.64 - 0.83 (m, 3H), 0.95 - 1.06 (m, 1H), 1.19 - 1.27 (m, 3H), 1.42 - 1.52 (m, 1H), 1.65 - 1.67 (m, 1H), 1.81 - 1.91 (m, 2H), 2.15 - 2.19 (m, 1H), 3.93 (s, 3H), 4.01 (d, 1H), 4.83 (d, 2H), 7.23 - 7.27 (m, 1H), 7.49 (t, 1H), 7.65 - 7.67 (m, 2H), 7.72 - 7.76 (m, 2H), 7.82 (t, 1H), 7.92 - 7.95 (m, 1 H), 8.16 - 8.20 (m, 1 H), 8.49 - 8.53 (m, 2H) |
| | | | UPLC-MS (ESI+): [M + H]+ = 594/596 (Cl isotope pattern).; Rt in min 1.46 |
| 12 | 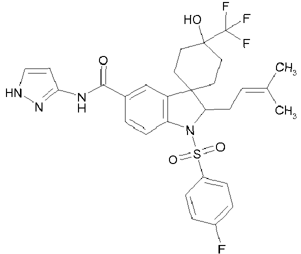 | 1'-[(4-fluorophenyl)sulfo nyl]-4-hydroxy-2'-(3-methylbut-2-en-1-yl)-N-(1H-pyrazol-3-yl)-4-(trifluoromethyl)-1',2'-dihydrospiro[cyclo hexane-1,3'-indole]-5'-carboxamide | UPLC-MS (ESI+): [M + H]+ = 607 Rt in min 1.37 |
| 13 | 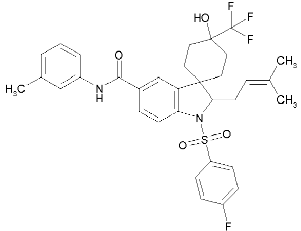 | 1'-[(4-fluorophenyl)sulfo nyl]-4-hydroxy-2'-(3-methylbut-2-en-1-yl)-N-(3-methylphenyl)-4-(trifluoromethyl)-1',2'-dihydrospiro[cyclo hexane-1,3'-indole]-5'-carboxamide | UPLC-MS (ESI+): [M + H]+ = 631 Rt in min 1.57 |
| 14 | 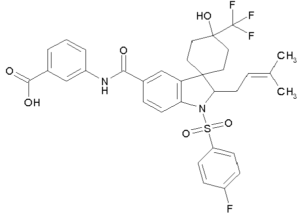 | 3-[({1'-[(4-fluorophenyl)sulfo nyl]-4-hydroxy-2'-(3-methylbut-2-en-1-yl)-4-(trifluoromethyl)-1',2'-dihydrospiro[cyclo hexane-1,3'-indol]-5'-yl}carbonyl)amino] benzoic acid | UPLC-MS (ESI+): [M + H]+ = 661 Rt in min 1.40 |
| 15 | 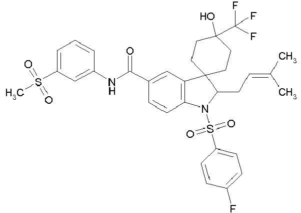 | 1'-[(4-fluorophenyl)sulfo nyl]-4-hydroxy-2'-(3-methylbut-2-en-1-yl)-N-[3-(methylsulfonyl)ph enyl]-4-(trifluoromethyl)-1',2'-dihydrospiro[cyclo hexane-1,3'-indole]-5'-carboxamide | UPLC-MS (ESI+): [M + H]+ = 695 Rt in min 1.45 |
| 16 | 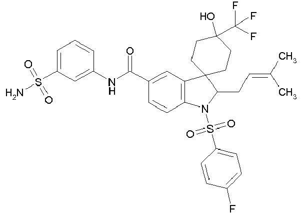 | 1'-[(4-fluorophenyl)sulfo nyl]-4-hydroxy-2'-(3-methylbut-2-en-1-yl)-N-(3-sulfamoylphenyl)-4-(trifluoromethyl)-1',2'-dihydrospiro[cyclo hexane-1,3'-indole]-5'-carboxamide | UPLC-MS (ESI+): [M + H]+ = 696 Rt in min 1.41 |
| 17 | 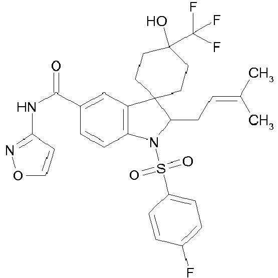 | 1'-[(4-fluorophenyl)sulfo nyl]-4-hydroxy-2'-(3-methylbut-2-en-1-yl)-N-(1,2-oxazol-3-yl)-4-(trifluoromethyl)-1',2'-dihydrospiro[cyclo hexane-1,3'-indole]-5'-carboxamide | UPLC-MS (ESI+): [M + H]+ = 608 Rt in min 1.43 |
| 18 | 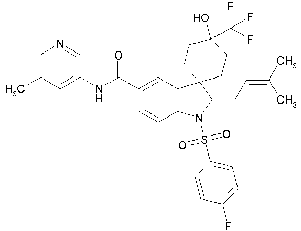 | 1'-[(4-fluorophenyl)sulfo nyl]-4-hydroxy-2'-(3-methylbut-2-en-1-yl)-N-(5-methylpyridin-3-yl)-4-(trifluoromethyl)-1',2'-dihydrospiro[cyclo hexane-1,3'-indole]-5'-carboxamide | UPLC-MS (ESI+): [M + H]+ = 632 Rt in min 1.32 |
| 19 | 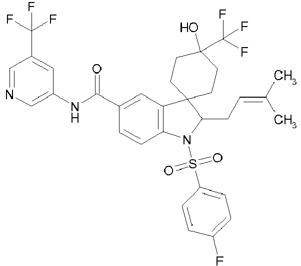 | 1'-[(4-fluorophenyl)sulfo nyl]-4-hydroxy-2'-(3-methylbut-2-en-1-yl)-4-(trifluoromethyl)-N-[5-(trifluoromethyl)py ridin-3-yl]-1',2'-dihydrospiro[cyclo hexane-1,3'-indole]-5'-carboxamide | UPLC-MS (ESI+): [M + H]+ = 686 Rt in min 1.55 |
| 20 | 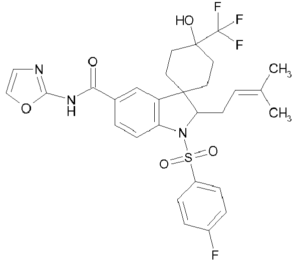 | 1'-[(4-fluorophenyl)sulfo nyl]-4-hydroxy-2'-(3-methylbut-2-en-1-yl)-N-(1,3-oxazol-2-yl)-4-(trifluoromethyl)-1',2'-dihydrospiro[cyclo hexane-1,3'-indole]-5'-carboxamide | UPLC-MS (ESI+): [M + H]+ = 608 Rt in min 1.39 |
| 21 | 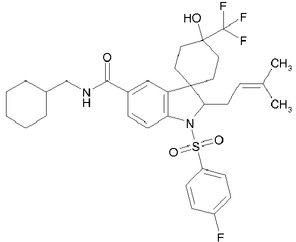 | N-(cyclohexylmethyl) -1'-[(4-fluorophenyl)sulfo nyl]-4-hydroxy-2'-(3-methylbut-2-en-1-yl)-4-(trifluoromethyl)-(trifluoromethyl)-1',2'-dihydrospiro[cyclo hexane-1,3'-indole]-5'-carboxamide | UPLC-MS (ESI+): [M + H]+ = 637 Rt in min 1.56 |
| 22 | 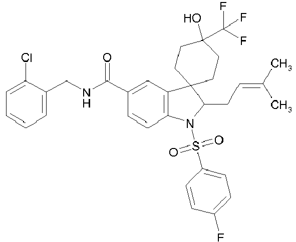 | N-(2-chlorobenzyl)-1'-[(4-fluorophenyl)sulfo nyl]-4-hydroxy-2'-(3-methylbut-2-en-1-yl)-4-(trifluoromethyl)-1',2'-dihydrospiro[cyclo hexane-1,3'-indole]-5'-carboxamide | UPLC-MS (ESI+): [M + H]+ = 665 Rt in min 1.52 |
| 23 | 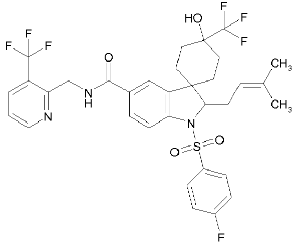 | 1'-[(4-fluorophenyl)sulfo nyl]-4-hydroxy-2'-(3-methylbut-2-en-1-yl)-4-(trifluoromethyl)-N-{[3-(trifluoromethyl)py ridin-2-yl]methyl}-1',2'-dihydrospiro[cyclo hexane-1,3'-indole]-5'-carboxamide | UPLC-MS (ESI+): [M + H]+ = 700 Rt in min 1.48 |
| 24 | 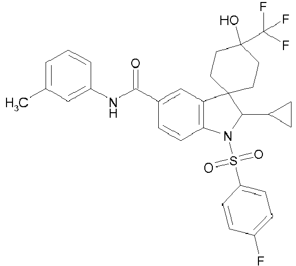 | 2'-cyclopropyl-1'-[(4-fluorophenyl)sulfo nyl]-4-hydroxy-N-(3-methylphenyl)-4-(trifluoromethyl)-1',2'-dihydrospiro[cyclo hexane-1,3'-indole]-5'-carboxamide | UPLC-MS (ESI+): [M + H]+ = 603 Rt in min 1.45 |
| 25 | 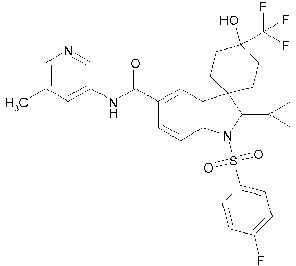 | 2'-cyclopropyl-1'-[(4-fluorophenyl)sulfo nyl]-4-hydroxy-N-(5-methylpyridin-3-yl)-4-(trifluoromethyl)-1',2'-dihydrospiro[cyclo hexane-1,3'-indole]-5'-carboxamide | UPLC-MS (ESI+): [M + H]+ = 604 Rt in min 1.17 |
| 26 | 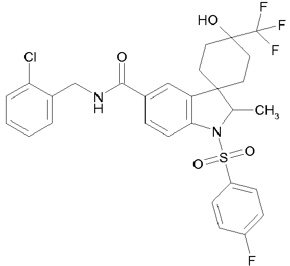 | N-(2-chlorobenzyl)-1'-[(4-fluorophenyl)sulfo nyl]-4-hydroxy-2'-methyl-4-(trifluoromethyl)-1',2'-dihydrospiro[cyclo hexane-1,3'-indole]-5'-carboxamide | UPLC-MS (ESI+): [M + H]+ = 611 Rt in min 1.39 |
| 27 | 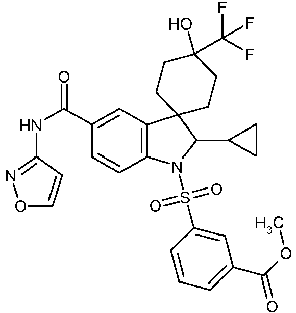 | methyl 3-{[2'-cyclopropyl-4-hydroxy-5'-(1,2-oxazol-3-ylcarbamoyl)-4-(trifluoromethyl)spi ro[cyclohexane-1,3'-indol]-1'(2'H)-yl]sulfonyl}benzoa te | UPLC-MS (ESI+): [M + H]+ = 620 Rt in min 1.37 |
| 28 | 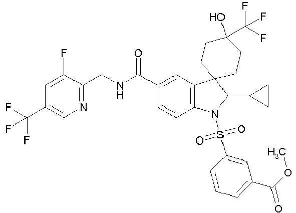 | methyl 3-{[2'-cyclopropyl-5'-({[3-fluoro-5-(trifluoromethyl)py ridin-2-yl]methyl}carbamo yl)-4-hydroxy-4-(trifluoromethyl)spi ro[cyclohexane-1,3'-indol]-1'(2'H)-yl]sulfonyl}benzoa te | UPLC-MS (ESI+): [M + H]+ = 730 Rt in min 1.43 |
| 29 | 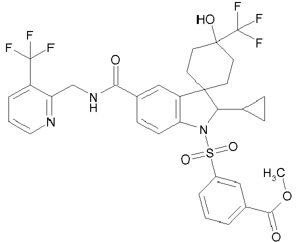 | methyl 3-{[2'-cyclopropyl-4-hydroxy-4-(trifluoromethyl)-5'-({[3-(trifluoromethyl)py ridin-2-yl]methyl}carbamo yl)spiro[cyclohexa ne-1,3'-indol]-1'(2'H)-yl]sulfonyl}benzoa te | UPLC-MS (ESI+): [M + H]+ = 712 Rt in min 1.40 |
| 30 | 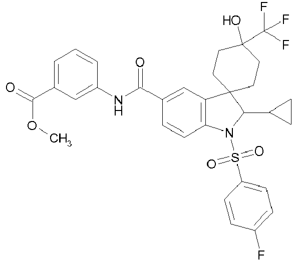 | methyl 3-[({2'-cyclopropyl-1'-[(4-fluorophenyl)sulfo nyl]-4-hydroxy-4-(trifluoromethyl)-1',2'-dihydrospiro[cyclo hexane-1,3'-indol]-5'-yl}carbonyl)amino] benzoate | UPLC-MS (ESI+): [M + H]+ = 647 Rt in min 1.42 |
| 30-1 | Enantiomer 1 of Ex. 30 | | System: Waters: Alliance 2695, DAD 996, ESA: Corona; Column: Chiralpak IA 5µm 150x4.6 mm; Solvent: hexane / ethanol 90:10(v/v); Flow: 1.0 ml/min; Temperature: 25°C; Solution: 1.0 mg/ml EtOH/MeOH 2:1; Injection: 5.0 µl; Detection: DAD 280 nm; Rt in min 12.9 |
| 30-2 | Enantiomer 2 of Ex. 30 | | Rt in min 17.2 |
| 31 | 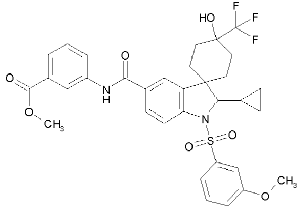 | methyl 3-[({2'-cyclopropyl-4-hydroxy-1'-[(3-methoxyphenyl)su Ifonyl]-4-(trifluoromethyl)-1',2'-dihydrospiro[cyclo hexane-1,3'-indol]-5'-yl}carbonyl)amino] benzoate | UPLC-MS (ESI+): [M + H]+ = 659 Rt in min 1.43 |
| 32 | 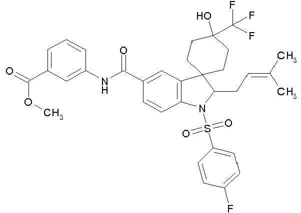 | methyl 3-[({1'-[(4-fluorophenyl)sulfo nyl]-4-hyd roxy-2'-(3-methylbut-2-en-1-yl)-4-(trifluoromethyl)-1',2'-dihydrospiro[cyclo hexane-1,3'-indol]-5'-yl}carbonyl)amino] benzoate | UPLC-MS (ESI+): [M + H]+ = 675 Rt in min 1.54 |
| 33 | 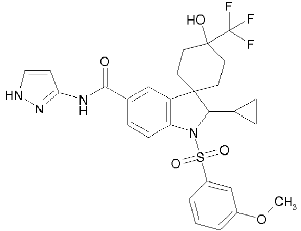 | 2'-cyclopropyl-4-hydroxy-1'-[(3-methoxyphenyl)su Ifonyl]-N-(1H-pyrazol-3-yl)-4-(trifluoromethyl)-1',2'-dihydrospiro[cyclo hexane-1,3'-indole]-5'-carboxamide | UPLC-MS (ESI+): [M + H]+ = 591 Rt in min 1.30 |
| 34 | 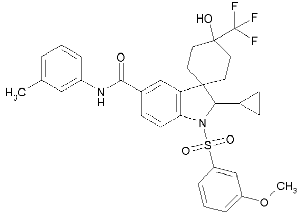 | 2'-cyclopropyl-4-hydroxy-1'-[(3-methoxyphenyl)su Ifonyl]-N-(3-methylphenyl)-4-(trifluoromethyl)-1',2'-dihydrospiro[cyclo hexane-1,3'-indole]-5'-carboxamide | UPLC-MS (ESI+): [M + H]+ = 615 Rt in min 1.46 |
| 35 | 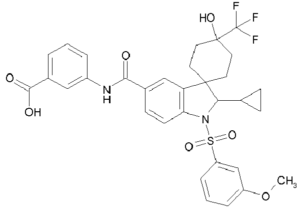 | 3-[({2'-cyclopropyl-4-hyd roxy-1 '-[(3-methoxyphenyl)su Ifonyl]-4-(trifluoromethyl)-1',2'-dihydrospiro[cyclo hexane-1,3'-indol]-5'-yl}carbonyl)amino] benzoic acid | UPLC-MS (ESI+): [M + H]+ = 645 Rt in min 1.33 |
| 36 | 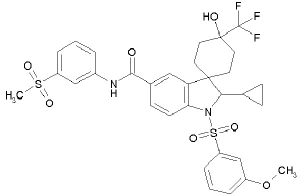 | 2'-cyclopropyl-4-hydroxy-1'-[(3-methoxyphenyl)su Ifonyl]-N-[3-(methylsulfonyl)ph enyl]-4-(trifluoromethyl)-1',2'-dihydrospiro[cyclo hexane-1,3'-indole]-5'-carboxamide | UPLC-MS (ESI+): [M + H]+ = 679 Rt in min 1.34 |
| 37 | 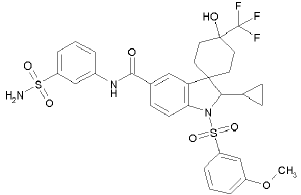 | 2'-cyclopropyl-4-hydroxy-1'-[(3-methoxyphenyl)su Ifonyl]-N-(3-sulfamoylphenyl)-4-(trifluoromethyl)-1',2'-dihydrospiro[cyclo hexane-1,3'-indole]-5'-carboxamide | UPLC-MS (ESI+): [M + H]+ = 680 Rt in min 1.29 |
| 38 | 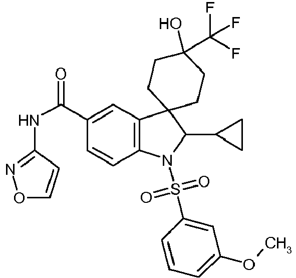 | 2'-cyclopropyl-4-hydroxy-1'-[(3-methoxyphenyl)su Ifonyl]-N-(1,2-oxazol-3-yl)-4-(trifluoromethyl)-1',2'-dihydrospiro[cyclo hexane-1,3'-indole]-5'-carboxamide | UPLC-MS (ESI+): [M + H]+ = 592 Rt in min 1.29 |
| 39 | 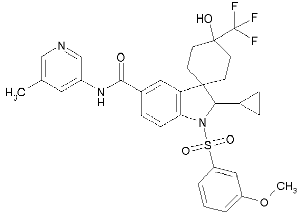 | 2'-cyclopropyl-4-hydroxy-1'-[(3-methoxyphenyl)su Ifonyl]-N-(5-methylpyridin-3-yl)-4-(trifluoromethyl)-1',2'-dihydrospiro[cyclo hexane-1,3'-indole]-5'-carboxamide | UPLC-MS (ESI+): [M + H]+ = 616 Rt in min 1.20 |
| 40 | 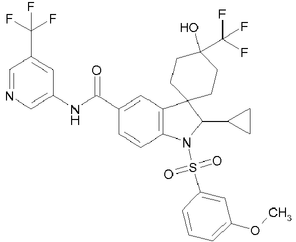 | 2'-cyclopropyl-4-hydroxy-1'-[(3-methoxyphenyl)su Ifonyl]-4-(trifluoromethyl)-N-[5-(trifluoromethyl)py ridin-3-yl]-1',2'-dihydrospiro[cyclo hexane-1,3'-indole]-5'-carboxamide | UPLC-MS (ESI+): [M + H]+ = 670 Rt in min 1.45 |
| 41 | 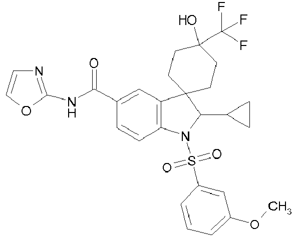 | 2'-cyclopropyl-4-hydroxy-1'-[(3-methoxyphenyl)su Ifonyl]-N-(1,3-oxazol-2-yl)-4-(trifluoromethyl)-1',2'-dihydrospiro[cyclo hexane-1,3'-indole]-5'-carboxamide | UPLC-MS (ESI+): [M + H]+ = 592 Rt in min 1.27 |
| 42 | 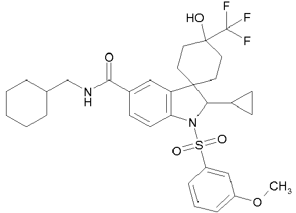 | N-(cyclohexylmethyl) -2'-cyclopropyl-4-hydroxy-1'-[(3-methoxyphenyl)su Ifonyl]-4-(trifluoromethyl)-1',2'-dihydrospiro[cyclo hexane-1,3'-indole]-5'-carboxamide | UPLC-MS (ESI+): [M + H]+ = 621 Rt in min 1.49 |
| 43 | 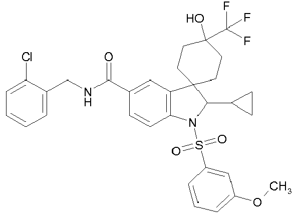 | N-(2-chlorobenzyl)-2'-cyclopropyl-4-hydroxy-1'-[(3-methoxyphenyl)su Ifonyl]-4-(trifluoromethyl)-1',2'-dihydrospiro[cyclo hexane-1,3'-indole]-5'-carboxamide | UPLC-MS (ESI+): [M + H]+ = 649 Rt in min 1.44 |
| 44 | 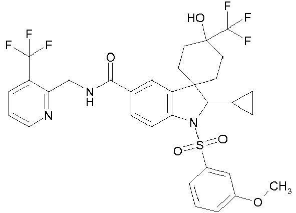 | 2'-cyclopropyl-4-hydroxy-1'-[(3-methoxyphenyl)su Ifonyl]-4-(trifluoromethyl)-N-{[3-(trifluoromethyl)py ridin-2-yl]methyl}-1',2'-dihydrospiro[cyclo hexane-1,3'-indole]-5'-carboxamide | UPLC-MS (ESI+): [M + H]+ = 684 Rt in min 1.40 |
| 45 | 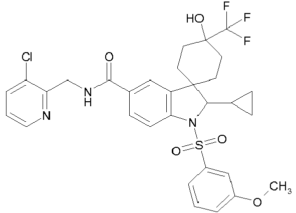 | N-[(3-chloropyridin-2-yl)methyl]-2'-cyclopropyl-4-hydroxy-1'-[(3-methoxyphenyl)su Ifonyl]-4-(trifluoromethyl)-1',2'-dihydrospiro[cyclo hexane-1,3'-indole]-5'-carboxamide | UPLC-MS (ESI+): [M + H]+ = 650 Rt in min 1.39 |
| 46 | 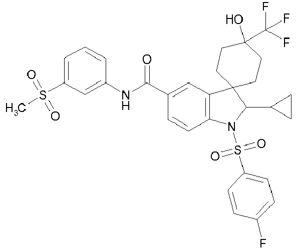 | 2'-cyclopropyl-1'-[(4-fluorophenyl)sulfo nyl]-4-hydroxy-N-[3-(methylsulfonyl)ph enyl]-4-(trifluoromethyl)-1',2'-dihydrospiro[cyclo hexane-1,3'-indole]-5'-carboxamide | UPLC-MS (ESI+): [M + H]+ = 667 Rt in min 1.37 |
| 47 | 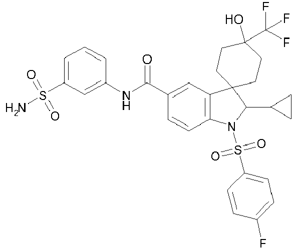 | 2'-cyclopropyl-1 [(4-fluorophenyl)sulfo nyl]-4-hydroxy-N-(3-sulfamoylphenyl)-4-(trifluoromethyl)-1',2'-dihydrospiro[cyclo hexane-1,3'-indole]-5'-carboxamide | UPLC-MS (ESI+): [M + H]+ = 668 Rt in min 1.32 |
| 48 | 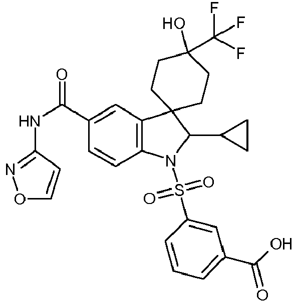 | 3-{[2'-cyclopropyl-4-hydroxy-5'-(1,2-oxazol-3-ylcarbamoyl)-4-(trifluoromethyl)spi ro[cyclohexane-1,3'-indol]-1'(2'H)-yl]sulfonyl}benzoic acid | UPLC-MS (ESI+): [M + H]+ = 605 Rt in min 1.20 |
| 49 | 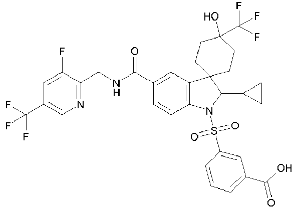 | 3-{[2'-cyclopropyl-5'-({[3-fluoro-5-(trifluoromethyl)py ridin-2-yl]methyl}carbamo yl)-4-hydroxy-4-(trifluoromethyl)spi ro[cyclohexane-1,3'-indol]-1'(2'H)-yl]sulfonyl}benzoic acid | UPLC-MS (ESI+): [M + H]+ = 716 Rt in min 1.30 |
| 50 | 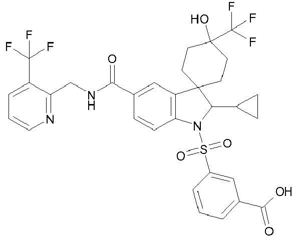 | 3-{[2'-cyclopropyl-4-hydroxy-4-(trifluoromethyl)-5'-({[3-(trifluoromethyl)py ridin-2-yl]methyl}carbamo yl)spiro[cyclohexa ne-1,3'-indol]-1'(2'H)-yl]sulfonyl}benzoic acid | UPLC-MS (ESI+): [M + H]+ = 698 Rt in min 1.27 |
| 51 | 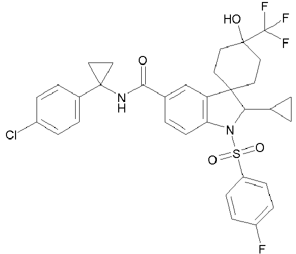 | N-[1-(4-chlorophenyl)cycl opropyl]-2'-cyclopropyl-1'-[(4-fluorophenyl)sulfo nyl]-4-hydroxy-4-(trifluoromethyl)-1',2'-dihydrospiro[cyclo hexane-1,3'-indole]-5'-carboxamide | UPLC-MS (ESI+): [M + H]+ = 663 Rt in min 1.51 |
| 52 | 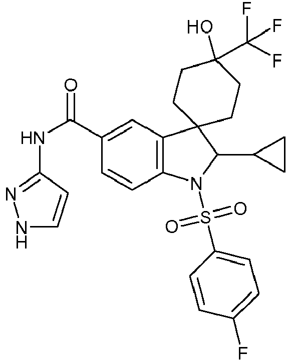 | 2'-cyclopropyl-1'-[(4-fluorophenyl)sulfo nyl]-4-hydroxy-N-(1H-pyrazol-3-yl)-4-(trifluoromethyl)-1',2'-dihydrospiro[cyclo hexane-1,3'-indole]-5'-carboxamide | UPLC-MS (ESI+): [M + H]+ = 579 Rt in min 1.30 |
| 53 | 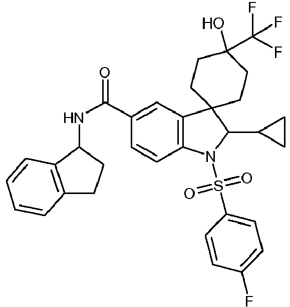 | 2'-cyclopropyl-N-(2,3-dihydro-1H-inden-1-yl)-1'-[(4-fluorophenyl)sulfo nyl]-4-hydroxy-4-(trifluoromethyl)-1',2'-dihydrospiro[cyclo hexane-1,3'-indole]-5'-carboxamide | UPLC-MS (ESI+): [M + H]+ = 629 Rt in min 1.45 |
| 54 | 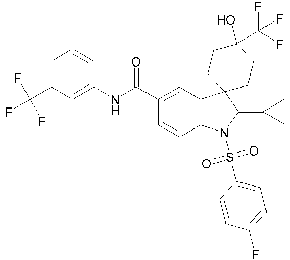 | 2'-cyclopropyl-1 [(4-fluorophenyl)sulfo nyl]-4-hydroxy-4-(trifluoromethyl)-N-[3-(trifluoromethyl)ph enyl]-1',2'-dihydrospiro[cyclo hexane-1,3'-indole]-5'-carboxamide | UPLC-MS (ESI+): [M + H]+ = 657 Rt in min 1.51 |
| 55 | 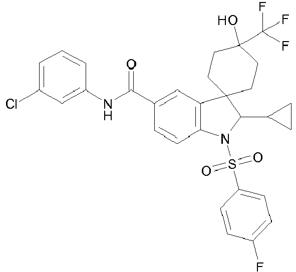 | N-(3-chlorophenyl)-2'-cyclopropyl-1'-[(4-fluorophenyl)sulfo nyl]-4-hydroxy-4-(trifluoromethyl)-1',2'-dihydrospiro[cyclo hexane-1,3'-indole]-5'-carboxamide | UPLC-MS (ESI+): [M + H]+ = 623 Rt in min 1.49 |
| 56 | 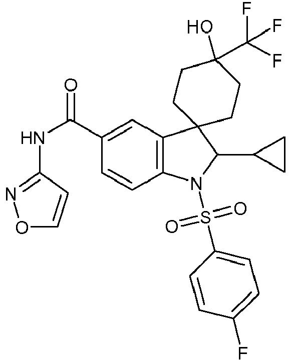 | 2'-cyclopropyl-1' [(4-fluorophenyl)sulfo nyl]-4-hydroxy-N-(1,2-oxazol-3-yl)-4-(trifluoromethyl)-1',2'-dihydrospiro[cyclo hexane-1,3'-indole]-5'-carboxamide | ¹H-NMR (400MHz, DMSO-d6): Shift [ppm]= -0.23 (d, 1H), 0.35 - 0.42 (m, 1 H), 0.45 - 0.52 (m, 1H), 0.54 - 0.65 (m, 2H), 0.92 - 1.00 (m, 1H), 1.18 - 1.26 (m, 2H), 1.64 - 1.79 (m, 3H), 1.94 - 2.00 (m, 1H), 2.20 - 2.26 (m, 1H), 3. 94 (d, 1 H), 5.75 (s, 1H), 6.99 (d, 1H), 7.36 - 7. 40 (m, 2H), 7.59 (d, 1H), 7.86 - 7.91 (m, 3H), 7.95 (dd, 1H), 8.81 (d, 1H), 11.43 (s, 1H) UPLC-MS (ESI+): [M + H]+ = 580 Rt in min 1.36 |
| 56-1 | Enantiomer 1 of Ex. 56 | | System: Waters: Alliance 2695, DAD 996, ESA: Corona; Column: Chiralpak IC 5µm 150x4.6 mm; Solvent: hexane / ethanol 70:30(v/v); Flow: 1.0 ml/min; Temperature: 25°C; Solution: 1.0 mg/ml EtOH/MeOH 1:1; Injection: 5.0 µl; Detection: DAD 280 nm; Rt in min 2.75 |
| 56-2 | Enantiomer 2 of Ex. 56 | | Rt in min 4.95 |
| 57 | 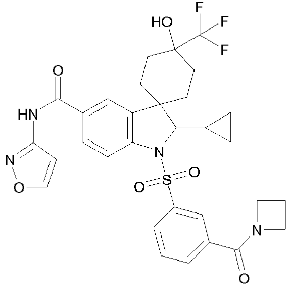 | 1'-{[3-(azetidin-1-ylcarbonyl)phenyl] sulfonyl}-2'-cyclopropyl-4-hydroxy-N-(1,2-oxazol-3-yl)-4-(trifluoromethyl)-1',2'-dihydrospiro[cyclo hexane-1,3'-indole]-5'-carboxamide | UPLC-MS (ESI+): [M + H]+ = 645 Rt in min 1.22 |
| 58 | 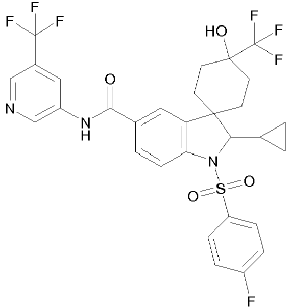 | 2'-cyclopropyl-1'-[(4-fluorophenyl)sulfo nyl]-4-hydroxy-4-(trifluoromethyl)-N-[5-(trifluoromethyl)py ridin-3-yl]-1',2'-dihydrospiro[cyclo hexane-1,3'-indole]-5'-carboxamide | UPLC-MS (ESI+): [M + H]+ = 658 Rt in min 1.47 |
| 59 | 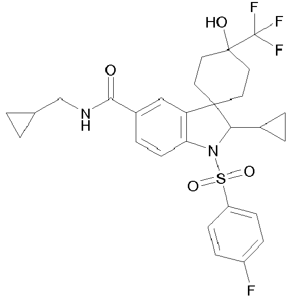 | 2'-cyclopropyl-N-(cyclopropylmethy l)-1'-[(4-fluorophenyl)sulfo nyl]-4-hydroxy-4-(trifluoromethyl)-1',2'-dihydrospiro[cyclo hexane-1,3'-indole]-5'-carboxamide | UPLC-MS (ESI+): [M + H]+ = 567 Rt in min 1.38 |
| 60 | 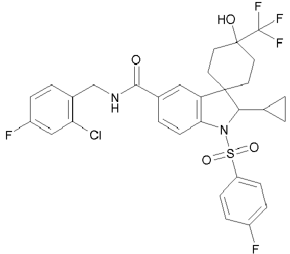 | N-(2-chloro-4-fluorobenzyl)-2'-cyclopropyl-1'-[(4-fluorophenyl)sulfo nyl]-4-hydroxy-4-(trifluoromethyl)-1',2'-dihydrospiro[cyclo hexane-1,3'-indole]-5'-carboxamide | UPLC-MS (ESI+): [M + H]+ = 655 Rt in min 1.49 |
| 61 | 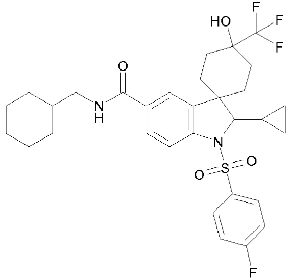 | N-(cyclohexylmethyl) -2'-cyclopropyl-1'-[(4-fluorophenyl)sulfo nyl]-4-hydroxy-4-(trifluoromethyl)-1',2'-dihydrospiro[cyclo hexane-1,3'-indole]-5'-carboxamide | UPLC-MS (ESI+): [M + H]+ = 609 Rt in min 1.52 |
| 62 | 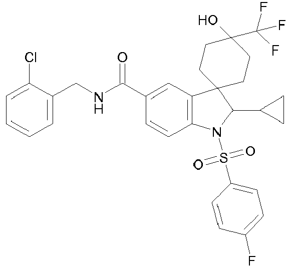 | N-(2-chlorobenzyl)-2'-cyclopropyl-1'-[(4-fluorophenyl)sulfo nyl]-4-hydroxy-4-(trifluoromethyl)-1',2'-dihydrospiro[cyclo hexane-1,3'-indole]-5'-carboxamide | ¹H-NMR (300MHz, DMSO-d6): Shift [ppm]= -0.22 (d, 1H), 0.33 - 0.43 (m, 1H), 0.44 - 0.50 (m, 1H), 0.52 - 0.65 (m, 2H), 0.89 - 1.01 (m, 1H), 1.06 - 1.28 (m, 2H), 1.60 - 1.78 (m, 3H), 1.91 - 2.01 (m, 1H), 2.14 - 2.26 (m, 1H),3. 93 (d, 1 H), 4.49 (d, 2H), 5.72 (s, 1H), 7.22 - 7.43 (m, 6H), 7.55 (d, 1H), 7.68 (d, 1H), 7.83 - 7.92 (m, 3H), 9.00 (t, 1H) |
| | | | UPLC-MS (ESI+): [M + H]+ = 637 Rt in min 1.45 |
| 62-1 | Enantiomer 1 of Ex. 62 | | System: Waters: Alliance 2695, DAD 996, ESA: Corona; Column: Chiralpak IA 5µm 150x4.6 mm; Solvent: hexane / IPA 80:20(v/v); Flow: 1.0 ml/min; Temperature: 25°C; Solution: 1.0 mg/ml EtOH/MeOH 1:1; Injection: 5.0 µl; Detection: DAD 280 nm; Rt in min 5.39 |
| 62-2 | Enantiomer 2 of Ex. 62 | | Rt in min 6.77 |
| 63 | 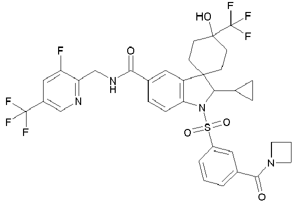 | 1'-{[3-(azetidin-1-ylcarbonyl)phenyl] sulfonyl}-2'-cyclopropyl-N-{[3-fluoro-5-(trifluoromethyl)py ridin-2-yl]methyl}-4-hydroxy-4-(trifluoromethyl)-1',2'-dihydrospiro[cyclo hexane-1,3'-indole]-5'-carboxamide | UPLC-MS (ESI+): [M + H]+ = 755 Rt in min 1.35 |
| 64 | 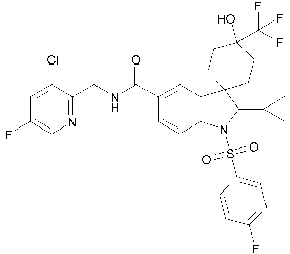 | N-[(3-chloro-5-fluoropyridin-2-yl)methyl]-2'-cyclopropyl-1'-[(4-fluorophenyl)sulfo nyl]-4-hydroxy-4-(trifluoromethyl)-1',2'-dihydrospiro[cyclo hexane-1,3'-indole]-5'-carboxamide | UPLC-MS (ESI+): [M + H]+ = 656 Rt in min 1.43 |
| 65 | 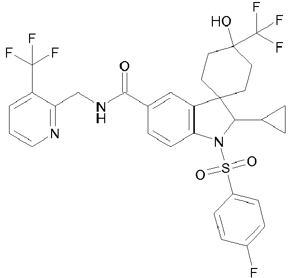 | 2'-cyclopropyl-1'-[(4-fluorophenyl)sulfo nyl]-4-hydroxy-4-(trifluoromethyl)-N-{[3-(trifluoromethyl)py ridin-2-yl]methyl}-1',2'-dihydrospiro[cyclo hexane-1,3'-indole]-5'-carboxamide | ¹H-NMR (400MHz, DMSO-d6): Shift [ppm]= -0.23 (d, 1H), 0.34 - 0.41 (m, 1H), 0.43 - 0.49 (m, 1H), 0.54 - 0.64 (m, 2H), 0.92 - 1.00 (m, 1H), 1.08 - 1.16 (m, 1H), 1.19 - 1.26 (m, 1H), 1.63 - 1.78 (m, 3H), 1.93 - 1.99 (m, 1H), 2.16 - 2.24 (m, 1H),3. 93 (d, 1H), 4.69 (d, 2H), 5.73 (s, 1H), 7.35 - 7. 39 (m, 2H), 7.50 (dd, 1H), 7.55 (d, 1H), 7.68 (d, 1 H), 7.82 - 7.92 (m, 3H), 8.14 (d, 1H), 8.76 (d, 1H), 8.99 (t, 1H) UPLC-MS (ESI+): [M + H]+ = 672 Rt in min 1.41 |
| 65-1 | Enantiomer 1 of Ex. 65 | | System: Dionex: Pump 680, ASI 100, Waters: UV-Detektor 2487; Column: Chiralpak AD-H 5µm 150x4.6 mm; Solvent: hexane / IPA / Diethylamine 80:20:0.1 (v/v/v); Flow: 1.0 ml/min; Temperature: 25°C; Solution: 1.0 mg/ml EtOH/MeOH 2:1; Injection: 5.0 µl; Detection: DAD 254 nm; Rt in min 3.91 |
| 65-2 | Enantiomer 1 of Ex. 65 | | Rt in min 6.29 |
| 66 | 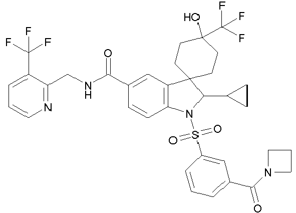 | 1'-{[3-(azetidin-1-ylcarbonyl)phenyl] sulfonyl}-2'-cyclopropyl-4-hydroxy-4-(trifluoromethyl)-N-{[3-(trifluoromethyl)py ridin-2-yl]methyl}-1',2'-dihydrospiro[cyclo hexane-1,3'-indole]-5'-carboxamide | UPLC-MS (ESI+): [M + H]+ = 737 Rt in min 1.30 |
| 66-1 | Enantiomer 1 of Ex. 66 | | System: Waters: Alliance 2695, DAD 996, ESA: Corona; Column:Chiralpak IC 3µm 100x4.6 mm; Solvent: hexane / ethanol 50:50(v/v); Flow: 1.0 ml/min; Temperature: 25°C; Solution: 1.0 mg/ml EtOH/MeOH 1:1; Injection: 5.0 µl; Detection: DAD 254 nm; Rt in min 2.69 |
| 66-2 | Enantiomer 2 of Ex. 66 | | Rt in min 4.55 |

### BIOLOGICAL ASSAYS

### 1. MATERIALS

Buserelin was purchased from Welding (Frankfurt/Main, Germany) or USbiological (#B8995, Swampscott, USA) for IP-One HTRF® assays and LHRH from Sigma-Aldrich® (Munich, Germany). Labelled cells, Tag-Lite buffer, labelled and unlabelled GnRHR binding peptide for Tag-lite® binding assay was purchased by Cisbio Bioassays (Bagnols-sur-Cèze Cedex, France). The radio labelling was performed in the Department of Isotope Chemistry of Bayer Pharma AG (Berlin, Germany) by the iodogen method using [¹²⁵I]sodium iodide (2000 Ci/mmol; PerkinElmer Life and Analytical Sciences, USA) yielding [¹²⁵I]monoiodo-buserelin. The radio-tracer was purified by reversed phase HPLC on a Spherisorb ODS II column (250 x 4 mm, particle size 3 µm) by elution with acetonitrile / water (34 : 66) containing 39 mM trifluoracetic acid at a flow rate of 1 ml / min.
The retention time of [¹²⁵I]monoiodo-buserelin was approximately 17 min. All other chemicals were obtained from commercial sources at the highest purity grade available.

### 2. METHODS

### 2.1. IP-ONE HTRF®ASSAY

By using homogenous time-resolved fluorescence resonance energy transfer (HTRF), the generation of one component of the GnRH-R signalling cascade can be measured. After stimulation of CHO cells stably expressing human GnRH receptor (established by Prof. Thomas Gudermann, currently University of Marburg, Germany; supplied as frozen cell aliquots by Cell Culture Services, Hamburg, Germany) with the EC₈₀ of the GnRH agonist buserelin, Gq protein-coupled receptor signalling cascade is activated resulting in PLC-dependent cleavage of PIP2 to Inositol-1,4,5-triphosphate (IP3) and Diacylglycerol. The second messenger IP3 is degraded intracellularly to myo-inositol. Inhibition of the final degradation step from Inositol-1-phosphate (IP1) to myo-inositol by addition of lithium chloride leads to accumulation of IP1 in the cells. In cell lysates, IP1 can be detected via an antibody-based HTRF detection technology, where IP1 can displace the FRET acceptor IP1-d2 from binding by Terbium-labelled anti-IP1 antibody as donor resulting in a signal decrease. Compounds were tested for their capability of inhibiting GnRH-R activation by buserelin.
For all IP-One HTRF® assays reagents of Cisbio Bioassays (IP-One Tb Jumbo kit, #62IPAPEJ; Cisbio Bioassays, Bagnols sur Cèze Cedex, France) were used.

For the assay, frozen cell aliquots were thawed and a cell suspension (3.33x10⁶ cells/ml) containing IP1-d2 (dilution 1:40) was prepared and incubated at 37°C. After 1 h 3 µl of the cell suspension were added to 50 nl of a 100-fold concentrated solution of the test compound in DMSO pre-dispensed in a well of a white low-volume 384-well microtiter plate (Greiner Bio-One, Frickenhausen, Germany). The mixture was incubated for 20 min at 22°C to allow for pre-binding of the test compound to the GnRH-R. The receptor signaling cascade was stimulated by addition of 2 µl buserelin or LHRH (at EC₅₀ or EC₈₀) in stimulation buffer (10 mM Hepes pH 7.4, 1 mM CaCl2, 0.5 mM MgCl2, 4.2 mM KCI, 146 mM NaCl, 5.5 mM α-D-Glucose, 0.05% BSA, 125 mM LiCl (final assay concentration 50 mM) in aqua dest.). Plates were incubated for 1 h at 37°C and 5% carbon dioxide before the cells were lysed by adding 3 µl Terbium-labelled anti-IP1 antibody (1:40) diluted in Conjugate & Lysis buffer as supplied with the kit. After an incubation for 1 h at 22°C to enable complete cell lysis and antibody binding to free IP1 or IP1-d2, plates were measured in an HTRF reader, e.g. a RUBYstar, PHERAstar (both BMG Labtechnologies, Offenburg, Germany) or a Viewlux (PerkinElmer LAS, Rodgau-Jügesheim, Germany).
From the fluorescence emissions at 665 nm (FRET) and at 620 nm (background signal of Terbium-antibody), the ratio (emission at 665 nm divided by emission at 620 nm) was calculated and the data were normalized (reaction without test compound = 0% inhibition; all other assay components except agonist = 100% inhibition). On the same microtiter plate, compounds were tested at 10 different concentrations in the range of 20 µM to 1 nM (20 µM, 6.7 µM, 2.2 µM, 0.74 µM, 0.25 µM, 82 nM, 27 nM, 9.2 nM, 3.1 nM and 1 nM; dilution series prepared before the assay at the level of the 100-fold conc. stock solutions by serial 1:3 dilutions in 100% DMSO) in duplicate values for each concentration. By using an in-house software, the IC₅₀ values were calculated by a 4 parameter fit.
The data reveal that the compounds of the present invention have antagonist activities on the human GnRH receptor.
Within the meaning of the present invention the antagonist activity is reflected by the ability of a compound of the invention to antagonize human GnRH receptor stimulation in IP-One HTRF® assay at least three times the standard deviation over the background level.

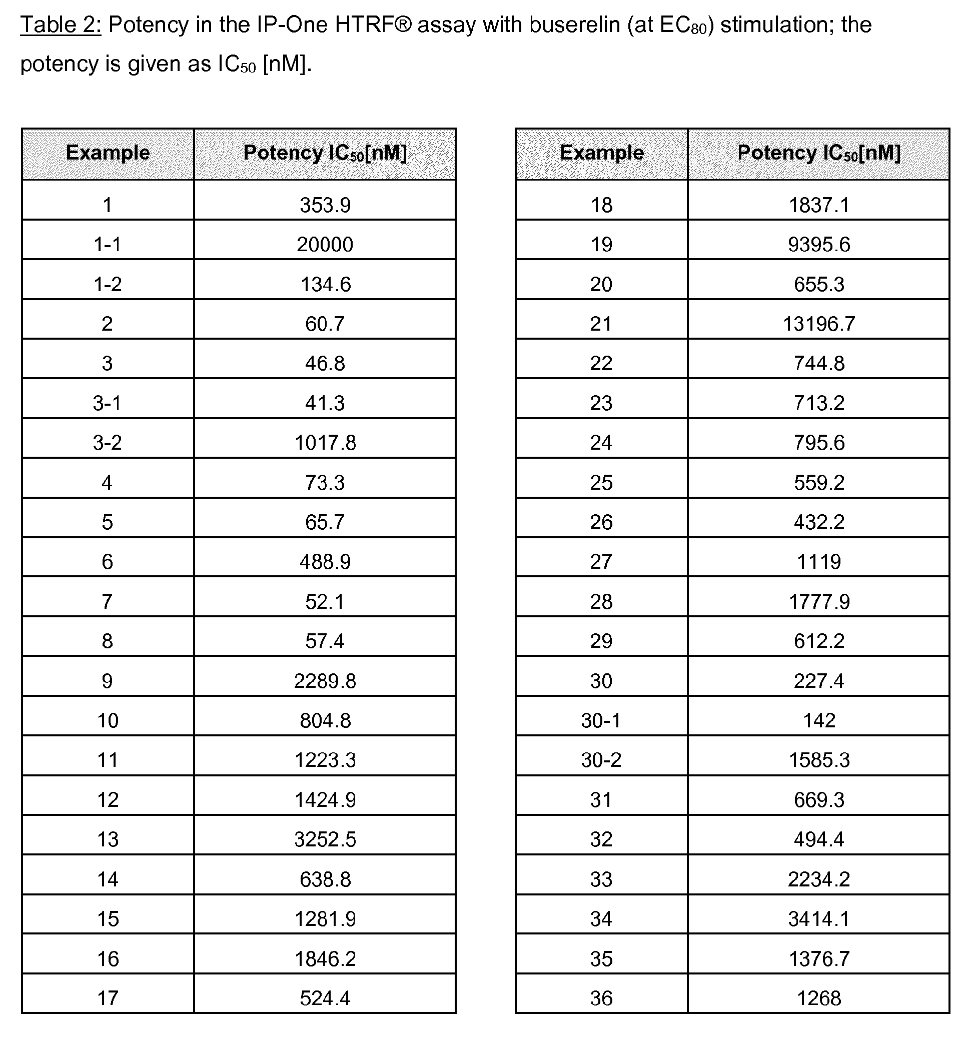

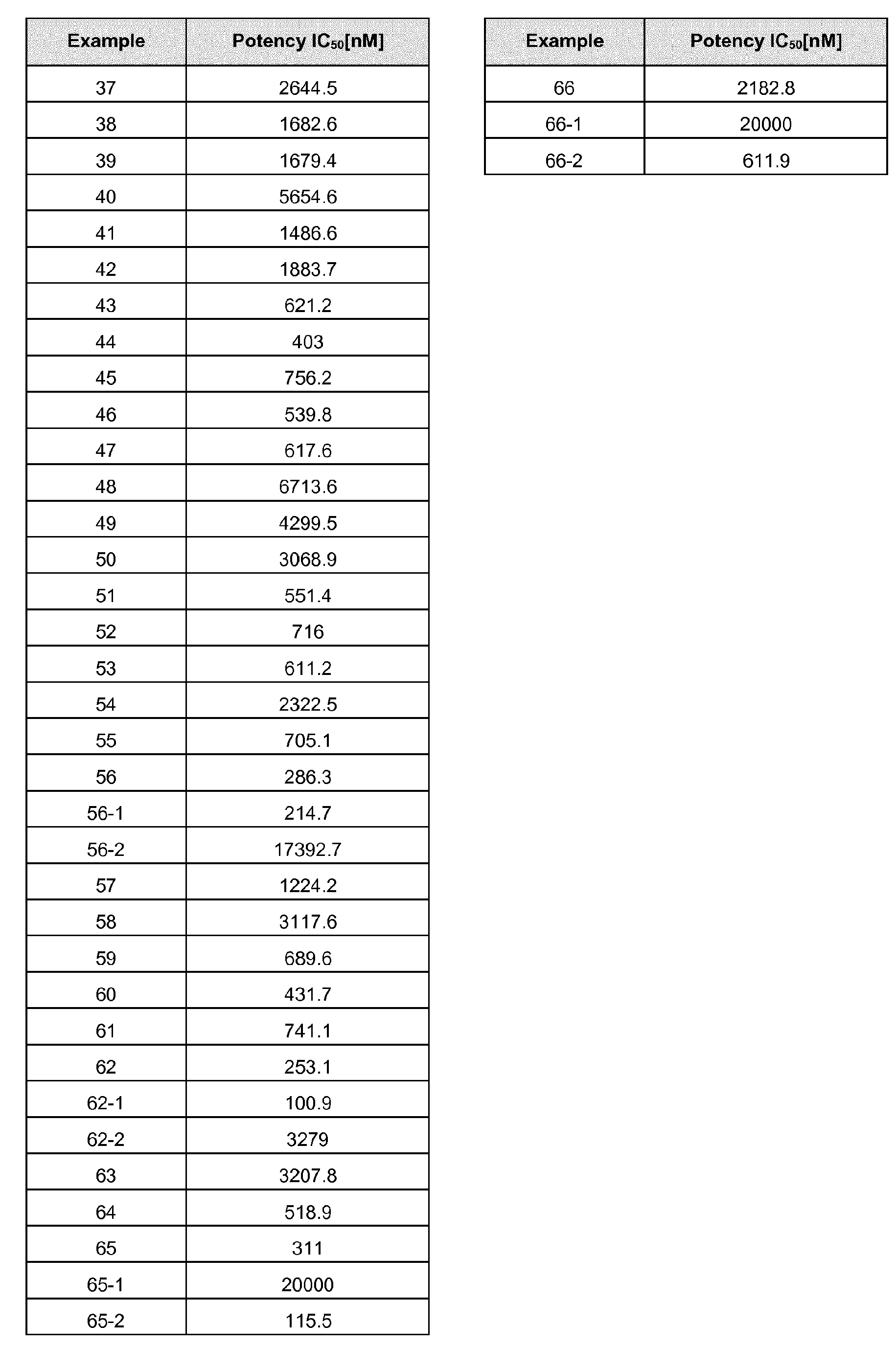

## Claims

1. A compound according to formula (I) 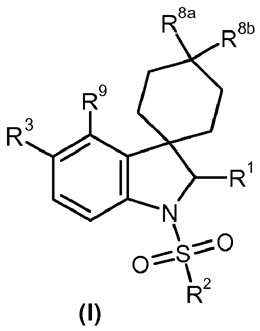 in which
R¹ is selected from the group consisting of hydrogen, C₁-C₆-alkyl, C₃-C₆-cycloalkyl, C₂-C₆-alkenyl, C₂-C₆-alkynyl, aryl, hydroxy-C₁-C₆-alkyl, C₁-C₆-alkoxy-C₁-C₆-alky;
R² is an aryl or heteroaryl group which can be unsubstituted or substituted one to three times with a group R⁴;
R³ is selected from the group consisting of C(O)N(R^{5a})(R^{5b}), N(H)C(O)R⁶, N(H)C(O)N(R^{5a})(R^{5b}) or N(H)C(O)OR⁷;
R⁴ is selected from the group consisting of halogen, hydroxy, CN, C₁-C₆-alkyl, halo-C₁-C₆-alkyl, C₁-C₆-alkoxy, halo-C₁-C₆-alkoxy, C(O)OH, C(O)O-C₁-C₆-alkyl, C(O)NH₂, C(O)NH-C₁-C₆-alkyl, C(O)N(C₁-C₆-alkyl)₂ in which the two alkyl groups are independent from each other or together form a 4-, 5-, 6- or 7-membered cyclic amine group;
R^{5a}, R^{5b} and R⁶ are selected, independently from one another, from the group consisting of hydrogen, C₁-C₆-alkyl, halo-C₁-C₆-alkyl, hydroxy-C₁-C₆-alkyl; C₂-C₆-alkenyl, C₂-C₆-alkynyl, C₁-C₆-alkoxy-C₁-C₆-alkyl, C₃-C₁₀-cycloalkyl, C₃-C₁₀-cycloalkyl-C₁-C₆-alkyl-, aryl, aryl-C₁-C₆-alkyl-, aryl-cyclopropyl, heteroaryl, heteroaryl-C₁-C₆-alkyl-, in which said cycloalkyl, aryl, heteroaryl groups are optionally substituted up to three times with halogen, hydroxy, C₁-C₆-alkyl, C₁-C₆-haloalkyl, C₁-C₆-alkoxy, C₁-C₆-haloalkoxy, C(O)OH, C(O)O-C₁-C₆-alkyl, CN, C(O)NH₂, S(O)₂-C₁-C₆-alkyl, S(O)₂NH₂, S(O)₂N(C₁-C₆-alkyl)₂ in which the two alkyl groups are independent from each other;
R⁷ is selected from the group consisting of C₁-C₆-alkyl, halo-C₁-C₆-alkyl, hydroxy-C₁-C₆-alkyl, C₂-C₆-alkenyl, C₂-C₆-alkynyl, C₁-C₆-alkoxy-C₁-C₆-alkyl, C₃-C₁₀-cycloalkyl, C₃-C₁₀-cycloalkyl-C₁-C₆-alkyl, aryl, aryl-C₁-C₆-alkyl, heteroaryl, or heteroaryl-C₁-C₆-alkyl in which said cycloalkyl, aryl, heteroaryl group is optionally substituted up to three times with a halogen, hydroxy, C₁-C₆-alkyl, C₁-C₆-haloalkyl, C₁-C₆-alkoxy, C₁-C₆-haloalkoxy, C(O)OH, C(O)O-C₁-C₆-alkyl, CN, C(O)NH₂, S(O)₂-C₁-C₆-alkyl, S(O)₂NH₂, S(O)₂N(C₁-C₆-alkyl)₂ in which the two alkyl groups are independent from each other;
R^{8a} is selected from the group consisting of hydrogen, hydroxy, halogen, C₁-C₆-alkyl, halo-C₁-C₆-alkyl;
R^{8b} is selected from the group consisting of hydrogen, halogen, C₁-C₆-alkyl, halo-C₁-C₆-alkyl;
or R^{8a} and R^{8b} together form an oxo group or an oxime group
R⁹ is hydrogen, halogen, hydroxy, CN, C₁-C₆-alkyl, halo-C₁-C₆-alkyl, C₁-C₆-alkoxy, halo-C₁-C₆-alkoxy, C(O)OH, C(O)O-C₁-C₆-alkyl, C(O)NH₂, C(O)NH-C₁-C₆-alkyl, C(O)N(C₁-C₆-alkyl)₂ in which the two alkyl groups are independent from each other.

2. A compound according to claim 1 **characterized in that**
R² is an aryl group which can be unsubstituted or substituted one to three times with a group R⁴;

3. A Compound according to claim 1 or 2 of formula **(Ia)** 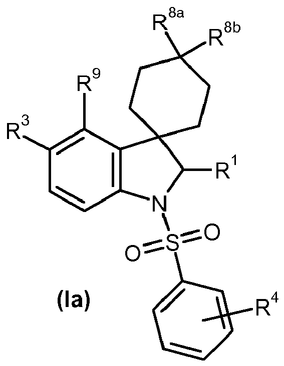 in which
R¹ is selected from the group consisting of hydrogen, C₁-C₆-alkyl, C₃-C₆-cycloalkyl, C₂-C₆-alkenyl;
R³ is selected from the group consisting of C(O)N(R^{5a})(R^{5b}), C(O)NH(R^{5a}), and N(H)C(O)R⁶;
R⁴ is selected from the group consisting of halogen, hydroxy, CN, C₁-C₆-alkyl, halo-C₁-C₆-alkyl, C₁-C₆-alkoxy, halo-C₁-C₆-alkoxy, C(O)OH, C(O)O-C₁-C₆-alkyl, C(O)NH₂, C(O)NH-C₁-C₆-alkyl, C(O)N(C₁-C₆-alkyl)₂ in which the two alkyl groups are independent from each other or together form a 4-, 5-, 6- or 7-membered cyclic amine group;
R^{5a}, R^{5b} and R⁶ are selected, independently from one another, from the group consisting of hydrogen, C₃-C₁₀-cycloalkyl-C₁-C₆-alkyl-, aryl, aryl-C₁-C₆-alkyl-, aryl-cyclopropyl, heteroaryl, heteroaryl-C₁-C₆-alkyl-, in which said cycloalkyl, aryl, heteroaryl groups are optionally substituted up to three times with halogen, C₁-C₆-alkyl, C₁-C₆-haloalkyl, C₁-C₆-alkoxy, C₁-C₆-haloalkoxy, C(O)OH, C(O)O-C₁-C₆-alkyl, S(O)₂-C₁-C₆-alkyl, S(O)₂NH₂, S(O)₂N(C₁-C₆-alkyl)₂ in which the two alkyl groups are independent from each other;
R⁷ is selected from the group consisting of C₁-C₆-alkyl, halo-C₁-C₆-alkyl, hydroxy-C₁-C₆-alkyl, C₂-C₆-alkenyl, C₂-C₆-alkynyl, C₁-C₆-alkoxy-C₁-C₆-alkyl, C₃-C₁₀-cycloalkyl, C₃-C₁₀-cycloalkyl-C₁-C₆-alkyl, aryl, aryl-C₁-C₆-alkyl-, heteroaryl, or heteroaryl-C₁-C₆-alkyl- in which said cycloalkyl, aryl, heteroaryl group is optionally substituted up to three times with a halogen, hydroxy, an C₁-C₆-alkyl, C₁-C₆-haloalkyl, C₁-C₆-alkoxy, C₁-C₆-haloalkoxy, C(O)OH, C(O)O-C₁-C₆-alkyl, CN, C(O)NH₂, S(O)₂-C₁-C₆-alkyl, S(O)₂NH₂, S(O)₂N(C₁-C₆-alkyl)₂ in which the two alkyl groups are independent from each other.
R^{8a} is selected from the group consisting of hydrogen, hydroxy, halogen, C₁-C₆-alkyl, halo-C₁-C₆-alkyl;
R^{8b} is selected from the group consisting of hydrogen, halogen, C₁-C₆-alkyl, halo-C₁-C₆-alkyl; or
R^{8a} and R^{8b} together form an oxo group or an oxime group;
R⁹ is hydrogen, halogen, hydroxy, CN, C₁-C₆-alkyl, halo-C₁-C₆-alkyl, C₁-C₆-alkoxy, halo-C₁-C₆-alkoxy, C(O)OH, C(O)O-C₁-C₆-alkyl, C(O)NH₂, C(O)NH-C₁-C₆-alkyl, C(O)N(C₁-C₆-alkyl)₂ in which the two alkyl groups are independent from each other.

4. A compound according to any one of the claims 1 to3 of formula **(Ib)** 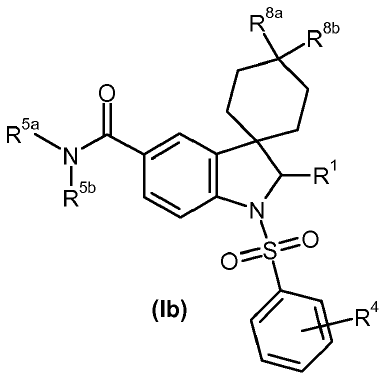
R¹ is selected from the group consisting of hydrogen, C₁-C₆-alkyl, C₃-C₆-cycloalkyl, C₂-C₆-alkenyl;
R⁴ is selected from the group consisting of halogen, hydroxy, CN, C₁-C₆-alkyl, halo-C₁-C₆-alkyl, C₁-C₆-alkoxy, halo-C₁-C₆-alkoxy, C(O)OH, C(O)O-C₁-C₆-alkyl, C(O)NH₂, C(O)NH-C₁-C₆-alkyl, C(O)N(C₁-C₆-alkyl)₂ in which the two alkyl groups are independent from each other or together form a 4-, 5-, 6- or 7-membered cyclic amine group;
R^{5a} and R^{5b} are selected, independently from one another, from the group consisting of hydrogen, C₃-C₁₀-cycloalkyl-C₁-C₆-alkyl-, aryl, aryl-C₁-C₆-alkyl-, aryl-cyclopropyl, heteroaryl, heteroaryl-C₁-C₆-alkyl-, in which said cycloalkyl, aryl, heteroaryl groups are optionally substituted up to three times with halogen, C₁-C₆-alkyl, C₁-C₆-haloalkyl, C₁-C₆-alkoxy, C₁-C₆-haloalkoxy, C(O)OH, C(O)O-C₁-C₆-alkyl, S(O)₂-C₁-C₆-alkyl, S(O)₂NH₂, S(O)₂N(C₁-C₆-alkyl)₂ in which the two alkyl groups are independent from each other;
R⁷ is selected from the group consisting of C₁-C₆-alkyl, halo-C₁-C₆-alkyl, hydroxy-C₁-C₆-alkyl, C₂-C₆-alkenyl, C₂-C₆-alkynyl, C₁-C₆-alkoxy-C₁-C₆-alkyl, C₃-C₁₀-cycloalkyl, C₃-C₁₀-cycloalkyl-C₁-C₆-alkyl, aryl, aryl-C₁-C₆-alkyl-, heteroaryl, or heteroaryl-C₁-C₆-alkyl- in which said cycloalkyl, aryl, heteroaryl group is optionally substituted up to three times with a halogen, hydroxy, an C₁-C₆-alkyl, C₁-C₆-haloalkyl, C₁-C₆-alkoxy, C₁-C₆-haloalkoxy, C(O)OH, C(O)O-C₁-C₆-alkyl, CN, C(O)NH₂, S(O)₂-C₁-C₆-alkyl, S(O)₂NH₂, S(O)₂N(C₁-C₆-alkyl)₂ in which the two alkyl groups are independent from each other.
R^{8a} is selected from the group consisting of hydrogen, hydroxy, halogen, C₁-C₆-alkyl, halo-C₁-C₆-alkyl;
R^{8b} is selected from the group consisting of hydrogen, halogen, C₁-C₆-alkyl, halo-C₁-C₆-alkyl; or
R^{8a} and R^{8b} together form an oxo group or an oxime group.

5. A compound according to any one of the claims 1 to 4 **characterized in that**
R¹ is,C₃-C₆-cycloalky or C₂-C₆-alkenyl I;

6. A compound according to any one of the claims 1 to 5 **characterized in that**
R³ is a C(O)NH(R^{5a});

7. A compound according to any one of the claims 1 to 6 **characterized in that**
R⁴ is a single group in para or meta position selected from the group consisting of halogen, C₁-C₆-alkoxy, C(O)OH, C(O)O-C₁-C₆-alkyl.

8. A compound according to any one of the claims 1 to 7 **characterized in that**
R^{5a} , R^{5b} or R⁶ are selected, independently from one another, from the group consisting of hydrogen, C₃-C₁₀-cycloalkyl-C₁-C₆-alkyl-, aryl, aryl-C₁-C₆-alkyl-, aryl-cyclopropyl, heteroaryl, heteroaryl-C₁-C₆-alkyl-, in which said cycloalkyl, aryl, heteroaryl groups are optionally substituted up to three times with halogen, C₁-C₆-alkyl, C₁-C₆-haloalkyl, C₁-C₆-alkoxy, C₁-C₆-haloalkoxy, C(O)OH, C(O)O-C₁-C₆-alkyl, S(O)₂-C₁-C₆-alkyl, S(O)₂NH₂, S(O)₂N(C₁-C₆-alkyl)₂ in which the two alkyl groups are independent from each other.

9. A compound according to any one of the claims 1 to 8 **characterized in that**
R^{8a} is selected from the group consisting of hydrogen, hydroxy, halogen; and
R^{8b} is selected from the group consisting of hydrogen, halogen, C₁-C₆-alkyl, halo-C₁-C₆-alkyl.

10. A compound according to any one of the claims 1 to 9 **characterized in that**
R^{8a} and R^{8b} together form an oxo group or an oxime group

11. A compound according to claim 1 with the following formula:
N-[(3-chloropyridin-2-yl)methyl]-2'-cyclopropyl-1'-[(4-fluorophenyl)sulfonyl]-4-hydroxy-4-(trifluoromethyl)-1',2'-dihydrospiro[cyclohexane-1,3'-indole]-5'-carboxamide
N-(2-chlorobenzyl)-2'-cyclopropyl-1'-[(4-fluorophenyl)sulfonyl]-4-hydroxy-1',2'-dihydrospiro[cyclohexane-1,3'-indole]-5'-carboxamide
N-(2-chlorobenzyl)-2'-cyclopropyl-1'-[(4-fluorophenyl)sulfonyl]-4-oxo-1',2'-dihydrospiro[cyclohexane-1,3'-indole]-5'-carboxamide
N-(2-chlorobenzyl)-2'-cyclopropyl-1'-[(4-fluorophenyl)sulfonyl]-4-hydroxy-4-methyl-1',2'-dihydrospiro[cyclohexane-1,3'-indole]-5'-carboxamide
N-(2-chlorobenzyl)-2'-cyclopropyl-1'-[(4-fluorophenyl)sulfonyl]-4-(hydroxyimino)-1',2'-dihydrospiro[cyclohexane-1,3'-indole]-5'-carboxamide
N-(2-chlorobenzyl)-2'-cyclopropyl-4,4-difluoro-1'-[(4-fluorophenyl)sulfonyl]-1',2'-dihydrospiro[cyclohexane-1,3'-indole]-5'-carboxamide
N-{[3-chloro-5-(trifluoromethyl)pyridin-2-yl]methyl}-2'-cyclopropyl-1'-[(4-fluorophenyl)sulfonyl]-4-hydroxy-1',2'-dihydrospiro[cyclohexane-1,3'-indole]-5'-carboxamide
N-{[3-chloro-5-(trifluoromethyl)pyridin-2-yl]methyl}-2'-cyclopropyl-1'-[(4-fluorophenyl)sulfonyl]-4-oxo-1',2'-dihydrospiro[cyclohexane-1,3'-indole]-5'-carboxamide
methyl3-({5'-[(2-chlorobenzyl)carbamoyl]-2'-cyclopropylspiro[cyclohexane-1,3'-indol]-1'(2'H)-yl}sulfonyl)benzoate
3-({5'-[(2-chlorobenzyl)carbamoyl]-2'-cyclopropylspiro[cyclohexane-1,3'-indol]-1'(2'H)-yl}sulfonyl)benzoic acid
methyl3-{[5'-{[(3-chloropyridin-2-yl)methyl]carbamoyl}-2'-cyclopropylspiro[cyclohexane-1,3'-indol]-1'(2'H)-yl]sulfonyl}benzoate
1'-[(4-fluorophenyl)sulfonyl]-4-hydroxy-2'-(3-methylbut-2-en-1-yl)-N-(1H-pyrazol-3-yl)-4-(trifluoromethyl)-1',2'-dihydrospiro[cyclohexane-1,3'-indole]-5'-carboxamide
1'-[(4-fluorophenyl)sulfonyl]-4-hydroxy-2'-(3-methylbut-2-en-1-yl)-N-(3-methylphenyl)-4-(trifluoromethyl)-1',2'-dihydrospiro[cyclohexane-1,3'-indole]-5'-carboxamide
3-[({1'-[(4-fluorophenyl)sulfonyl]-4-hydroxy-2'-(3-methylbut-2-en-1-yl)-4-(trifluoromethyl)-1',2'-dihydrospiro[cyclohexane-1,3'-indol]-5'-yl}carbonyl)amino]benzoic acid
1'-[(4-fluorophenyl)sulfonyl]-4-hydroxy-2'-(3-methylbut-2-en-1-yl)-N-[3-(methylsulfonyl)phenyl]-4-(trifluoromethyl)-1',2'-dihydrospiro[cyclohexane-1,3'-indole]-5'-carboxamide
1'-[(4-fluorophenyl)sulfonyl]-4-hydroxy-2'-(3-methylbut-2-en-1-yl)-N-(3-sulfamoylphenyl)-4-(trifluoromethyl)-1',2'-dihydrospiro[cyclohexane-1,3'-indole]-5'-carboxamide
1'-[(4-fluorophenyl)sulfonyl]-4-hydroxy-2'-(3-methylbut-2-en-1-yl)-N-(1,2-oxazol-3-yl)-4-(trifluoromethyl)-1',2'-dihydrospiro[cyclohexane-1,3'-indole]-5'-carboxamide
1'-[(4-fluorophenyl)sulfonyl]-4-hydroxy-2'-(3-methylbut-2-en-1-yl)-N-(5-methylpyridin-3-yl)-4-(trifluoromethyl)-1',2'-dihydrospiro[cyclohexane-1,3'-indole]-5'-carboxamide
1'-[(4-fluorophenyl)sulfonyl]-4-hydroxy-2'-(3-methylbut-2-en-1-yl)-4-(trifluoromethyl)-N-[5-(trifluoromethyl)pyridin-3-yl]-1',2'-dihydrospiro[cyclohexane-1,3'-indole]-5'-carboxamide
1'-[(4-fluorophenyl)sulfonyl]-4-hydroxy-2'-(3-methylbut-2-en-1-yl)-N-(1,3-oxazol-2-yl)-4-(trifluoromethyl)-1',2'-dihydrospiro[cyclohexane-1,3'-indole]-5'-carboxamide
N-(cyclohexylmethyl)-1'-[(4-fluorophenyl)sulfonyl]-4-hydroxy-2'-(3-methylbut-2-en-1-yl)-4-(trifluoromethyl)-1',2'-dihydrospiro[cyclohexane-1,3'-indole]-5'-carboxamide
N-(2-chlorobenzyl)-1'-[(4-fluorophenyl)sulfonyl]-4-hydroxy-2'-(3-methylbut-2-en-1-yl)-4-(trifluoromethyl)-1',2'-dihydrospiro[cyclohexane-1,3'-indole]-5'-carboxamide
1'-[(4-fluorophenyl)sulfonyl]-4-hydroxy-2'-(3-methylbut-2-en-1-yl)-4-(trifluoromethyl)-N-{[3-(trifluoromethyl)pyridin-2-yl]methyl}-1',2'-dihydrospiro[cyclohexane-1,3'-indole]-5'-carboxamide
2'-cyclopropyl-1'-[(4-fluorophenyl)sulfonyl]-4-hydroxy-N-(3-methylphenyl)-4-(trifluoromethyl)-1',2'-dihydrospiro[cyclohexane-1,3'-indole]-5'-carboxamide
2'-cyclopropyl-1'-[(4-fluorophenyl)sulfonyl]-4-hydroxy-N-(5-methylpyridin-3-yl)-4-(trifluoromethyl)-1',2'-dihydrospiro[cyclohexane-1,3'-indole]-5'-carboxamide
N-(2-chlorobenzyl)-1'-[(4-fluorophenyl)sulfonyl]-4-hydroxy-2'-methyl-4-(trifluoromethyl)-1',2'-dihydrospiro[cyclohexane-1,3'-indole]-5'-carboxamide
methyl 3-{[2'-cyclopropyl-4-hydroxy-5'-(1,2-oxazol-3-ylcarbamoyl)-4-(trifluoromethyl)spiro[cyclohexane-1,3'-indol]-1'(2'H)-yl]sulfonyl}benzoate
methyl 3-{[2'-cyclopropyl-5'-({[3-fluoro-5-(trifluoromethyl)pyridin-2-yl]methyl}carbamoyl)-4-hydroxy-4-(trifluoromethyl)spiro[cyclohexane-1,3'-indol]-1'(2'H)-yl]sulfonyl}benzoate
methyl 3-{[2'-cyclopropyl-4-hydroxy-4-(trifluoromethyl)-5'-({[3-(trifluoromethyl)pyridin-2-yl]methyl}carbamoyl)spiro[cyclohexane-1,3'-indol]-1'(2'H)-yl]sulfonyl}benzoate
methyl 3-[({2'-cyclopropyl-1'-[(4-fluorophenyl)sulfonyl]-4-hydroxy-4-(trifluoromethyl)-1',2'-dihydrospiro[cyclohexane-1,3'-indol]-5'-yl}carbonyl)amino]benzoate
methyl 3-[({2'-cyclopropyl-4-hydroxy-1'-[(3-methoxyphenyl)sulfonyl]-4-(trifluoromethyl)-1',2'-dihydrospiro[cyclohexane-1,3'-indol]-5'-yl}carbonyl)amino]benzoate
methyl 3-[({1'-[(4-fluorophenyl)sulfonyl]-4-hydroxy-2'-(3-methylbut-2-en-1-yl)-4-(trifluoromethyl)-1',2'-dihydrospiro[cyclohexane-1,3'-indol]-5'-yl}carbonyl)amino]benzoate
2'-cyclopropyl-4-hydroxy-1'-[(3-methoxyphenyl)sulfonyl]-N-(1H-pyrazol-3-yl)-4-(trifluoromethyl)-1',2'-dihydrospiro[cyclohexane-1,3'-indole]-5'-carboxamide
2'-cyclopropyl-4-hydroxy-1'-[(3-methoxyphenyl)sulfonyl]-N-(3-methylphenyl)-4-(trifluoromethyl)-1',2'-dihydrospiro[cyclohexane-1,3'-indole]-5'-carboxamide
3-[({2'-cyclopropyl-4-hydroxy-1'-[(3-methoxyphenyl)sulfonyl]-4-(trifluoromethyl)-1',2'-dihydrospiro[cyclohexane-1,3'-indol]-5'-yl}carbonyl)amino]benzoic acid
2'-cyclopropyl-4-hydroxy-1'-[(3-methoxyphenyl)sulfonyl]-N-[3-(methylsulfonyl)phenyl]-4-(trifluoromethyl)-1',2'-dihydrospiro[cyclohexane-1,3'-indole]-5'-carboxamide
2'-cyclopropyl-4-hydroxy-1'-[(3-methoxyphenyl)sulfonyl]-N-(3-sulfamoylphenyl)-4-(trifluoromethyl)-1',2'-dihydrospiro[cyclohexane-1,3'-indole]-5'-carboxamide
2'-cyclopropyl-4-hydroxy-1'-[(3-methoxyphenyl)sulfonyl]-N-(1,2-oxazol-3-yl)-4-(trifluoromethyl)-1',2'-dihydrospiro[cyclohexane-1,3'-indole]-5'-carboxamide
2'-cyclopropyl-4-hydroxy-1'-[(3-methoxyphenyl)sulfonyl]-N-(5-methylpyridin-3-yl)-4-(trifluoromethyl)-1',2'-dihydrospiro[cyclohexane-1,3'-indole]-5'-carboxamide
2'-cyclopropyl-4-hydroxy-1'-[(3-methoxyphenyl)sulfonyl]-4-(trifluoromethyl)-N-[5-(trifluoromethyl)pyridin-3-yl]-1',2'-dihydrospiro[cyclohexane-1,3'-indole]-5'-carboxamide
2'-cyclopropyl-4-hydroxy-1'-[(3-methoxyphenyl)sulfonyl]-N-(1,3-oxazol-2-yl)-4-(trifluoromethyl)-1',2'-dihydrospiro[cyclohexane-1,3'-indole]-5'-carboxamide
N-(cyclohexylmethyl)-2'-cyclopropyl-4-hydroxy-1'-[(3-methoxyphenyl)sulfonyl]-4-(trifluoromethyl)-1',2'-dihydrospiro[cyclohexane-1,3'-indole]-5'-carboxamide
N-(2-chlorobenzyl)-2'-cyclopropyl-4-hydroxy-1'-[(3-methoxyphenyl)sulfonyl]-4-(trifluoromethyl)-1',2'-dihydrospiro[cyclohexane-1,3'-indole]-5'-carboxamide
2'-cyclopropyl-4-hydroxy-1'-[(3-methoxyphenyl)sulfonyl]-4-(trifluoromethyl)-N-{[3-(trifluoromethyl)pyridin-2-yl]methyl}-1',2'-dihydrospiro[cyclohexane-1,3'-indole]-5'-carboxamide
N-[(3-chloropyridin-2-yl)methyl]-2'-cyclopropyl-4-hydroxy-1'-[(3-methoxyphenyl)sulfonyl]-4-(trifluoromethyl)-1',2'-dihydrospiro[cyclohexane-1,3'-indole]-5'-carboxamide
2'-cyclopropyl-1'-[(4-fluorophenyl)sulfonyl]-4-hydroxy-N-[3-(methylsulfonyl)phenyl]-4-(trifluoromethyl)-1',2'-dihydrospiro[cyclohexane-1,3'-indole]-5'-carboxamide
2'-cyclopropyl-1'-[(4-fluorophenyl)sulfonyl]-4-hydroxy-N-(3-sulfamoylphenyl)-4-(trifluoromethyl)-1',2'-dihydrospiro[cyclohexane-1,3'-indole]-5'-carboxamide
3-{[2'-cyclopropyl-4-hydroxy-5'-(1,2-oxazol-3-ylcarbamoyl)-4-(trifluoromethyl)spiro[cyclohexane-1,3'-indol]-1'(2'H)-yl]sulfonyl}benzoic acid
3-{[2'-cyclopropyl-5'-({[3-fluoro-5-(trifluoromethyl)pyridin-2-yl]methyl}carbamoyl)-4-hydroxy-4-(trifluoromethyl)spiro[cyclohexane-1,3'-indol]-1'(2'H)-yl]sulfonyl}benzoic acid
3-{[2'-cyclopropyl-4-hydroxy-4-(trifluoromethyl)-5'-({[3-(trifluoromethyl)pyridin-2-yl]methyl}carbamoyl)spiro[cyclohexane-1,3'-indol]-1'(2'H)-yl]sulfonyl}benzoic acid
N-[1-(4-chlorophenyl)cyclopropyl]-2'-cyclopropyl-1'-[(4-fluorophenyl)sulfonyl]-4-hydroxy-4-(trifluoromethyl)-1',2'-dihydrospiro[cyclohexane-1,3'-indole]-5'-carboxamide
2'-cyclopropyl-1'-[(4-fluorophenyl)sulfonyl]-4-hydroxy-N-(1H-pyrazol-3-yl)-4-(trifluoromethyl)-1',2'-dihydrospiro[cyclohexane-1,3'-indole]-5'-carboxamide
2'-cyclopropyl-N-(2,3-dihydro-1H-inden-1-yl)-1'-[(4-fluorophenyl)sulfonyl]-4-hydroxy-4-(trifluoromethyl)-1',2'-dihydrospiro[cyclohexane-1,3'-indole]-5'-carboxamide
2'-cyclopropyl-1'-[(4-fluorophenyl)sulfonyl]-4-hydroxy-4-(trifluoromethyl)-N-[3-(trifluoromethyl)phenyl]-1',2'-dihydrospiro[cyclohexane-1,3'-indole]-5'-carboxamide
N-(3-chlorophenyl)-2'-cyclopropyl-1'-[(4-fluorophenyl)sulfonyl]-4-hydroxy-4-(trifluoromethyl)-1',2'-dihydrospiro[cyclohexane-1,3'-indole]-5'-carboxamide
2'-cyclopropyl-1'-[(4-fluorophenyl)sulfonyl]-4-hydroxy-N-(1,2-oxazol-3-yl)-4-(trifluoromethyl)-1',2'-dihydrospiro[cyclohexane-1,3'-indole]-5'-carboxamide
1'-{[3-(azetidin-1-ylcarbonyl)phenyl]sulfonyl}-2'-cyclopropyl-4-hydroxy-N-(1,2-oxazol-3-yl)-4-(trifluoromethyl)-1',2'-dihydrospiro[cyclohexane-1,3'-indole]-5'-carboxamide
2'-cyclopropyl-1'-[(4-fluorophenyl)sulfonyl]-4-hydroxy-4-(trifluoromethyl)-N-[5-(trifluoromethyl)pyridin-3-yl]-1',2'-dihydrospiro[cyclohexane-1,3'-indole]-5'-carboxamide
2'-cyclopropyl-N-(cyclopropylmethyl)-1'-[(4-fluorophenyl)sulfonyl]-4-hydroxy-4-(trifluoromethyl)-1',2'-dihydrospiro[cyclohexane-1,3'-indole]-5'-carboxamide
N-(2-chloro-4-fluorobenzyl)-2'-cyclopropyl-1'-[(4-fluorophenyl)sulfonyl]-4-hydroxy-4-(trifluoromethyl)-1',2'-dihydrospiro[cyclohexane-1,3'-indole]-5'-carboxamide
N-(cyclohexylmethyl)-2'-cyclopropyl-1'-[(4-fluorophenyl)sulfonyl]-4-hydroxy-4-(trifluoromethyl)-1',2'-dihydrospiro[cyclohexane-1,3'-indole]-5'-carboxamide
N-(2-chlorobenzyl)-2'-cyclopropyl-1'-[(4-fluorophenyl)sulfonyl]-4-hydroxy-4-(trifluoromethyl)-1',2'-dihydrospiro[cyclohexane-1,3'-indole]-5'-carboxamide
1'-{[3-(azetidin-1-ylcarbonyl)phenyl]sulfonyl}-2'-cyclopropyl-N-{[3-fluoro-5-(trifluoromethyl)pyridin-2-yl]methyl}-4-hydroxy-4-(trifluoromethyl)-1',2'-dihydrospiro[cyclohexane-1,3'-indole]-5'-carboxamide
N-[(3-chloro-5-fluoropyridin-2-yl)methyl]-2'-cyclopropyl-1'-[(4-fluorophenyl)sulfonyl]-4-hydroxy-4-(trifluoromethyl)-1',2'-dihydrospiro[cyclohexane-1,3'-indole]-5'-carboxamide
2'-cyclopropyl-1'-[(4-fluorophenyl)sulfonyl]-4-hydroxy-4-(trifluoromethyl)-N-{[3-(trifluoromethyl)pyridin-2-yl]methyl}-1',2'-dihydrospiro[cyclohexane-1,3'-indole]-5'-carboxamide
1'-{[3-(azetidin-1-ylcarbonyl)phenyl]sulfonyl}-2'-cyclopropyl-4-hydroxy-4-(trifluoromethyl)-N-{[3-(trifluoromethyl)pyridin-2-yl]methyl}-1',2'-dihydrospiro[cyclohexane-1,3'-indole]-5'-carboxamide

12. A compound according to any one of the claims 1 to 15 for use as a medicament.

13. A compound according to any one of the claims 1 to 15 for use in the treatment of endometriosis, uterine leiomyoma (fibroids), polycystic ovarian disease, menorrhagia, dysmenorrhea, hirsutism, precocious puberty, gonadal steroid-dependent neoplasia such as cancers of the prostate, breast and ovary, gonadotrope pituitary adenomas, sleep apnea, irritable bowel syndrome, premenstrual syndrome, benign prostatic hypertrophy, contraception, infertility, assisted reproductive therapy such as in vitro fertilization, in the treatment of growth hormone deficiency and short stature, and in the treatment of systemic lupus erythematosus.

14. A compound according to any one of the claims 1 to 15 for use as contraceptive.

15. A pharmaceutical composition comprising a compound according to any one of the claims 1 to 15.
